(19) Europäisches Patentamt
European Patent Office
Office européen des brevets

(11) EP 2 198 891 A1

(12) EUROPEAN PATENT APPLICATION
published in accordance with Art. 153(4) EPC

(43) Date of publication:
23.06.2010 Bulletin 2010/25

(21) Application number: 08829249.5

(22) Date of filing: 05.09.2008

(51) Int Cl.:
A61K 48/00 (2006.01) A61K 31/337 (2006.01)
A61K 31/7105 (2006.01) A61K 31/711 (2006.01)
A61P 35/00 (2006.01) A61P 43/00 (2006.01)

(86) International application number:
PCT/JP2008/066127

(87) International publication number:
WO 2009/031671 (12.03.2009 Gazette 2009/11)

(84) Designated Contracting States:
AT BE BG CH CY CZ DE DK EE ES FI FR GB GR HR HU IE IS IT LI LT LU LV MC MT NL NO PL PT RO SE SI SK TR
Designated Extension States:
AL BA MK RS

(30) Priority: 07.09.2007 JP 2007233316

(71) Applicant: The University of Tokushima
Tokushima-shi
Tokushima 770-8501 (JP)

(72) Inventors:
- KONDO, Shigetada,
The University of Tokushima Graduate School
Tokushima-shi
Tokushima 770-8503 (JP)
- ROKUTAN, Kazuhito
The University of Tokushima Graduate School
Tokushima-shi
Tokushima 770-8503 (JP)

(74) Representative: Woods, Geoffrey Corlett et al
J.A. Kemp & Co.
14 South Square
Gray's Inn
London
WC1R 5JJ (GB)

# (54) METHOD FOR PROMOTING THE EXPRESSION OF P53, AND P53 EXPRESSION PROMOTER FOR USE IN THE METHOD

(57) A method for promoting expression of p53 mRNA and an expression promoting agent for use in the method are provided. The expression of the p53 gene can be promoted by inhibiting the binding between RNA transcribed from exon 1 of the vegf-A gene and mRNA transcribed from the p53 gene. The inhibition of the binding can be achieved by, for example, degrading the RNA transcribed from the exon 1 of the vegf-A gene, inhibiting transcription of RNA from the exon 1 of the vegf-A gene, or binding a nucleic acid that is different from the mRNA transcribed from the p53 gene to the RNA transcribed from the exon 1 of the vegf-A gene.

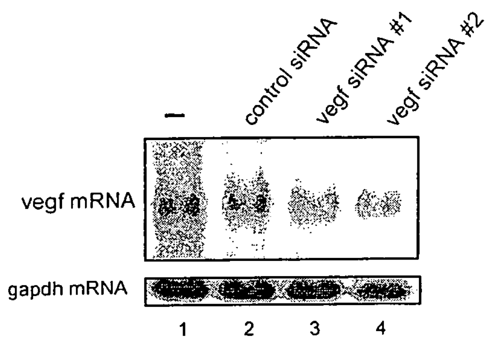


FIG. 1A

EP 2 198 891 A1

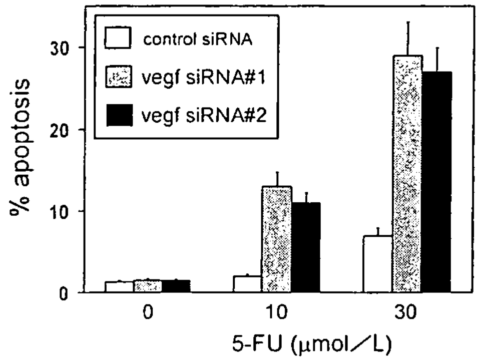


FIG. 1B

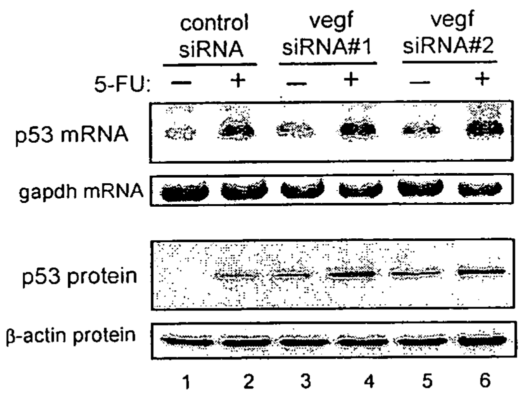


FIG. 1C

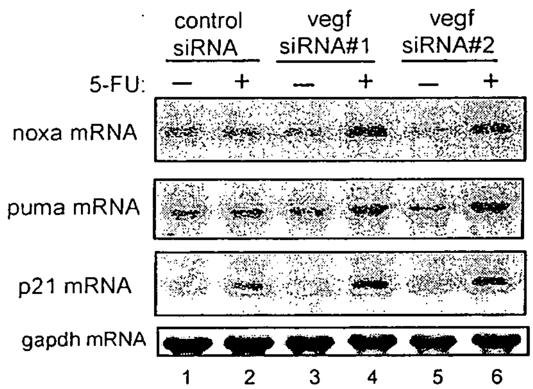


FIG. 1D

**Description**

Technical Field

The present invention relates to a method for promoting expression of p53 in a cell, a p53 expression promoting agent used in the method, and uses thereof.

Background Art

When DNA is damaged, the cell tries to repair the damage. If the damage is irreparable, the cell undergoes apoptosis. As a protein playing an important role in the process of this apoptosis, p53 has been known. p53, which is a transcription factor, is a nucleoprotein composed of 393 amino acids and having a molecular weight of 53,000 Da. Since p53 is degraded rapidly in a normal cell, the expression level of this protein in the cell is low.
However, when DNA damage or the like occurs in a normal cell, p53 is phosphorylated and stabilized by transduction of signals caused thereby. As a result, p53-dependent transcription of various genes (p21 gene, noxa gene, puma gene, pig3 gene, etc.) is promoted, and by the involvement of gene products thereof, apoptosis of the cell progresses.
However, in the case of a tumor cell, apoptosis is less apt to be caused even by the DNA damage that causes apoptosis to progress in a normal cell as described above. As one of the causes thereof, mutation of the p53 gene has been known. When mutation occurs in the p53 gene, the expressed p53 protein has lost its function, so that the progress of apoptosis is inhibited consequently. Also, from the fact that mutation of the p53 gene is involved in the development of tumors with high frequency, it has been known that p53 plays an important role as a tumor suppressor (Non-Patent Documents 1 to 3). The mutation of the p53 gene, however, is a phenomenon observed at a relatively late stage in the tumor development process (malignant progression), and there has been reported a phenomenon in which, in many clinically diagnosed tumors, the mutation of the p53 gene is not observed and expression of p53 is decreased. For example, in the case of breast tumors, it has been reported that mutation of the p53 gene is not observed in about 80% of the cases, and the amount of expressed p53 mRNA is decreased to about 1/5 to about 1/10 as compared with those in surrounding normal tissues (Non-Patent Documents 1, 4, and 5).
Non-Patent Document 1: Steele, R. et al., Br. J. Surg. 85, 1460-7 (1998)
Non-Patent Document 2: Levine, A. et al., Cell 88, 323-331(1997)
Non-Patent Document 3: Vogelstein, B. et al., Nature 408, 307-310 (2000)
Non-Patent Document 4: Pharoah, P. et al., Br. J. Cancer 80, 1968-1973 (1999)
Non-Patent Document 5: Raman, V. et al., Nature 405, 974-978 (2000)

Disclosure of Invention

Hence, it is expected that, by suppressing the decrease in the amount of expressed p53 mRNA in tumor cells to maintain or increase the amount of expressed p53 mRNA, p53-dependent transcription of the various genes is promoted, so that it becomes possible to cause apoptosis of the tumor cells to progress by the resultant various gene products, for example.
With the foregoing in mind, it is an object of the present invention to provide a method for promoting expression of p53 mRNA and an expression promoting agent used in the method.
In order to achieve the above object, a p53 expression promoting method according to the present invention is a method for promoting expression of a p53 gene in a cell, wherein the expression of the p53 gene is promoted by inhibiting binding between RNA transcribed from exon 1 of a vegf-A gene and mRNA transcribed from the p53 gene.
A p53 expression promoting agent (a p53 expression promoter) according to the present invention is a p53 expression promoting agent for use in the p53 expression promoting method of the present invention. The p53 expression promoting agent contains at least one binding inhibitor that inhibits binding between RNA transcribed from exon 1 of a vegf-A gene and mRNA transcribed from a p53 gene. The binding inhibitor is selected from the group consisting of the following (A1) to (A4).

(A1) at least one binding inhibitor selected from the group consisting of siRNA, a siRNA precursor that releases siRNA when degraded by ribonuclease, an antisense, and ribozyme, each of which degrades the RNA transcribed from the exon 1 of the vegf-A gene;
(A2) at least one binding inhibitor selected from the group consisting of siRNA, a siRNA precursor that releases siRNA when degraded by ribonuclease, an antisense, ribozyme, and a decoy nucleic acid, each of which inhibits transcription of RNA from the exon 1 of the vegf-A gene;

(A3) a binding inhibitor that contains a nucleic acid different from the mRNA transcribed from the p53 gene and binds to the RNA transcribed from the exon 1 of the vegf-A gene; and
(A4) a binding inhibitor that contains an expression vector in which DNA coding the at least one binding inhibitor selected from the group consisting of (A1) to (A3) is inserted.

An apoptosis promoting method according to the present invention is a method for promoting apoptosis of a cell. In this method, expression of p53 in the cell is promoted by the p53 expression promoting method of the present invention.
An apoptosis promoting agent according to the present invention is an apoptosis promoting agent for promoting apoptosis of a cell, containing the p53 expression promoting agent of the present invention.
An enhancement method according to the present invention is a method for enhancing sensitivity of a cell to an anticancer agent. In this method, expression of p53 in the cell is promoted by the p53 expression promoting method of the present invention.
An enhancer according to the present invention is an enhancer for enhancing sensitivity of a cell to an anticancer agent. The enhancer contains the p53 expression promoting agent of the present invention.
A treatment method according to the present invention includes the step of administering the p53 expression promoting agent of the present invention to a cancer cell that is *in vivo.*
An anticancer agent according to the present invention contains the p53 expression promoting agent of the present invention.
A p53 mRNA binding agent according to the present invention is a binding agent that can bind to p53 mRNA. The p53 mRNA binding agent contains a nucleic acid, and the nucleic acid includes RNA having a sequence from base 694 to base 714 in vegf-A mRNA of SEQ ID NO. 1.
A vegf-A mRNA binding agent according to the present invention is a binding agent that can bind to vegf-A mRNA. The vegf-A mRNA binding agent contains a nucleic acid, and the nucleic acid includes RNA having a sequence from base 462 to base 481 in p53 mRNA of SEQ ID NO. 2.
A screening method according to the present invention is a method for screening an expression promoting agent for promoting expression of p53.
The method includes the following steps (A) to (C).

(A) bringing the p53 mRNA binding agent of the present invention and the vegf-A RNA binding agent of the present invention into contact with each other in the presence of a candidate substance;
(B) detecting whether binding between the p53 mRNA binding agent and the vegf-A RNA binding agent is inhibited by the candidate substance; and
(C) selecting the candidate substance that inhibited the binding between the p53 mRNA binding agent and the vegf-A RNA binding agent as a p53 expression promoting agent.

Hereinafter, RNA transcribed from the vegf-A gene is referred to as "vegf-A RNA". The vegf-A RNA encompasses, for example, mRNA transcribed from the vegf-A gene. Also, mRNA transcribed from the p53 gene is referred to as "p53 mRNA".
The inventors of the present invention conducted a diligent study in order to identify the cause of the decrease in the amount of p53 mRNA expressed in tumor cells. As a result, it was revealed that RNA (vegf-A RNA) of a vascular endothelial growth factor A (VEGF-A) is involved in the expression of p53 mRNA. Then, as a result of a further detailed study, they found that, among RNAs transcribed from the vegf-A gene, RNA transcribed from exon 1 of the vegf-A gene suppresses the expression of p53 mRNA by binding to the p53 mRNA. Based on this finding, they found that, by inhibiting the binding between the RNA transcribed from the exon 1 of the vegf-A gene and the p53 mRNA, the decrease in the expression of p53 mRNA is avoided and the expression of p53 (transcription of p53 mRNA and translation of p53 protein) is promoted. Thus, the inventors of the present invention achieved the present invention. As described above, according to the present invention, the expression of p53 can be promoted, whereby the transcription of genes that are involved in apoptosis and transcribed in a p53-dependent manner can be promoted. Accordingly, it is possible to promote the progress of the apoptosis of a target cell in the end. In particular, in the medical field, cell apoptosis is utilized in preventing the malignant progression of tumors and treatments for cancers, and hence, it can be said that the present invention can serve as useful means in the treatments for cancers etc. Furthermore, it has been known that, in tumor cells in which the amount of the expressed p53 is decreased, the sensitivity to an anticancer agent is lowered, and the tumor cells show resistance to the anticancer agent. However, according to the present invention, it is possible to enhance the sensitivity of a cell to an anticancer agent. Thus, it becomes possible to allow conventionally known anticancer agents to act efficiency As specifically described above, it can be said that the present invention is a very beneficial technology in the medical field including treatments for cancers etc. By the way, the decrease in the amount of expressed p53 mRNA is considered to be caused by the following mechanism. That is, RNA transcribed from exon 1 of the vegf-A gene binds to the transcribed p53 mRNA to form a heteroduplex RNA, and the p53 mRNA forming the heteroduplex RNA is degraded in the translation process. It is to be noted, however, the present invention is not limited to this mechanism.

As opposed to a p53 protein serving as a tumor-suppression factor, a VEGF-A protein is involved in the neogenesis of tumor vessels and also functions as a survival factor of tumor cells. Thus, the VEGF-A protein is known to play a key role in tumor development. On this account, heretofore, the technology for suppressing malignant progression of tumors by suppressing the expression of the VEGF-A protein has been developed. Specifically, suppressing the expression of the VEGF-A protein by siRNA targeting exons 2, 3, 4, and 5, which are protein-coding regions, in vegf mRNA has been reported. However, it has not been reported that RNA is involved in the decrease in the amount of expressed p53 mRNA or that RNA transcribed from exon 1 of the vegf-A gene is involved in the same.

Brief Description of Drawings

[FIG. 1] FIG. 1 shows the results obtained in Example A1 of the present invention. FIG. 1A are photographs showing the expression of vegf mRNA in each type of cells; FIG. 1B is a graph showing the proportion (%) of apoptosis in each type of the cells treated with an anticancer agent 5-FU; FIG. 1C shows photographs showing the expression of p53 mRNA and the p53 protein in the cells expressing vegf siRNA; and FIG. 1D shows photographs showing the expression of mRNAs of p53 target genes in the cells expressing vegf siRNA.

[FIG. 2] FIG. 2 shows the results obtained in Example A2 of the present invention. FIG. 2A is a schematic view showing various plasmid constructs;

FIG. 2B is a photograph showing the expression of p53 mRNA in each type of cells, and FIG. 2C is a photograph showing the result obtained regarding the p53 protein in each type of the cells.

[FIG. 3] FIG. 3 shows the results obtained in Example A3 of the present invention. FIG. 3A is a schematic view showing various plasmid constructs;

FIG. 3B shows photographs showing the result obtained regarding p53 mRNA in each type of cells; and FIG. 3C shows photographs showing the expressions of p53 mRNA, the p53 protein, and the L-VEGF protein in each type of the cells.

[FIG. 4] FIG. 4 is directed to a reference example of the above-described Example A3 of the present invention. FIG. 4 are photographs showing the expression of p53 mRNA in H1299 cells cotransfected with plasmids expressing chimeric mRNA composed of 5' UTR of each kind of genes and CAT mRNA and plasmids expressing p53.

[FIG. 5] FIG. 5 shows the results obtained in Example A4 of the present invention. FIG. 5A shows photographs showing the expression of endogenous p53 mRNA in each type of cells; FIG. 5B shows photographs showing the expression of endogenous p53 protein in each type of the cells; and FIG. 5C shows photographs showing the expressions of noxa mRNA, bax mRNA, and p21 mRNA.

[FIG. 6] FIG. 6 shows the results obtained in the above-described Example A4. FIG. 6D is a graph showing the proportion (%) of apoptosis in each type of cells relative to the change in the concentration of 5-FU; and FIG. 6E is a graph showing the change in the proportion (%) of apoptosis with time in each type of the cells treated with 5-FU.

[FIG. 7] FIG. 7 shows the results obtained in Example A5 of the present invention. FIG. 7A is a photograph showing the presence of endogenous p53 mRNA and a degradation product thereof in each type of cells; FIG. 7B shows photographs showing the expression of p53 mRNA in each type of the cells; and FIG. 7C is a photograph showing the association of vegf 5' UTR and p53 mRNA *in vitro*.

[FIG. 8] FIG. 8 shows the results obtained in the above-described Example A5. FIG. 8D shows photographs showing the presence of p53 mRNA and a degradation product thereof in a cell-free protein synthesis reaction solution under the coexistence of vegf 5' UTR; and FIG. 8E is an autoradiography showing the synthesis of p53 protein in a cell-free protein reaction solution under the coexistence of vegf 5' UTR.

[FIG. 9] FIG. 9 is a schematic view showing various plasmid constructs in Example A6 of the present invention.

[FIG. 10] FIG. 10 shows photographs showing the presence of vegf IresRNA in various cancer cells in Example A6 of the present invention.

[FIG. 11] FIG. 11 shows the results obtained in the above-described Example A6. FIGs. 11A and 11B are photographs showing the expression of p53 mRNA in each type of the cells.

[FIG. 12] FIG. 12 shows the results obtained in Example A7 of the present invention. FIG. 12A shows photographs of mice to which transfected cells expressing vegf-A 5' UTR were xenografted; and FIG. 12B shows photographs of mice to which transfected cells expressing mutant-type vegf-A 5' UTR were xenografted.

[FIG. 13] FIG. 13 is a graph showing the change in tumor volume with time in the mice to which the transfected cells were xenografted in the above-described Example A7 of the present invention.

[FIG. 14] FIG. 14 is a graph showing the proportion (%) of apoptosis in various vegf 5' UTR-expressing cells treated with various anticancer agents in Example A8 of the present invention.

[FIG. 15] FIG. 15 a graph showing the proportion (%) of apoptosis in various siRNA-expressing cells treated with various anticancer agents in Example A9 of the present invention.

[FIG. 16] FIG. 16 is a graph showing a relative survival rate (%) of various siRNA-expressing cells treated with various anticancer agents in Example A10 of the present invention.

[FIG. 17] FIG. 17 shows the results obtained in Example A11 of the present invention. FIG. 17A is a schematic view showing various plasmid constructs; and FIG. 17B is a photograph showing the expression level of p53 mRNA.

[FIG. 18] FIG. 18 shows the results obtained in Example B1 of the present invention. FIG. 18A shows photographs showing the expression of vegf IresRNA in cells transfected with various expression vectors; FIG. 18B is a graph showing the expression of p53 mRNA in the transfected cells; FIG. 18C shows photographs showing the expression of p53 protein in the transfected cells; FIG. 18D is a graph showing the proportion (%) of apoptosis in the transfected cells treated with various anticancer agents; and FIG. 18E is a graph showing the change in tumor volume with time in the transfected cells.

[FIG. 19] FIG. 19 shows the results obtained in Example B1 of the present invention. FIG. 19F shows photographs showing tumor in the cell.

[FIG. 20] FIG. 20 is a graph showing the change in tumor volume with time in cells expressing siRNA against vegf mRNA in Example B2 of the present invention.

[FIG. 21] FIG. 21 shows the results obtained in Example B3 of the present invention. FIG. 21A shows a graph and photographs showing the expressions of vegf mRNA and vegf IresRNA in cells expressing siRNA against vegf mRNA; FIG. 21B is a graph showing the proportion (%) of apoptosis in the cells expressing siRNA; FIG. 21C is a graph showing the expression of p53 mRNA in the cells expressing siRNA; and FIG. 21D shows photographs showing the expression of p53 protein in the cells expressing siRNA.

[FIG. 22] FIG. 22 shows the results obtained in Example B3 of the present invention. FIG. 22E shows graphs showing the expression of mRNA of p53 target genes in cells expressing siRNA; and FIG. 22F shows photographs showing the expressions of various RNAs in cells exposed to various stresses.

[FIG. 23] FIG. 23 shows the results obtained in Example B4 of the present invention. FIG. 23A shows photographs showing the expressions of p53 mRNA and vegf IresRNA in cells transfected with various expression vectors, and FIGs. 23B and 23C show regions where p53 mRNA and vegf IresRNA interact with each other.

[FIG. 24] FIG. 24 shows the results obtained in Example B4 of the present invention. FIG. 24D shows the secondary structure of vegf IresRNA; FIG. 24E shows regions where deletion is caused artificiality in vegf IresRNA; FIG. 24F shows photographs showing the expression of RNA in cells expressing partially-deleted vegf IresRNA, and FIG. 24G shows photographs showing the bindings between various vegf IresRNAs and p53 mRNA by RNA pull-down assay.

[FIG. 25] FIG. 25 shows the results obtained in Example C1 of the present invention. FIG. 25A shows photographs showing the expression of p53 mRNA in cells transfected with p53 decoys, and FIG. 25B is a graph showing the survival rate of the cells.

[FIG. 26] FIG. 26 shows the results obtained in Example C2 of the present invention. FIG. 26A shows photographs showing the expression of p53 mRNA in cells transfect with antisenses for vegf IresRNA, and FIG. 26B is a graph showing the survival rate of the cells.

## Best Mode for Carrying out the Invention

In the present invention, "expression of p53" means the expression of p53 mRNA (i.e., transcription) directly, but it also includes the expression of p53 proteins (i.e., translation) indirectly. In the present invention, mRNA may be either a mRNA precursor (a primary transcript mRNA) or a mature RNA after being subjected to processing (splicing). Furthermore, RNA may be a partial sequence of the mRNA precursor or a partial sequence of the mature RNA.

In the present invention, vegf-A is a vascular endothelial growth factor A. In the present invention, SEQ ID NO. 1 is a complete sequence of a mature mRNA transcribed from the human vegf-A gene. That is, it is a full-length mature mRNA including untranslated regions except introns. In SEQ ID NO. 1, a region from base 1 to base 1104 is an "exon 1 region", and a region from base 1 to base 1038 is a "5' UTR region". A region from base 600 to base 879 in SEQ ID NO. 1 is a region expressed as a small temporal RNA. Hereinafter, RNA of this region is referred to as vegf IresRNA or IresRNA. The 5' UTR and IresRNA are untranslated RNAs. An untranslated region in the exon 1 region means a region upstream of the initiation codon (AUG from base 1039 in SEQ ID NO. 1). In SEQ ID NO. 1, a region from base 1039 to base 1041 is the "initiation codon (aug)", and a region from base 1684 to base 1686 is the "termination codon (uga)". With regard to this mature mRNA sequence, the sequence downstream from the 8th base, for example, is registered under NCBI Accession No. NM_003376. Note here that the sequence corresponding to SEQ ID NO. 1 in which "u" is represented by "t" is a sequence ofcDNAofthe vegf-A gene. On the other hand, SEQ ID NO. 45 is a partial sequence of a primary transcript of the human vegf-A gene, i.e., a partial sequence of a mRNA precursor including introns. In SEPA ID NO. 45, a region from base 1 to base 1104 is the same as that in SEQ ID NO. 1, and a region downstream from the 1105th base is a partial sequence of "intron 1" on the 5'-end side. Note here that the sequence corresponding to SEQ ID NO. 45 in which "u" is represented by "t" is a partial sequence of a genomic DNA of the vegf-A gene, and it is registered under NCBI Accession No. AL 136131.15, for example. In the present invention, the sequence of vegf-A mRNA is not limited to the sequence of SEQ ID NO. 1, and examples thereof may include sequences having various kinds of polymorphisms such as SNP.

SEQ ID NO. 2 shows a complete sequence of a mature mRNA transcribed from the human p53 gene. That is, it is a full-length mature mRNA including untranslated regions except introns. This complete sequence is registered under Ensemble Transcript ID: ENST 00000269305, for example. In SEQ ID NO. 2, a region from base 252 to base 254 is the "initiation codon (aug)", and a region from base 1431 to base 1433 is the "termination codon (uga)". In the present invention, the sequence of p53 mRNA is not limited to the sequence of SEQ ID NO. 2, and examples thereof may include sequences having various kinds of polymorphisms such as SNP

<p53 expression promoting method>

The p53 expression promoting method according to the present invention is, as described above, a method for promoting expression of a p53 gene in a cell, wherein the expression of the p53 gene is promoted by inhibiting binding between RNA transcribed from exon 1 of a vegf-A gene and mRNA transcribed from the p53 gene. The step of inhibiting the binding hereinafter also is referred to as a step (a).

As described above, according to the present invention, suppression of the p53 expression by vegf-A RNA can be suppressed by inhibiting the binding between the vegf-A RNA and the p53 mRNA. Thus, the present invention also can be referred to as "a method for inhibiting suppression of p53 expression", "a method for restoring p53 expression", or "a method for maintaining p53 expression". In the present invention, RNA transcribed from exon 1 of the vegf-A gene, whose binding with p53 mRNA is inhibited, may be, for example, RNA transcribed from the whole of the exon 1, RNA transcribed from a region including the exon 1, RNA transcribed from a partial region of the exon 1, or RNA transcribed from a region including a partial region of the exon 1.

In the step (a) of the present invention, a method for inhibiting the binding between RNA transcribed from the exon 1 of the vegf-A gene and the mRNA transcribed from the p53 gene may be, for example, a method including at least one step selected from the group consisting of the following steps (a1) to (a3). In the present invention, the method may include any one of these steps or two or more of these steps. Furthermore, the two or more steps may be performed simultaneously or separately, for example.

(a1) the step of degrading the RNA transcribed from the exon 1 of the vegf-A gene
(a2) the step of inhibiting transcription of RNA from the exon 1 of the vegf-A gene
(a3) the step of causing a nucleic acid different from the mRNA transcribed from the p53 gene to bind to the RNA transcribed from the exon 1 of the vegf-A gene, thereby inhibiting the binding between the RNA and the mRNA transcribed from the p53 gene

First, the step (a1) will be described. The step (a1) only requires that the transcribed RNA is decreased, for example. The mechanism for achieving it is not particularly limited, and examples thereof include degradation. In the present invention, degradation encompasses cleavage, for example. According to the step (a1), even if RNA is transcribed from the exon 1 of the vegf-A gene, the transcribed RNA is degraded (or cleaved), so that, consequently, the amount of expressed RNA (the amount of transcribed RNA) can be suppressed. Thus, it is possible to suppress the binding of the RNA transcribed from the exon 1 of the vegf-A gene to the p53 mRNA, whereby suppression of expression of p53 mRNA is prevented and thus expression of p53 is promoted.

In the step (a1), RNA to be degraded hereinafter is referred to as a target RNA, and a region thereof is referred to as a target region. In the step (a1), the degradation of the RNA transcribed from the exon 1 of the vegf-A gene may be the degradation of the whole of the exon 1 RNA in the vegf-A mRNA or the degradation of a part of the exon 1 RNA, for example. That is, in the step (a1), the target RNA may be the whole of the exon 1 RNA or a part of the exon 1 RNA. Furthermore, the target RNA may be RNA consisting of a region including the exon 1 RNA, RNA having a partial sequence of the exon 1 RNA, or RNA consisting of a region including a partial sequence of the exon 1, in RNA transcribed from the vegf-A gene.

The target RNA preferably is RNA consisting of the whole or a part of a sequence at least from base 600 to base 879 in SEQ ID NO. 1, for example. That is, the target RNA preferably is the whole or a part of the IresRNA present in the exon 1 RNA of the vegf-A mRNA. Furthermore, the target RNA may be RNA consisting of a region including the IresRNA in the vegf-A mRNA, or may be RNA consisting of a region including a part of the IresRNA. The number of bases in the partial sequence of the IresRNA in the target RNA is, for example, 100 to 150, preferably 80 to 100, and more preferably 20 to 40. Also, the partial sequence of the IresRNA preferably is, for example, RNA consisting of a sequence from base 694 to base 714, from base 693 to base 714, from base 691 to base 703, or from base 711 to base 723 in SEQ ID NO. 1, or sequences including the above-described RNAs. It is to be noted, however, the present invention is not limited thereto.

Specific examples of the target RNA will be given below. One kind of RNA selected therefrom may be used as the target RNA or two of more kinds of them may be used as the target RNAs. Also, RNA consisting of a region including either of these RNAs may be used as the target RNA. It is to be noted, however, the present invention is not limited thereto.

(1) RNA having a sequence from base 600 to base 879 in SEQ ID NO. 1
(2) RNA having a sequence from base 659 to base 879 in SEQ ID NO. 1
(3) RNA having a sequence from base 659 to base 780 in SEQ ID NO. 1
(4) RNA having a sequence from base 659 to base 752 in SEQ ID NO. 1
(5) RNA having a sequence from base 659 to base 745 in SEQ ID NO. 1
(6) RNA having a sequence from base 694 to base 714 in SEQ ID NO. 1
(7) RNA having a sequence from base 693 to base 714 in SEQ ID NO. 1
(8) RNA having a sequence from base 691 to base 703 in SEQ ID NO. 1
(9) RNA having a sequence from base 711 to base 723 in SEQ ID NO. 1
(10) RNA having a sequence from base 16 to base 780 in SEQ ID NO. 1
(11) RNA having a sequence from base 659 to base 917 in SEQ ID NO. 1
(12) RNA having a sequence from base 526 to base 1104 in SEQ ID NO. 1
(13) RNA having a sequence from base 526 to base 1216 in SEQ ID NO. 45
(14) 5' UTR RNA in vegf mRNA
(15) exon 1 RNA in vegf mRNA
(16) vegf mRNA

The RNA described in (1) is IresRNA, the RNAs described in (2) to (9) are partial sequences of IresRNA, the RNAs described in (10) and (11) are sequences including a part of IresRNA, the RNAs described in (12) to (16) are sequences including the whole of IresRNA. A region from base 16 to base 780 in the RNA described in (10) is registered as a sequence with an unknown function under NCBI Accession No. BC 019867, and a region from base 526 to base 1216 in the RNA described in (12) is registered under NCBI Accession No. BC 011177. The 5' UTR RNA described in (14) is, as described above, a region from 1 to 1038 in SEQ ID NO. 1, and the exon 1 RNA described in (15) is a region from 1 to 1104 in SEQ ID NO. 1. The vegf-A mRNA described in (16) may be either a mature mRNA or a mRNA precursor. Among the above-described target RNAs, it is preferable to degrade, in particular, RNA consisting of a region from base 659 to base 879 described in (2), RNA consisting of a region from base 694 to base 714 described in (6), RNA consisting of a region from base 693 to base 714 described in (7), RNA consisting of a region from base 691 to base 703 described in (8), or RNA consisting of a region from base 711 to base 723 described in (9) in the IresRNA, or RNAs respectively consisting of regions including the above-described RNAs in the vegf-A mRNA.

Examples of the target RNA also include the following (17): (17) RNAs that have base sequences corresponding to the sequences of the RNAs described in (1) to (16), respectively, in which one or more bases are substituted, deleted, inserted, or added, and have a function of suppressing expression of p53 or a function of binding to p53 mRNA (or a function of forming a hybrid).

In the step (a1), a method for degrading the target RNA is not particularly limited, and a conventionally known technique used for the degradation of RNA can be employed. Specific examples include, for example, a method using a degradation nucleic acid such as siRNA, a siRNA precursor that releases siRNA when cleaved by ribonuclease, an antisense, or ribozyme, and a method using an expression vector that expresses the degradation nucleic acid. By degrading the target RNA with the degradation nucleic acid, the binding between the RNA transcribed from the exon 1 of the vegf-A gene and the p53 mRNA is inhibited. Thus, in the present invention, the degradation nucleic acid and the expression vector that expresses the degradation nucleic acid also are referred to as "binding inhibitors". As described above, these binding inhibitors degrade the target RNA to inhibit the binding between the RNA transcribed from the exon 1 of the vegf-A gene and the p53 mRNA, so that, consequently, the expression of p53 can be promoted. Accordingly, the binding inhibitors also can be referred to as p53 expression promoting agents for promoting the expression of p53. These binding inhibitors will be described later as p53 expression promoting agents according to the present invention.

A treatment method with the binding inhibitor is not particularly limited. The binding inhibitor can be transfected into a target cell directly, for example. Furthermore, in the case where the binding inhibitor is an expression vector that expresses the degradation nucleic acid, the expression vector may be transfected into a target cell, and the binding inhibitor may be expressed from the expression vector in the cell.

Next, the step (a2) will be described. In the step (a2), it is only required that transcription of RNA from the exon 1 of the vegf gene is inhibited, and the mechanism for achieving it is not particularly limited. According to the step (a2), transcription of RNA from the exon 1 of the vegf-A gene itself is inhibited, so that the binding of the RNA to the p53 mRNA can be suppressed. It is considered that this prevents the suppression of the expression of p53 mRNA, so that the expression of p53 can be promoted consequently. In the step (a2), "inhibition of transcription of RNA" encompasses, for example, not only the complete stop of the transcription but also the decrease in the amount of the transcription. Furthermore, "inhibition of transcription of RNA" encompasses, for example, transcribing RNA having no function of suppressing the expression of p53, RNA having no function of forming a hybrid with p53 mRNA, or RNA having an inferior function of suppressing the expression of p53 or forming the hybrid with p53 mRNA, through substitution, deletion, insertion, or addition of one or more bases in the vegf-A gene.

In the step (a2), RNA to be subjected to the inhibition of transcription hereinafter is referred to as a target RNA, and a region thereof is referred to as a target region. The target RNA in the step (a2) is the same as that in the step (a1). Specifically, in the step (a2), inhibition of transcription of RNA from the exon 1 of the vegf-A gene may be, for example, inhibition of transcription of the whole of the exon 1 RNA or inhibition of transcription of a part of the exon 1 RNA. That is, in the step (a2), the target RNA may be the whole of the exon 1 RNA or a part of the exon 1 RNA. Furthermore, the target RNA may be RNA consisting of a region including the exon 1 RNA or RNA consisting of a region including a part of the exon 1 RNA, in vegf-A mRNA.

The target RNA preferably is RNA consisting of the whole or a part of a sequence at least from base 600 to base 879 in SEQ ID NO. 1. That is, the target RNA preferably is the whole or a part of the IresRNA present in the exon 1 RNA of the vegf-A mRNA. Furthermore, the target RNA may be RNA consisting of a region including the IresRNA in the RNA transcribed from the vegf-A mRNA, or may be RNA consisting of a region including a part of the IresRNA. The number of bases in the partial sequence and specific examples of the sequence are as described above, for example. Specific examples of the target RNA include, as in the case of the step (a1), the RNAs described in (1) to (17), for example. It is to be noted, however, the target RNA is not limited thereto.

In the step (a2), a method for inhibiting the transcription of the target RNA is not particularly limited, and a conventionally known technique used for inhibition of transcription of RNA can be employed. Specific examples include, for example, a method using a transcription-inhibiting nucleic acid such as siRNA, a siRNA precursor that releases siRNA when cleaved by ribonuclease, an antisense, ribozyme, or a decoy nucleic acid, and a method using an expression vector that expresses the transcription-inhibiting nucleic acid. Specifically, for example, by inhibiting a promoter activity with siRNA, an antisense, or ribozyme, each of which targets a sequence of a promoter region of the vegf-A gene, or a decoy nucleic acid including a sequence of a promoter region of the vegf-A gene, or the like, the transcription of the target RNA from the vegf-A gene can be suppressed. By inhibiting transcription of the target RNA with the transcription-inhibiting nucleic acid, the binding between the RNA transcribed from the exon 1 of the vegf- A gene and the p53 mRNA is inhibited. Thus, in the present invention, the transcription-inhibiting nucleic acid and the expression vector that expresses the transcription-inhibiting nucleic acid also are referred to as "binding inhibitors", as described above regarding the step (a1). As described above, these binding inhibitors inhibit the transcription of the target RNA to inhibit the binding between the RNA from the exon 1 of the vegf-A gene and the p53 mRNA, so that, consequently, the expression of p53 can be promoted. Accordingly, the binding inhibitors also can be referred to as p53 expression promoting agents for promoting the expression of p53, as described above regarding the step (a1). These binding inhibitors will be described later as p53 expression promoting agents according to the present invention. A treatment method with the binding inhibitor is not particularly limited, and may be the same as that in the step (a1), for example.

Next, the step (a3) will be described. In the step (a3), for example, to the RNA transcribed from the exon 1 of the vegf-A gene in a cell, a nucleic acid different from the p53 mRNA transcribed in the cell may be caused to bind. The different nucleic acid hereinafter is referred to as a decoy nucleic acid.

By causing the decoy nucleic acid to bind to the RNA transcribed from the exon 1 of the vegf-A gene to mask the binding site of the RNA with the p53RNA as described above, the binding between the RNA and the p53 mRNA can be inhibited. This allows the suppression of the expression of p53 mRNA by the vegf-A RNA to be inhibited, whereby the expression of p53 can be promoted. Also, by using an expression vector that expresses the decoy nucleic acid, the decoy nucleic acid expressed from the expression vector in a cell may be caused to bind to the RNA transcribed from the exon 1 of the vegf-A gene. Since the decoy nucleic acid and the vector expressing the decoy nucleic acid can promote the expression of p53 as described above, they also can be referred to as p53 expression promoting agents. They will be described later as p53 expression promoting agents according to the present invention.

A treatment method with the decoy nucleic acid is not particularly limited, and the decoy nucleic acid can be transfected into a target cell directly, for example. Furthermore, in the case where an expression vector that expresses the decoy nucleic acid is used, the expression vector may be transfected into a target cell, and the decoy nucleic acid may be expressed from the expression vector in the cell.

As described above, according to the present invention, by promoting (restoring) the expression of p53, transcription of various genes transcribed in a p53-dependent manner is promoted, whereby cell apoptosis can be promoted. Thus, although a cell to which the present invention is applied is not particularly limited, it preferably is a cell for which the progress of apoptosis is desired. Specifically, for example, it is desirable to apply the present invention to a cell in which the amount of expressed p53 mRNA is decreased, in particular, a tumor cell. Specific examples of the cell include, for example, a liver cancer cell, a large bowel cancer cell (a colon cancer cell), a lung cancer cell, a breast cancer cell, a kidney cancer cell, an ovarian cancer cell, a prostate cancer cell, a skin cancer cell, and a stomach cancer cell. The origin of the cell is not particularly limited, and examples thereof include humans and animals such as mammals other than humans.

The cell may be a cell collected from a living organism or a cell in a living organism, for example. Thus, the p53 expression promoting method according to the present invention may be applied to a target cell or a target tissue *in vitro*, or may be applied to a target cell or a target tissue in a living organism *in vivo*, for example. Also, the method may be applied to a target cell or a target tissue *ex vivo*.

<p53 expression promoting agent>

For example, as described above, the p53 expression promoting agent according to the present invention contains at least one binding inhibitor that inhibits the binding between the RNA transcribed from the exon 1 of the vegf-A gene and the mRNA transcribed from the p53 gene. The binding inhibitor is selected from the group consisting of the following (A1) to (A4). The p53 expression promoting agent according to the present invention also can be referred to as a "p53 expression restoring agent" or a "p53 expression maintaining agent".

(A1) at least one binding inhibitor selected from the group consisting of siRNA, a siRNA precursor that releases siRNA when degraded by ribonuclease, an antisense, and ribozyme, each of which degrades the RNA transcribed from the exon 1 of the vegf-A gene

(A2) at least one binding inhibitor selected from the group consisting of siRNA, a siRNA precursor that releases siRNA when degraded by ribonuclease, an antisense, ribozyme, and a decoy nucleic acid, each of which inhibits transcription of RNA from the exon 1 of the vegf-A gene

(A3) a binding inhibitor that contains a nucleic acid different from the mRNA transcribed from the p53 gene and binds to the RNA transcribed from the exon 1 of the vegf-A gene

(A4) a binding inhibitor that contains an expression vector in which DNA coding the at least one binding inhibitor selected from the group consisting of (A1) to (A3) is inserted

The p53 expression promoting agent according to the present invention may contain one kind or two or more kinds of binding inhibitors selected from the following (A1) to (A4), for example.

Technology of siRNA, an antisense, ribozyme, and a decoy nucleic acid has been established, for example, as means for degrading RNA transcribed from a gene or for inhibiting transcription of RNA from a gene. Accordingly, when conducting the present invention, those skilled in the art can design siRNA, an antisense, ribozyme, a decoy nucleic acid, and the like for degradation of a target RNA or inhibition of transcription of a target RNA, based on common general technical knowledge. Furthermore, components of the siRNA, the antisense, the ribozyme, the decoy nucleic acid, and the like are not particularly limited. They may be, for example, naturally-occurring nucleic acids, artificial nucleic acids such as PNA, or the like, and they are by no means limited (hereinafter the same).

First, the binding inhibitor described in (A1) will be described. This binding inhibitor corresponds to, for example, the binding inhibitor described in the step (a1) of the p53 expression promoting method according to the present invention. Target RNAs degraded by this binding inhibitor are the same as those described in the step (a1) of the p53 expression promoting method according to the present invention.

(A1) at least one binding inhibitor selected from the group consisting of siRNA, a siRNA precursor that releases siRNA when degraded by ribonuclease, an antisense, and ribozyme, each of which degrades the RNA transcribed from the exon 1 of the vegf-A gene

The p53 expression promoting agent according to the present invention may contain, as the binding inhibitor of (A1), any of the siRNA, the siRNA precursor that releases siRNA when cleaved by ribonuclease, the antisense, and the ribozyme. One kind of the binding inhibitor may be used, or two or more kinds of the binding inhibitors may be used in combination.

As the binding inhibitor, siRNA is preferable, for example. The siRNA generally is a double-stranded RNA composed of an antisense strand complementary to RNA such as mRNA and a sense strand complementary thereto. The length of each of the strands of the siRNA is not particularly limited. Generally, each of the strands is an oligonucleotide consisting of 19 to 25 bases, preferably 19 bases. The siRNA may have an overhang, for example, at the 3' end of each of the strands. In this case, the siRNA has a shape in which the 3' end of each of the strands is protruding. The length of the overhang is not particularly limited, and generally, the overhang consists of 2 bases. The sequence of the overhang is not particularly limited, and, for example, in an antisense strand, it may be a sequence complementary to RNA to which it binds, and in a sense strand, it may be a sequence complementary to the antisense strand, i.e., the same sequence as that of RNA to which the antisense strand binds. It also may be other sequences, examples of which include sequences such as uu, au, ag, gc, and cc. The sense strand corresponding to the antisense strand may be such that, for example, the sequence inside thereof is shorter than the antisense strand by 2 bases.

It is not necessary that the sequence of the antisense strand is perfectly complementary to, for example, the sequence of RNA to which it binds. However, the homology thereof to the mRNA is, for example, 70% or more, preferably 80% or more, more preferably 90% or more, particularly preferably 95% or more, and most preferably 100% (perfect match). Also, in the antisense strand, it is not necessary that the sequence of the oligonucleotide excluding the overhang is perfectly complementary to, for example, the sequence of RNA to which it binds. However, the homology thereof to the mRNA is, for example, 70% or more, preferably 80% or more, more preferably 90% or more, particularly preferably 95% or more, and most preferably 100% (perfect match).

Furthermore, in vegf-A RNA, a region to which the siRNA binds is not limited to a region of the above-described target RNA. Generally, it has been known that cleavage of mRNA by siRNA occurs first in a region to which the siRNA has bound, which triggers cleavage in other regions, so that a target region is cleaved completely in the end. Accordingly, the siRNA may be designed so that, for example, it binds to a target region of vegf-A RNA or it binds to a region other than the target region. In the latter case, with the cleavage in the binding region of siRNAbeing a trigger, the target region (the target RNA) to be degraded also is degraded eventually. Thus, even if RNA is transcribed from the exon 1 of the vegf-A gene, the RNA is degraded consequently, so that the binding of the RNA to p53 mRNA is inhibited and the RNA no longer exhibits its function of suppressing the expression of p53.

The binding region of siRNA against vegf-A mRNA is not limited, as described above. However, it is preferable that the binding region is, for example, in the inside of a region of the above-described target RNA, and examples thereof include the inside of IresRNA, the inside of 5' UTR, the inside of exon 1 RNA, and the vicinity of the boundary between exon 1 RNA and intron 1RNA. Specific examples include, for example, the inside of the target RNAs described in (1) to (9) above (the inside of IresRNA) and the inside of the target RNAs described in (10) to (16) above. It is to be noted, however, the present invention is not limited thereto.

Specific examples of the antisense strand of siRNA will be given below. The overhang in each of the following antisense strands #1 to #10 is underlined. In the following #1 to #17, n is an arbitrary nucleic acid, which can be a, u, g, c, or t. It should be noted that, in siRNA, the overhang is optional, and, for example, it is not necessary that siRNA has an overhang (hereinafter the same). In the present invention, the antisense strand of siRNA is not limited thereto.

#1 5' -agcaaggcaaggcuccaaugcnn-3' (SEQ ID NO. 4) complementary to bases 1062-1082 in SEQ ID NO. 1
#2 5' -agagcagcaaggcaaggcuccnn-3' (SEQ ID NO. 6) complementary to bases1067-1087 in SEQ ID NO. 1
#3 5' -cacgaccgcuuaccuuggcaunn-3' (SEQ ID NO. 8) complementary to bases 1097-1117 in SEQ ID NO. 45
#4 5' -uagcacuucucgcggcuccgcnn-3' (SEQ ID NO. 10) complementary to bases 728-748 in SEQ ID NO. 1
#5 5' -cgagcuagcacuucucgcggcnn-3' (SEQ ID NO. 12) complementary to bases 733-753 in SEQ ID NO. 1
#6 5' -uggacgaaaaguuucagugcgacgcnn-3' (SEQ ID NO. 14) complementary to bases 644-668 in SEQ ID NO. 1
#7 5' -agaaguuggacgaaaaguuucagugnn-3' (SEQ ID NO. 16) complementary to bases 650-674 in SEQ ID NO. 1
#8 5' -gaaguuggacgaaaaguuucagugcnn-3' (SEQ ID NO. 18) complementary to bases 649-673 in SEQ ID NO. 1
#9 5' -ggacgaaaaguuucagugcgacgccnn-3' (SEQ ID NO. 20) complementary to bases 643-667 in SEQ ID NO. 1
#10 5' -uuggacgaaaaguuucagugcgacgnn-3' (SEQ ID NO. 22) complementary to bases 645-669 in SEQ ID NO. 1
#11 5' -agaaguuggacgaaaaguuucagugnn-3' (SEQ ID NO. 61) complementary to bases 650-674 in SEQ ID NO. 1
#12 5' -aguuucagugcgacgccgcgagcccnn-3' (SEQ ID NO. 63) complementary to bases 635-659 in SEQ ID NO. 1
#13 5' -gaagcgagaacagcccagaaguuggnn-3' (SEQ ID NO. 65) complementary to bases 666-690 in SEQ ID NO. 1
#14 5' -agcaaggcaaggcuccaaugcacccnn-3' (SEQ ID NO. 67) complementary to bases 1058-1082 in SEQ ID NO. 1
#15 5' -agagcagcaaggcaaggcuccaaugnn-3' (SEQ ID NO. 69) complementary to bases 1063-1087 in SEQ ID NO. 1
#16 5' -cgagcuagcacuucucgcggcuccgnn-3' (SEQ ID NO. 71) complementary to bases 729-753 in SEQ ID NO. 1
#17 5' -cgcggaccacggcuccuccgaagcgnn-3' (SEQ ID NO. 73) complementary to bases 685-709 in SEQ ID NO. 1

Specific examples of the siRNA composed of an antisense strand and a sense strand will be given below. In the following examples, the underlined "nn" denotes an overhang, and an example of the sequence of the overhang is shown in the upper part of nn in the sense and in the lower part of nn in the antisense. Note here that, in siRNAs #1 to #17, for example, the overhang is optional in each of the antisense strand and the sense strand, and it is not necessary that these strands have an overhang. It is to be noted that, in the present invention, siRNA is not limited thereto.

siRKA #1

sense cguaaccucggaacggaacga-5' (SEQ ID NO. 4)
gu

antisense
gu or ca
;cauugga cuugccuugcunn-3' (SEQ ID NO. 3)

siRNA #2

sense 3' -nnccucggaacggaacgacgaga-5' (SEQ ID NO. 6)
ac or gu

antisense
gu or ca
5' -ggagccuug uugcugcucunn-3' (SEQ ID NO. 5)

siRNA #3

sense 3' -nnuacgguuccauucgccagcac-5' (SEQ ID NO. 8)
ac or gu

antisense
gu or ca
5' -augccaag aagcggucgugnn-3' (SEQ ID NO. 7)

siRNA #4

sense 3' -nncgccucggcgcucuucacgau-5' (SEQ ID NO. 10)
ac or gu

antisense
gu or ca
5' -gcggagccg agaagugcuann-3' (SEQ ID NO. 9)

siRNA #5

sense 3' -nncggcgcucuucacgaucgagc-5' (SEQ ID NO. 12)
ac or gu

antisenses
gu or ca
5' -gccgcgag gugcuagcucgnn-3' (SEQ ID NO. 11)

siRNA #6

sense 3' -nncgcagcgugacuuugaaaagcaggu-5' (SEQ ID NO. 14)
ua

antisense
ag
5' -gcgucgcacugaaacuuuucguccann-3' (SEQ ID NO. 13)

siRNA #7

sense 3' -nngugacuuugaaaagcagguugaaga-5' (SEQ ID NO. 16)
ua

antisenses
ag
5' -cacugaaacuuuucguccaacuucunn-3' (SEQ ID NO. 15)

(continued)

| | | |
|---|---|---|
| siRNA #8 | | |
| | sense | ugacuuugaaaagcagguugaag-5' (SEQ ID NO. 18) |
| | antisense | ag<br>acugaaacuuuucguccaacuucnn-3' (SEQ ID NO. 17) |
| siRNA #9 | | |
| | sense | 3' -nnccgcagcgugacuuugaaaagcagg-5' (SEQ ID NO. 20)<br>ua |
| | antisense | ag<br>5' -ggcgucgcacugaaacuuuucguccnn-3' (SEQ ID NO. 19) |
| siRNA #10 | | |
| | sense | 3' -nngcagcgugacuuugaaaagcagguu-5' (SEQ ID NO. 22)<br>ua |
| | antisense | ag<br>5' -cgucgcacugaaacuuuucguccaann-3' (SEQ ID NO. 21) |
| siRNA #11 | | |
| | sense | 5' -cacugaaacuuuucguccaacuucunn-3' (SEQ ID NO. 60) |
| | antisense | 3' -nngugacuuugaaaagcagguugaaga-5' (SEQ ID NO. 61) |
| siRNA #12 | | |
| | sense | 5' -gggcucgcggcgucgcacugaaacunn-3' (SEQ ID NO. 62) |
| | antisense | 3' -nncccgagcgccgcagcgugacuuuga-5' (SEQ ID NO. 63) |
| siRNA #13 | | |
| | sense | 5' -ccaacuucugggcuguucucgcuucnn-3' (SEQ ID NO. 64) |
| | antisense | 3' -nngguugaagacccgacaagagcgaag-5' (SEQ ID NO. 65) |
| siRNA #14 | | |
| | sense | 5' -gggugcauuggagccuugccuugcunn-3' (SEQ ID NO. 66) |
| | antisense | 3' -nncccacguaaccucggaacggaacga-5' (SEQ ID NO. 67) |
| siRNA #15 | | |
| | sense | 5' -cauuggagccuugccuugcugcucunn-3' (SEQ ID NO. 68) |
| | antisense | 3' -nnguaaccucggaacggaacgacgaga-5' (SEQ ID NO. 69) |
| siRNA #16 | | |
| | sense | 5' -cggagccgcgagaagugcuagcucgnn-3' (SEQ ID NO. 70) |
| | antisense | 3' -nngccucggcgcucuucacgaucgagc-5' (SEQ ID NO. 71) |
| siRNA #17 | | |
| | sense | 5' -cgcuucggaggagccgugguccgcgnn-3' (SEQ ID NO. 72) |
| | antisense | 3' -nngcgaagccuccucggcaccaggcgc-3' (SEQ ID NO. 73) |

Each of these siRNAs may be transfected into a cell, for example, as a double-stranded siRNA having any one of the above-described sequences, or may be transfected into a cell, for example, as a siRNA expression vector that expresses any one of the above-described sequences, as described in the section of the binding inhibitor (A4) to be described later.

The binding inhibitor of (A1) may be a siRNA precursor that releases siRNA when degraded by ribonuclease, as described above. Examples of the siRNA precursor include a double-stranded RNA and a single-stranded RNA. In the former case, for example, the double-stranded RNA is cleaved by ribonuclease when it is transfected into a cell, whereby siRNA as described above is generated. Examples of the latter single-stranded RNA include a fold-back RNA precursor in which a plurality of hairpin structures are linked by self-annealing. For example, when this fold-back RNA is transfected into a cell, first, it is cleaved by Drosha, whereby a hairpin-shaped miRNA-like RNA precursor is generated. This is further cleaved by Dicer, whereby a double-stranded siRNA as described above is generated. Furthermore, the latter single-stranded RNA may be a hairpin-shaped miRNA-like RNA precursor, for example. For example, when this hairpin-shaped miRNA-like RNA precursor is transfected into a cell, it is cleaved by Drosha and Dicer, whereby siRNA as described above is generated. Also, the latter single-stranded RNA may be a hairpin-shaped siRNA precursor. In the latter case, the siRNA precursor transfected into a cell may have formed a hairpin structure previously or may form a hairpin structure in the cell, for example. The sequence of the siRNA precursor is not particularly limited, and can be designed as appropriate depending on the sequence of siRNA, the sequence of a region to which the antisense strand of siRNA binds, and the sequence of the target RNA, for example. Examples of the ribonuclease include Dicer and Drosha (hereinafter the same).

The binding inhibitor described in (A1) above may be an antisense as described above. The antisense can be, for example, an antisense having a sequence complementary to the above-described target RNA. The antisense may be either DNA or RNA, for example. Examples of the mechanism for suppressing the expression by the antisense include: inhibition of initiation of transcription by triplex formation; inhibition of transcription by hybrid formation with a site at which an open loop structure is formed locally by RNA polymerase; and inhibition of transcription by hybrid formation with RNA that are being synthesized (Hirashima and Inoue; Shin Seikagaku Jikken Koza 2, Kakusan IV, Idenshi no Fukusei to Hatsugen (edited by the Japanese Biochemical Society, Tokyo Kagaku Dojin) pp. 319-347, 1993). The length of the antisense is not particularly limited. However, for example, it generally is such that the lower limit thereof is 15 bases or more, preferably 100 bases or more, and more preferably 500 bases or more, and the upper limit thereof is, for example, 5000 bases (5 kb) or less, preferably 2500 bases (2.5 kb) or less.

The binding inhibitor described in (A1) above may be ribozyme, as described above. Ribozyme means an RNA molecule having a catalytic activity, and examples of the ribozyme used in the present invention include those that cleave RNA in a site-specific manner. The ribozyme used in the present invention has, for example, a ribozyme activity of specifically cleaving the above-described target RNA. The sequence of the ribozyme is not particularly limited, and the ribozyme can be designed so that, for example, it has a sequence complementary to a sequence to be cleaved by the ribozyme. The kind of ribozyme is not particularly limited, and examples thereof include hammerhead ribozyme and hairpin ribozyme.

Next, the binding inhibitor described in (A2) above will be described. This binding inhibitor corresponds to, for example, the various kinds of binding inhibitors described in the step (a2) of the p53 expression promoting method according to the present invention. A target RNA degraded by this binding inhibitor is the same as the target RNA in the step (a2) of the p53 expression promoting method according to the present invention.

(A2) at least one binding inhibitor selected from the group consisting of siRNA, a siRNA precursor that releases siRNA when degraded by ribonuclease, an antisense, ribozyme, and a decoy nucleic acid, each of which inhibits transcription of RNA from the exon 1 of the vegf-A gene

The p53 expression promoting agent according to the present invention may contain, as the binding inhibitor of (A2), any of the siRNA, the siRNA precursor that releases siRNA when cleaved by ribonuclease, the ribozyme RNA, and the decoy nucleic acid. Furthermore, the siRNA, the siRNA precursor, the ribozyme, and the decoy nucleic acid can be designed, for example, in the same manner as that for the binding inhibitor of (A1), unless otherwise stated.

Preferably, the binding inhibitor is designed so that, for example, it inhibits the promoter activity of the vegf-A gene. This allows transcription from the exon 1 of the vegf-A gene to be suppressed. Examples of the binding inhibitor include an antisense that is complementary to a promoter region of the vegf-A gene and thus can bind to the region. According to such an antisense, by binding the antisense to the promoter of the vegf-A gene, its promoter activity is inhibited, so that, consequently, the transcription from the exon 1 of the vegf-A gene can be suppressed.

Next, the binding inhibitor described in (A3) above will be described. This binding inhibitor corresponds to, for example, the binding inhibitor composed of a decoy nucleic acid, described in the step (a3) of the p53 expression promoting method according to the present invention.

(A3) a binding inhibitor that contains a nucleic acid (a decoy nucleic acid) different from the mRNA transcribed from the p53 gene and binds to the RNA transcribed from the exon 1 of the vegf-A gene

The kind of the decoy nucleic acid is not particularly limited, and may be either DNA or RNA, for example. The sequence of the decoy nucleic acid is not particularly limited, and examples thereof include a sequence that can bind to a binding region of RNA transcribed from the exon 1 of the vegf-A gene with p53 mRNA. Specifically, the sequence may be, for example, a sequence complementary to a binding region of vegf-A RNA with p53 mRNA or a sequence complementary to a region including the binding region of vegf-A RNA with p53 mRNA, or a sequence consisting of a binding region of p53 mRNA with RNA transcribed from the exon 1 of the vegf-A gene or a sequence consisting of a region including the binding region. The number of bases in the decoy nucleic acid is not particularly limited, and is, for example, 25 to 50 bases, preferably 25 to 30 bases.
Specific examples of the decoy nucleic acid will be given below. One kind or two or more kinds of the binding inhibitors may be contained, for example. It is to be noted, however, the present invention is not limited thereto.

(D1) RNA having a sequence from base 462 to base 481 in SEQ ID NO. 2, or DNA having a sequence corresponding to the sequence of this RNA in which base u is substituted with base t;
(D2) RNA having a sequence from base 462 to base 482 in SEQ ID NO. 2, or DNA having a sequence corresponding to the sequence of this RNA in which the base u is substituted with base t
(D3) RNA having a sequence from base 462 to base 491 in SEQ ID NO. 2, or DNA having a sequence corresponding to the sequence of this RNA in which the base u is substituted with base t
(D4) RNA having a sequence from base 462 to base 505 in SEQ ID NO. 2, or DNA having a sequence corresponding to the sequence of this RNA in which the base u is substituted with base t
(D5) RNA having a sequence from base 252 to base 551 in SEQ ID NO. 2, or DNA having a sequence corresponding to the sequence of this RNA in which the base u is substituted with base t
(D6) RNA having a sequence complementary to a sequence from base 694 to base 714 in SEQ ID NO. 1, or DNA having a sequence corresponding to the sequence of this RNA in which the base u is substituted with base t
(D7) RNA having a sequence complementary to a sequence from base 693 to base 714 in SEQ ID NO. 1, or DNA having a sequence corresponding to the sequence of this RNA in which the base u is substituted with base t

5' -ccccgcgcggaccacggctcct-3' (SEQ ID NO. 74)

(D8) a nucleic acid that has a base sequence corresponding to the sequence of at lease one nucleic acid selected from the group consisting of (D1) to (D7), in which one or more bases are substituted, deleted, inserted, or added, and can bind to the RNA transcribed from the exon 1 of the vegf-A gene

Each of the RNAs and DNAs described in (D1) to (D5) above has the same sequence as a partial sequence of the p53 gene (RNA or DNA). Each of the RNAs and DNAs described in (D6) and (D7) above is an antisense having a sequence complementary to a partial sequence in the exon 1 of the vegf-A gene (RNA or DNA).
Next, the binding inhibitor described in (A4) above will be described. (A4) a binding inhibitor that contains an expression vector in which DNA coding the at least one binding inhibitor selected from the group consisting of (A1) to (A3) is inserted.
The binding inhibitor of (A4) may be, for example, an expression vector that expresses at least one of the above-described (A1) to (A3). When the expression vector is used, for example, by transfecting the expression vector into a cell, the binding inhibitor is expressed in the cell (for example, transcription of RNA or replication of DNA). There is no limitation on the systems for transfecting the expression vector and for expressing the coding sequence inserted in the expression vector, and conventionally known techniques can be employed. For example, in the case where siRNA is generated by the expression vector in a cell, it is possible to use a commercially available kit such as a siRNA expression system or a miRNA expression system.
The expression vector is obtained by, for example, inserting DNA coding any of the above-described various binding inhibitors to a vector. The vector is not particularly limited, and can be determined as appropriate depending on, for example, the kind of a cell into which the expression vector is transfected, a method for transfecting the expression vector, etc. Examples of the vector include a virus vector and a nonviral vector. The nonviral vector may be, for example, a vector that can express the above-described RNA in a target cell or *in vivo*. As specific examples, expression vectors such as pcDNA3.1 (Invitrogen), pZeoSV (Invitrogen), pBK-CMV (Stratagene), pCAGGS (Gene108,193-200 (1991)), and the like can be given, for example. Generally, the DNA may be inserted downstream of promoters of these vectors in such a manner that it can be expressed. Examples of the virus vector include retrovirus vectors, lentivirus vectors such as a human immunodeficiency virus (HIV) vector, adenovirus vectors, adeno associated virus vectors (AAV vectors), DNA viruses such as herpesvirus, vaccinia virus, poxvirus, poliovirus, Sindbis virus, Sendai virus, and SV40, and RNA viruses. Expression vectors can be produced by inserting the DNA to the above-described vectors based on a conventionally known method.

The expression vector further may include a regulatory sequence for regulating the expression of the above-described DNA. Examples of the regulatory sequence include: a promoter derived from cytomegalovirus (CMV); constitutive promoters such as Rous sarcoma virus (RSV), simian virus-40(SV-40), musculus β-actin promoter, and herpes simplex virus (HSV); tissue-specific promoters such as a thymidine kinase promoter; somatotropin regulation promoters; promoters under control of a lacoperon sequence; inducible promoters such as a zinc-induced metallothionein promoter; and regulation promoters. Such regulatory sequences may be arranged at or bound to a site where the expression of the gene can be regulated functionally, based on a conventionally known method. Other than the regulatory sequence, the expression vector may include, for example, an enhancer sequence, a polyadenylation signal, an origin of replication (ori) sequence, or the like.

Furthermore, the expression vector may include, for example, a sequence coding a selection marker such as a drug-resistant marker, a fluorescent protein marker, an enzyme marker, a cell surface receptor marker, or the like.

In the present invention, in the case where siRNA is generated in a cell, an expression vector that expresses the siRNA may be transfected into the cell, or an expression vector that expresses the siRNA precursor that releases siRNA when cleaved by ribonuclease may be transfected into the cell, for example. In the former case, for example, siRNA is generated in the cell. In the latter case, for example, the siRNA precursor generated in the cell forms a stem-loop through binding of complementary strands in a molecule, and further, as described above, it is cleaved by ribonucleases such as Drosha and Dicer, whereby the above-described siRNA is generated. The same applies to an expression vector that expresses an antisense RNA and to an expression vector that expresses ribonucleotide.

A method for transfecting the p53 expression promoting agent according to the present invention into a cell is not particularly limited, and a conventionally known method can be employed. In the case where the p53 expression promoting agent is transfected into a cell *in vitro*, examples of the transfection method include a calcium phosphate method, a polyethylene glycol method, a lipofection method, an electroporation method, nucleic acid transfection using ultrasonic waves, transfection using a gene gun, a DEAE-dextran method, and direct injection using a minute glass tube. Also, transfection by infection using the above-described virus vectors, and the like may be employed. Also, in the case where the p53 expression promoting agent according to the present invention is transfected into a cell or a tissue *in vivo*, a conventionally known method can be employed as the transfection method as appropriate depending on, for example, the kind of the target cell or tissue. Specific examples of the method include: intramuscular or intraperitoneal injection, oral administration, administration to subcutaneous tissues, administration using a gene gun, immersion, a hydrodynamic method, a cationic liposome method, and a method using atelocollagen. On the other hand, in the case where it is transfected *ex vivo,* examples of the transfection method include a method in which a target cell or tissue is collected from a subject, the p53 expression promoting agent according to the present invention is transfected into the collected cell or the like by any of the above-described methods, and then the transfected cell or the like is returned to the body of the subject.

The p53 expression promoting agent according to the present invention further may contain various kinds of additives such as the above-described binding inhibitor, an adjuvant for assisting the transfection of the expression vector into a cell, and the like. Examples of the adjuvant include Lipofectamine, liposome such as cationic liposome, and atelocollagen.

<Apoptosis promoting method and apoptosis promoting agent>

The apoptosis promoting method of the present invention is a method for promoting apoptosis of a cell. In this method, expression of p53 in the cell is promoted by the p53 expression promoting method according to the present invention.

In the present invention, it is preferable that the step of treating the cell with an anticancer agent further is included. Specifically, in addition to the step (a), i.e., the step of inhibiting the binding between RNA transcribed from exon 1 of the vegf-A gene and p53 mRNA in a cell, the step of treating the cell with an anticancer agent preferably further is included as the step (b). Although the order of the steps (a) and (b) is not particularly limited, it is preferable that, for example, after the cell is subjected to a treatment for inhibiting the above-described binding as the step (a), the treatment with an anticancer agent is performed with respect to the treated cell. Although the treatment for inhibiting the binding is not particularly limited, it preferably includes at least one of the above-described steps (a1) to (a3).

The kind of the anticancer agent is not particularly limited. Specific examples thereof include, for example, 5-fluorouracil (5-FU), cisplatin, doxorubicin, etoposide, leucovorin, mitomycin C, paclitaxel, vincristine, and camptothecin, (hereinafter the same).

The apoptosis promoting agent according to the present invention contains, as described above, the p53 expression promoting agent according to the present invention. It only is required that the apoptosis promoting agent according to the present invention contains the p53 expression promoting agent, and other compositions etc. are by no means limited. Furthermore, the apoptosis promoting agent according to the present invention can be used in the apoptosis promoting method according to the present invention, and a specific method for using it etc. are the same as those described for the above-described p53 expression promoting agent of the present invention. It is preferable that the apoptosis promoting agent according to the present invention further contains an anticancer agent.

< Method for enhancing sensitivity of cell to anticancer agent and enhancer>

The method for enhancing sensitivity to an anticancer agent according to the present invention is a method for enhancing sensitivity of a cell to an anticancer agent. In this method, expression of p53 in the cell is promoted by the p53 expression promoting method of the present invention. In the enhancement method according to the present invention, it is only required that, for example, the p53 expression promoting method of the present invention is performed with respect to a target cell prior to a treatment with an anticancer agent, and other steps are by no means limited. By conducting such a treatment, it is possible to enhance the sensitivity of the cell to the anticancer agent.

The enhancer according to the present invention is, as described above, an enhancer for enhancing sensitivity of a cell to an anticancer agent. The enhancer contains the p53 expression promoting agent of the present invention. It only is required that the enhancer of the present invention contains the p53 expression promoting agent, and other composition etc. are by no means limited. Furthermore, the enhancer according to the present invention can be used in the method for enhancing sensitivity to an anticancer agent according to the present invention, and a specific method for using the enhancer etc. are the same as those described for the above-described p53 expression promoting agent of the present invention.

<Method for treating cancer and anticancer agent>

The method for treating cancer according to the present invention includes the step of administering the p53 expression promoting agent of the present invention to a cancer cell that is *in vivo.* A subject to which the p53 expression promoting agent is administered is not particularly limited, and examples thereof include humans and animals such as mammals other than humans.

The anticancer agent according to the present invention contains the p53 expression promoting agent of the present invention, as described above. It is only required that the anticancer agent according to the present invention contains the p53 expression promoting agent, and other compositions etc. are by no means limited. The anticancer agent according to the present invention further may contain, for example, an anticancer agent(s) other than the above-described anticancer agent and a pharmaceutically acceptable component(s). Furthermore, the anticancer agent according to the present invention can be used in the method for treating cancer according to the present invention, and a specific method for using the anticancer agent is the same as that for the p53 expression promoting agent of the present invention.

<p53 mRNAbinding agent>

The p53 mRNAbinding agent according to the present invention is a binding agent that can bind to p53 mRNA. The p53 mRNA binding agent contains a nucleic acid, and the nucleic acid includes RNA having a sequence from base 694 to base 714 in vegf-A mRNA of SEQ ID NO. 1.

RNA contained in the nucleic acid may be, for example, RNA having the sequence from base 694 to base 714, or RNA having a sequence including a region from base 694 to base 714 in vegf-A mRNA. Examples of the RNA include RNAs described in (1) to (17) below. The nucleic acid may contain one kind of the RNA or two or more kinds of the RNAs.

(1) RNA having a sequence from base 600 to base 879 in SEQ ID NO. 1
(2) RNA having a sequence from base 659 to base 879 in SEQ ID NO. 1
(3) RNA having a sequence from base 659 to base 780 in SEQ ID NO. 1
(4) RNA having a sequence from base 659 to base 752 in SEQ ID NO. 1
(5) RNA having a sequence from base 659 to base 745 in SEQ ID NO. 1
(6) RNA having a sequence from base 694 to base 714 in SEQ ID NO. 1
(7) RNA having a sequence from base 693 to base 714 in SEQ ID NO. 1
(8) RNA having a sequence from base 691 to base 703 in SEQ ID NO. 1
(9) RNA having a sequence from base 711 to base 723 in SEQ ID NO. 1
(10) RNA having a sequence from base 16 to base 780 in SEQ ID NO. 1
(11) RNA having a sequence from base 659 to base 917 in SEQ ID NO. 1
(12) RNA having a sequence from base 526 to base 1104 in SEQ ID NO. 1
(13) RNA having a sequence from base 526 to base 1216 in SEQ ID NO. 45

(14) 5'UTR RNA in vegf mRNA
(15) exon 1 RNA in vegf mRNA
(16) vegf mRNA
(17) RNAs that have base sequences corresponding to the sequences of the RNAs described in (1) to (16), respectively, in which one or more bases are substituted, deleted, inserted, or added, and have a function of suppressing expression of p53 or a function of binding to p53 mRNA (or a function of forming a hybrid)

<vegf-A mRNA binding agent>

The vegf-A mRNA binding agent according to the present invention is a binding agent that can bind to vegf-A mRNA. The vegf-A mRNA binding agent contains a nucleic acid, and the nucleic acid includes RNA having a sequence from base 462 to base 481 in p53 mRNA of SEQ ID NO. 2.

RNA contained in the nucleic acid may be, for example, RNA having the sequence from base 462 to base 481, or RNA having a sequence including a region from base 462 to base 481 in p53 mRNA. Examples of the RNA include RNAs described in (I) to (VI) below. The nucleic acid may contain one kind of the RNA or two or more kinds of the RNAs.

(I) RNA having a sequence from base 462 to base 481 in SEQ ID NO. 2
(II) RNA having a sequence from base 462 to base 482 in SEQ ID NO. 2
(III) RNA having a sequence from base 462 to base 491 in SEQ ID NO. 2
(IV) RNA having a sequence from base 462 to base 505 in SEQ ID NO. 2
(V) RNA having a sequence from base 252 to base 551 in SEQ ID NO. 2
(VI) RNA that has a base sequence corresponding to the sequence of at least one RNA selected from the group consisting of (I) to (V), in which one or more bases are substituted, deleted, inserted, or added, and has a function of binding to RNA transcribed from exon 1 of vegf-A

<Screening method>

The screening method according to the present invention is a method for screening an expression promoting agent for promoting expression of p53. The method includes the following steps (A) to (C).

(A) bringing the p53 mRNA binding agent of the present invention and the vegf-A RNA binding agent of the present invention into contact with each other in the presence of a candidate substance;
(B) detecting whether binding between the p53 mRNA binding agent and the vegf-A RNA binding agent is inhibited by the candidate substance; and
(C) selecting the candidate substance that inhibited the binding between the p53 mRNA binding agent and the vegf-A RNA binding agent as a p53 expression promoting agent.

According to such a method, it is possible to conduct screening of a substance that inhibits the binding between the p53 mRNA and the vegf-A RNA. If the binding can be inhibited, it is possible to suppress the degradation of p53 mRNA, whereby suppression of the expression of the p53 mRNA can be avoided and the expression of p53 can be promoted (restored) consequently. Thus, the substance that has been subjected to the screening according to the present invention can be used as a p53 expression promoting agent. Note here that the method for detecting the inhibition in the step (C) is not particularly limited, and a conventionally known method for checking the presence or absence of heteroduplex formation can be employed.

Examples

Next, Examples of the present invention are described. However, the present invention is not limited to the following Examples. Commercially available reagents are used based on the protocol thereof unless otherwise stated. Hereinafter, a vegf-A gene is referred to as a vegf gene, vegf-A mRNA is referred to as vegf mRNA, vegf-A RNA is referred to as vegf RNA, and VEGF-A protein is referred to as VEGF protein.

<Cell culture>

Human large bowel cancer HCT116 cells (parent cells, wild-type p53), human large bowel cancer p53-deleted HCT116 cells (p53-KO HCT116), HT-29 cells, and SW480 cells; human liver cancer HepG2 cells, Hep3B cells, and HLE cells; human stomach cancer AGS cells; and normal human dermal fibroblasts were used. The culture of these cells was carried out using McCoy's 5A medium or Dulbecco's modified Eagle's (DMEM) medium containing 10% fetal bovine serum and/or an antibiotic.

<Transfection>

The transfection of various vectors into cells was carried out using a JetPEI transfection reagent (trade name, Polyplus-transfection) following the specifications thereof.

<vegf siRNA>

Various expression vectors were produced in order to express the following siRNAs #1 to #5 against vegf mRNA in target cells. Hereinafter, these siRNAs also are referred to as vegf siRNAs and vectors that express siRNA also are referred to as vegf siRNA vectors. A commercially available siRNA expression system (Invitrogen) was applied for expression of siRNA.

First, double-stranded oligonucleotides including coding sequences of vegf siRNA were provided (oligonucleotides #1 to #5). The sense strand sequences of these oligonucleotides are as follows. In the oligonucleotide sequences, the underlined parts are antisense sequences complementary to the insides of regions of exon 1 of vegf genes or antisense sequences complementary to the insides of regions including exon 1 and intron 1 of vegf genes, coding sequences finally acting as the siRNAs #1 to #5. Further, in the following sequences, sequences written in non capital letters are DNA sequences corresponding to sequences of RNA with deletion in two bases from the inside of a region of vegf mRNA to which antisense of siRNA is hybridized. Sense and antisense of siRNA to be expressed and sequences of regions of vegf mRNA (target RNA) to which antisense of siRNA is hybridized are shown below each of the oligonucleotide sequences.

[Table 1]

Oligonucleotide #1 (SEQ ID NO: 23)

5' -TGCTGAGCAAGGCAAGGCTCCAATGCGTTTTGGCCACTGACTGACgcattggacttgccttgct-3'

| | |
|---|---|
| siRNA #1 sense | 5' -gcauugga cuugccuugcuca-3' (SEQ ID NO: 3) |
| Antisense | 3' -guCGUAACCUCGGAACGGAACGA-5'(SEQ ID NO: 4) |
| Target RNA | 5' -gcauuggagccuugccuugcu-3' (SEQ ID NO: 24) |

Oligonucleotide #2 (SEQ ID NO: 25)

5' -TGCTGAGAGCAGCAAGGCAAGGCTCCGTTTTGGCCACTGACTGACggagccttcttgctgctct-3'

| | |
|---|---|
| siRNA #2 sense | 5' -ggagccuuc uugcugcucuca-3' (SEQ ID NO: 5) |
| Antisense | 3' -guCCUCGGAACGGAACGACGAGA-5' (SEQ ID NO: 6) |
| Target RNA | 5' -ggagccuugccuugcugcucu-3' (SEQ ID NO: 26) |

Oligonucleotide #3 (SEQ ID NO: 27)

5' -TGCTGCACGACCGCTTACCTTGGCATGTTTTGGCCACTGACTGACatgccaagaagcggtcgtg-3'

| | |
|---|---|
| siRNA #3 sense | 5' -augccaag aagcggucgugca-3' (SEQ ID NO: 7) |
| Antisense | 3' -guUACGGUUCCAUUCGCCAGCAC-5' (SEQ ID NO: 8) |
| Target RNA | 5' -augccaagguaagcggucgug-3' (SEQ ID NO: 28) |

Oligonucleotide #4 (SEQ ID NO: 29)

5' -TGCTGTAGCACTTCTCGCGCCTCCGCGTTTTGGCCACTGACTGACgcggagccgagaagtgcta-3'

| | |
|---|---|
| siRNA #4 sense | 5' -gcggagccg agaagugcuaca-3' (SEQ ID NO: 9) |
| Antisense | 3' -guCGCCUCGGCGCUCUUCACGAU-5' (SEQ ID NO: 10) |
| Target RNA | 5' -gcggagccgcgagaagugcua-3' (SEQ ID NO: 30) |

Oligonucleotide #5 (SEQ ID NO: 31)

5' -TGCTGCGAGCTAGCACTTCTCGCGGCGTTTTGGCCACTGACTGACgccgcgaggtgctagctcg-3'

| | |
|---|---|
| siRNA #5 sense | 5' -gccgcgag gugcuagcucgca-3' (SEQ ID NO: 11) |
| Antisense | 3' -guCGGCGCUCUUCACGAUCGAGC-3' (SEQ ID NO: 12) |
| Target RNA | 5' -gccgcgagaagugcuagcucg-3' (SEQ ID NO: 32) |

The double-stranded oligonucleotides each were cloned into a plasmid vector for siRNA expression according to the protocol. As the vector, a pcDNA6.2-GW/EmGFP-miR vector (Invitrogen) that includes a GFP coding region within a pre-miRNA expression cassette was used. Thus obtained recombinant vectors are referred to as a vegf siRNA#1 vector to a vegf siRNA #5 vector. With respect to each type of the recombinant vectors, the insertion sequence was identified by a DNA sequence. As a control vector of siRNA, a double-stranded oligonucleotide of a sequence (SEQ ID NO: 46: 5'-gaaatgtactgcgcgtggagacgttttggccactgactgacgtctccacgcagtacattt-3'), which does not have homology to known genes of vertebrates, was cloned into the pcDNA6.2-GW/EmGFP-miR vector. The thus obtained recombinant vector is referred to as a control siRNA vector. When cells are transfected with the various recombinant vectors, transfection efficiency was about 70% based on the proportion (%) of cells in which green fluorescence of GFP was generated.

When the vegf siRNA#1 vectors to the vegf siRNA#5 vectors are transfected into cells, chimera mRNA is expressed from chimera DNA of an EmGFP coding sequence and the oligonucleotides (#1 to #5). At this time, RNA for the oligonucleotides is expressed as 3' UTR at the 3' side of EmGFP mRNA. This 3' UTR part is bound to a complementary strand in a molecule, and thus formed base pair is cleaved by Drosha and then is further cleaved by Dicer. Thereby, in the cells transfected with the vectors, double-stranded siRNAs (#1 to #5) are formed and target RNAs are cleaved by antisense strands thereof. Both strands of thus obtained double-stranded siRNA have dibasic overhang at the 3'-ends thereof.

As a control siRNA, Silencer® negative control #1 (Ambion, Inc.) or siRISC-free control siRNA (Dharmacon, Inc.) was bought. These siRNAs do not show homology to human gene products. Further, the following siRNAs were bought from Ambion, Inc. These siRNAs were transfected into cells using a Lipofectamine RNAiMax reagent (Invitrogen) following the specifications thereof. The transfection efficiency of siRNA was about 60 to 70% when measured with BLOCK-iT Alexa Fluor Red Fluorescent Oligo (Invitrogen).

<Apoptotic evaluation 1>

Objective cells were treated with various anticancer agents, and then incubated with trypsin/EDTA to prepare cell suspensions. The cell suspensions were centrifuged and supernatants thereof were removed. Thereafter, the cell suspensions each were immobilized and stained with a solution of formaldehyde of 3.7 %, Nonidet P40 of 0.5%, and 10 μg/mL of Hoechst33258 (Invitrogen) in PBS. The stained nuclei were observed with a fluorescence microscope and the number of split nuclei was counted to score. The counting was performed with respect to at least 300 cells in one measurement.

<Apoptotic evaluation 2>

Apoptotic cells were evaluated by a TUNEL staining method using in situ Cell Death Detection kit (Roche Diagnostic). TUNEL-positive cells were observed with a microscope, and the proportion (%) of apoptosis-positive cells was calculated. The counting was performed with respect to at least 300 cells in one measurement.

<Western immunoblotting>

Western immunoblotting was performed according to Huez, I. et al. Mol. Endocrinol. 15 2197-2210 (2001) or Masuda, K., et al. PLoS Med. 5, e94 (2008). As a primary antibody, anti p53 antibody ("DO-1" or "FL-393", Santa Cruz Biotechnology, Inc.), anti β-actin ("clone AC-15", Sigma Chemical Co.), anti HA ("Y-11", Santa Cruz Biotechnology Inc.), and anti L-VEGF (polyclonal anti L-VEGF antibody) mentioned in the Huez, I. et al. (2001) were used.

<Northern blotting>

Total RNAs were extracted according to a guanidine thiocyanate method. Labeled cDNAs were produced using the following antisense primers, a klenow fragment (New England BioLabs, Inc., Inc.), and [$\alpha$-$^{32}$P]dCTP, with the following cDNAs being templates. Thus obtained labeled cDNAs were used as probes for the Northern blotting.

Probe 1 for vegf mRNA
Template: cDNA of human vegf exons 1 to exons 5
Antisense primer:

5'-gtcttgctctatctttctttggtctgc-3' (SEQ ID NO: 47)
5'-catctctcctatgtg-3' (SEQ ID NO: 48)
5'-tccaggccctcgtca-3' (SEQ ID NO: 49)

Probe 2 for vegf mRNA (for Example B)
Template: cDNA of human vegf exons 2 to 5
Antisense primer:

5'-ttgtcttgctctatctttctttg-3' (SEQ ID NO: 75)

Probe for vegf IresRNA
Template: cDNA of human vegf IresRNA (600-879 in SEQ ID NO: 1)
Antisense primer:

5'-gcgcacaccgccgcctcacccgtccatgagcccgg-3' (SEQ ID NO: 76)

Probe 1 for p53 mRNA and degraded p53 mRNA
Template: human p53 full length cDNA
Antisense primer:

5'-tcagtctgagtc-3' (SEQ ID NO: 50)
5'-gcccacggatct-3' (SEQ ID NO: 51)
5'-tgatggtgagga-3' (SEQ ID NO: 52)
5'-cctcacaacctc-3' (SEQ ID NO: 53)
5'-aagatgacaggg-3' (SEQ ID NO: 54)

Probe 2 for p53 mRNA (for Example B)
Template: human p53 full length cDNA
Antisense primer:

5'-ttaaaagctttcagtctgagtcaggc-3' (SEQ ID NO: 77)

Probes for vegf-YFP mRNA and YFP mRNA
Template: YFP cDNA (Clontech)
Antisense primer:

5'-ttgatcctagcagaagcaca-3' (SEQ ID NO: 55)

With respect to the following antisense probes, the 3-ends thereof were labeled using a terminal Deoxynucleotidyl Transferase (New England BioLabs, Inc.) and [$\alpha$-$^{32}$P]dCTP.

Antisense probe for noxa mRNA (SEQ ID NO: 56)
5'-gctctgctggagcccgcgcggggctcggttgagcgttcttgcgcg-3'
Antisense probe for puma mRNA (SEQ ID NO: 57)
5'-ccctctacgggctccggggagctgccctcctggcgtgcgcggg-3'
Antisense probe for bax mRNA (SEQ ID NO: 58)
5'-ggtggacgcatcctgaggcaccgggtccagggccagctcgggtg-3'
Antisense probe for p21 mRNA (SEQ ID NO: 59)
5'-ggcaggccttgctgccgcatgggttctgacggacatccccagccggttctgacat-3'

<Quantitative RT-PCR>

From 1 $\mu$g of total RNA, cDNA was synthesized using SuperScriptII RNaseH(-) reverse transcriptase (Invitrogen), and PCR was performed using primers for amplification-purpose nucleic sequences with the cDNA being a template. The amplification and the quantitative analysis of PCR products were performed using Applied Biosystems 7500 (Applied Biosystems) by the method recommended by the manufacturer.

Forward primer for vegf mRNA (SEQ ID NO: 78)
5'-gagccttgccttgctgctctac-3'
Reverse primer for vegf mRNA (SEQ ID NO: 79)
5'-caccagggtctcgattggatg -3
Forward primer 1 for p53 mRNA (SEQ ID NO: 80)

5'-ctttccacgacggtgaca-3'
Reverse primer 1 for p53 mRNA (SEQ ID NO: 81)
5'-tcctccatggcagtgacc-3
Forward primer for bax mRNA (SEQ ID NO: 82)
5'-atgttttctgacggcaacttc-3'
Reverse primer for bax mRNA (SEQ ID NO: 83)
5'-atcagttccggcaccttg-3'
Forward primer for noxa mRNA (SEQ ID NO: 84)
5'-gagatgcctgggaagaagg-3'
Reverse primer for noxa mRNA (SEQ ID NO: 85)
5'-ttgagtagcacactcgacttcc-3'

<RNA pull down assay>

pcDNA3.1-based various expression vectors having T7 sequences derived from the aforementioned vector sequences and pCMV-neo-Bam-based p53 expression vectors not having the T7 sequences derived from the aforementioned vector sequences were cotransfected into p53-deleted HCT116 cells. After 12 hours from the cotransfection, the cells were washed twice with PBS and the cells were dissolved by buffer A. The composition of the buffer A was 10mmol/L of HEPES (pH7.4), 60mmol/L of KCl, 3mmol/L of $MgCl_2$, 0.1% NP-40, 1mmol/L of DTT, and 10mmol/L of ribonucleoside vanadyl complex. The thus obtained cell extract was incubated with an anti T7 probe with a biotin tag. Sedimentation of a complex bound to the anti T7 probe was performed using Dynabeads M-270 streptavidin (Invitrogen), which is a magnetic bead coated with streptavidin. All RNAs collected from the sediment were treated with DNaseI ("TurboDNaseI", Ambion, Inc.). Thereafter reverse transcription and PCR were performed according to the protocol. After PCR, the presence or absence of amplification of PCR products was verified by electrophoresis.

The anti T7 probe, a PCR primer set for vegf IresRNA, and a PCR primer set for p53 mRNA used here are as follows. In the anti T7 probe, bases written in capital letters are a sequence corresponding to T7 and bases written in non capital letters are an additional sequence.

Anti T7 probe (SEQ ID NO: 86)
5'-CCCTATAGTGAGTCGTATTATTTaaaa-biotin-3'
Forward primer for vegf IresRNA (SEQ ID NO: 87)
5'-GAGCCCGCGCCCGGAGGCGGGGTGG-3'
Reverse primer for vegf IresRNA (SEQ ID NO: 88)
5'-GCGCACACCGCCGCCTCACCCGTCCATGAGCCCGG-3'
Forward primer 2 for p53 mRNA (SEQ ID NO: 89)
5'-AATATCTAGAATGGAGGAGCCGCA-3'
Reverse primer 2 for p53 mRNA (SEQ ID NO: 90)
5'-TTAAAAGCTTTCAGTCTGAGTCAGGC-3'

<Example A1>

Human liver cancer HepG2 cells having VEGF signal pathways have tolerance to anticancer agents. Thus, the influence of decrease in vegf mRNA due to RNA interference on anticancer agent tolerance of HepG2 cells was examined.

(1) Decrease in vegf mRNA

The vegf siRNA #1 vectors, the vegf siRNA #2 vectors, and the control siRNA vectors were transfected into HepG2 cells (wild-type p53). These cells were cultured for 18 hours, and the expression level of vegf mRNA was analyzed by the Northern blotting. Also, with respect to the HepG2 cells not transfected with the vectors, the analysis was performed in the same manner. At the time of analysis, gapdh mRNA in each type of the cells was monitored as a loading control (hereinafter, the same applies). The cells were cultured using DMEM medium containing 10% fetal bovine serum and 100 $\mu$g/ml (100 units/ml) of streptomycin under the condition of 37°C and 5% $CO_2$ (hereinafter, the same applies).

The results are shown in FIG. 1A. FIG. 1A shows the results of the Northern blotting, which indicate the expression of vegf mRNA in each type of the cells. In FIG. 1A, the lane 1 "-" indicates the result obtained regarding the cells not transfected with the vectors, the lane 2 "control siRNA" indicates the result obtained regarding the control siRNA-expressing cells, the lane 3 "vegf siRNA#1" indicates the result obtained regarding the vegf siRNA #1-expressing cells, and the lane 4 "vegf siRNA #2" indicates the result obtained regarding the vegf siRNA #2-expressing cells (hereinafter, the same applies).

As shown in FIG. 1A, as compared to the cells not transfected with the vectors (lane 1) and the control siRNA-expressing cells (lane 2), the expression amount of vegf mRNA in the vegf siRNA#1-expressing cells (lane 3) or the vegf siRNA#2-expressing cells (lane 4) was decreased markedly.

(2) Increase in apoptosis

Each type of the recombinant vectors was transfected into HepG2 cells for 18 hours in the same manner as in the above-described item (1). Each type of the transfection cells was treated with an anticancer agent "5-FU" having a predetermined concentration (0, 10, 30 μmol/L) for 60 hours. Then, the apoptosis (%) of the treated cells was examined according to "Apoptosis evaluation 1". The results are shown in FIG. 1B. FIG. 1B is a graph showing apoptosis (%) in each type of the cells treated with 5-FU having a predetermined concentration (means ± SD, n = 3).

As shown in FIG. 1B, when the control siRNA-expressing cells were treated with a genotoxic anticancer agent "5-FU", the proportion of apoptosis was slightly increased. In contrast, with respect to the cells expressing vegf siRNA #1 or vegf siRNA #2, although basal apoptosis (0 μmol/L) was not affected by silencing of vegf mRNA due to siRNA, the proportion of apoptosis was increased markedly in response to 5-FU in a concentration-dependent manner when treated with 5-FU. Results similar to those of the HepG2 cells in the above-described items (1) and (2) were obtained also with human colon cancer HCT116 cells (parent cells, wild-type p53).

(3) Expression of p53

It has been known that apoptosis induced by 5-FU depends on p53 in HepG2 cells and HCT116 cells (Bunz, F. et al. J. Clin. Invest. 104, 263-269 (1999)). Thus, the expression levels of p53 mRNA and p53 protein were measured for examining whether or not p53 pathways in cells are affected by decrease in vegf mRNA in the above-described item (1).

Each type of the vectors was transfected into HepG2 cells for 18 hours in the same manner as in the above-described item (1). Then, the cells were treated in the presence of 5-FU (30 μmol/L) (+) or the absence of 5-FU (-). With respect to the cells treated for 8 hours, the expression level of p53 mRNA was analyzed by the Northern blotting. With respect to the cells treated for 12 hours, the expression level of p53 protein was analyzed by the Western immunoblotting. Analysis of protein also was performed with respect to β-actin protein that is an endogenous control (hereinafter, the same applies). The results are shown in FIG. 1C. FIG. 1C shows the results indicating expression of p53 mRNA and p53 protein in the cells expressing vegf siRNA. In FIG. 1C, the first row from the top indicates expression of p53 mRNA, the second row indicates expression of gapdh mRNA, the third row indicates expression of p53 protein, and the fourth row indicates expression of β-actin protein. Further, in FIG. 1C, "-"indicates the treatment in the absence of 5-FU and "+" indicates the treatment in the presence of 5-FU.

In the same manner, HepG2 cells transfected with each type of the vectors were treated for 16 hours in the presence of 5-FU (30 μmol/L) (+) or the absence of 5-FU (-). With respect to the treated cells, expression of noxa mRNA, puma mRNA, and p21 mRNA induced in p53 pathways was analyzed by the Northern blotting. The results are shown in FIG. 1D. FIG. 1D shows the results of the Northern blotting, which indicate expression of p53 mRNA in the cells expressing vegf siRNA. In FIG. 1D, the first row from the top indicates expression of noxa mRNA, the second row indicates expression of puma mRNA, the third row indicates expression of p21 mRNA, and the fourth row indicates expression of gapdh mRNA. Definitions of the lanes in FIG. 1D are the same as those of FIG. 1C.

As shown in FIG. 1C, in the absence of 5-FU (-), as compared to the control cells (lane 1), expression of p53 mRNA and p53 protein in the vegf siRNA #1-expressing cells (lane 3) and the vegf siRNA #2-expressing cells (lane 5) was increased by silencing of vegf. Further, as shown in the lanes 4 and 6 in FIG. 1C, in the presence of 5-FU (+), in the vegf siRNA #1-expressing cells (lane 4) and the vegf siRNA #2-expressing cells (lane 6), expression induction of p53 due to 5-FU was enhanced by silencing of vegf, and expression of p53 mRNA and p53 protein was increased markedly. In accordance with this, as shown in FIG. 1D, in the vegf siRNA #1-expressing cells (lane 4) and the vegf siRNA #2-expressing cells (lane 6), expression induction of noxa, mRNA, puma mRNA, and p21 mRNA was enhanced markedly. In contrast, as shown in FIG. 1D, in the control cells (lane 2), expression of p21 mRNA was slightly induced in response to 5-FU.

These results show that the increase in the expression level of p53 RNA due to knockdown of vegf mRNA increases the sensitivity of cells to anticancer agents and allows the response of the cell to 5-FU of the optimal administration or of less than the optimal administration. These results suggest that expression of p53 is controlled by vegf mRNA in cells.

<Example A2>

It was examined whether or not VEGF protein or vegf mRNA was directly involving control of p53 expression.

(1) Plasmid construct

Sequences corresponding to coding genes (vegf genes) of human $VEGF_{165}$, the 5' UTR thereof, and the 3' UTR thereof are provided based on the document (J. Abraham et al. J. Biol. Chem. 266, 18, 11947-11954 (1991)). FIG. 2A schematically shows plasmid constructs produced according to the following method. The plasmid constructs A (p165mSPHA3'), B (p165mSPHA), C (p165-3'), and D (pVC) were produced in reference to the methods mentioned in documents (Bornes, S. et al. J. Biol. Chem. 279, 18717-18726 (2004) and Huez, I. et al. Mol. Endocrinol. 15 2197-2210 (2001)).

Construct A (p 165 mSPHA3') (full length cDNA of vegf gene)

A construct A (p165mSPHA3') was produced in reference to the method mentioned in the document (Bornes, S. et al. J. Biol. Chem. 279, 18717-18726 (2004)). The construct A has cDNA of a vegf gene, i.e., the construct A has a DNA sequence corresponding to 5' UTR, a coding sequence of $VEGF_{165}$ protein, and a DNA sequence corresponding to 3' UTR. In addition, a DNA fragment having a coding sequence of an HA tag added between the 3'-end of the protein coding sequence and the 5'-end of the 3' UTR DNA sequence is inserted into a pSCT plasmid vector.

Construct B (p165 mSPHA) (3'UTR deletion)

A construct B (p 165 mSPHA) was produced by making reference to the method mentioned in the document (Bornes, S. et al. J. Biol. Chem. 279, 18717-18726 (2004)). The construct B has a DNA sequence corresponding to 5'UTR of a vegf gene and a coding sequence of $VEGF_{165}$ protein. In addition, a DNA fragment having a coding sequence of an HA tag added to the 3'-end of the protein coding sequence is inserted into a pSCT plasmid vector.

Construct C (p165-3) (5' UTR deletion)

A construct C (p165-3') having the following DNA fragment inserted into a pSCT plasmid vector was produced. The DNA fragment has a coding sequence of $VEGF_{165}$ protein and a DNA sequence corresponding to 3' UTR of a vegf gene. In addition, the DNA fragment has a coding sequence of an HA tag added between the 3'-end of the protein coding sequence and the 5'-end of the 3' UTR DNA sequence. Specifically, the construct A (p165mSPHA3') was treated with XbaI and NgoM IV, which are restriction enzymes, to remove a 5' UTR part. Thereafter, the DNA was blunt-ended by a klenow fragment treatment, and the blunt ends were connected using a ligase to obtain the construct C.

Construct D (pVC) (5' UTR only)

A construct D (pVC) was produced in reference to the method mentioned in the document (Huez, I. et al. Mol. Endocrinol. 15 2197-2210 (2001)). In the construct D, chimera DNA of a CAT coding sequence and a DNA sequence corresponding to a region from base 1 to base 1205 in SEQ ID NO: 1 is inserted into a pSCT plasmid vector. The region from base 1 to base 1205 in SEQ ID NO: 1 includes 5' UTR and a sequence from an initiation codon atg to base 167 in vegf mRNA.

Construct mock

An empty vector (pSCT plasmid vector) into which a DNA fragment of a vegf gene is not inserted was used as a construct mock.

(2) Expression of p53 mRNA and p53 protein

Plasmid vectors that express p53 and each type of the plasmid constructs as effectors were cotransfected into human lung cancer H1299 cells (p53 null). Hereinafter, the plasmid vector that expresses p53 is referred to as "p53 expression vector". The p53 expression vector (pcDNA3.1-p53) was produced by inserting a protein coding sequence of human p53 (from base 252 to base 1433 in SEQ ID NO: 2) into a BamHI-EcoRI site of a pcDNA3.1 vector (Invitrogen). Hereinafter, the same applies. Each type of the cotransfection cells was cultured for 18 hours, and the expression level of exogenous p53 mRNA was analyzed by the Northern blotting. Further, each type of the cotransfection cells was cultured for 24 hours, and the expression level of exogenous p53 protein and the expression level of exogenous VEGF protein were analyzed by the Western immunoblotting respectively using anti p53 antibodies and anti HA tag antibodies. The cells were cultured using DMEM medium containing 10% fetal bovine serum and 100 μg/ml (100 units/ml) of streptomycin under the condition of 37°C and 5% $CO_2$ (hereinafter, the same applies).

The results are shown in FIGs. 2B and 2C. FIG. 2B shows the results of the Northern blotting, which indicate expression of p53 mRNA in each type of the cells. In FIG. 2B, the upper row indicates expression of p53 mRNA and the lower row indicates expression of gapdh mRNA. FIG. 2C shows the results of the Western immunoblotting, which indicate expression of p53 protein in each type of the cells. In FIG. 2C, the upper row shows expression of p53 protein and the lower row shows expression of β-actin protein. In FIGs. 2B and 2C, the lane 1 "NT" indicates the cells not transfected with the vectors, the lane 2 "A" indicates the cells transfected with the construct A (p165mSPHA3'), the lane 3 "B" indicates the cells transfected with the construct B (p165mSPHA), the lane 4 "C" indicates the cells transfected with the construct C (p165-3'), the lane 5 "mock" indicates the cells transfected with the empty vectors, and the lane 6 "D" indicates the cells transfected with the construct D (pVC). In FIGs. 2B and 2C, "p53+" indicates that the transfection of p53 expression vectors was conducted.

As shown in FIGs. 2B and 2C, in the cells transfected with the construct A expressing full length mature mRNA of vegf genes (lane 2), expression of exogenous p53 mRNA was markedly suppressed. This result shows that VEGF protein or vegf mRNA is involved in expression of p53. Thus, in order to confirm the involvement of an UTR sequence of vegf mRNA in suppression of expression of p53, expression of p53 was examined using the construct B in which 3' UTR is deleted and the construct C in which 5' UTR is deleted. As a result, in the cells transfected with the construct B in which 3' UTR is deleted (lane 3), expression of exogenous p53 was suppressed markedly in the same manner as the cells transfected with the construct A including full length cDNA of a vegf gene (lane 2). In contrast, in the cells transfected with the construct C in which 5' UTR is deleted (lane 4), expression of p53 was not affected. This result showed that suppression of p53 mRNA is not mediated by VEGF protein but mediated by vegf mRNA, especially by a 5' UTR sequence of vegf mRNA. In order to confirm the involvement of 5' UTR, expression of p53 with respect to the construct D was examined in the same manner. The construct D has a CAT coding gene fused with a DNA sequence of 5' UTR, and expresses chimera mRNA of CAT mRNA and 5' UTR. Since the cells transfected with the construct D only have 5' UTR of vegf genes, VEGF protein is not produced. As a result, although VEGF protein is not produced, suppression of expression of p53 mRNA and p53 protein was observed. From this result, it was ascertained that not VEGF protein but vegfmRNA, especially 5' UTR of mRNA was involved in suppression of expression of p53. Accordingly, with respect to vegf genes, down regulation of p53 due to vegf mRNA can be released by suppression of expression of 5' UTR or cleaving of expressed 5' UTR. Similar results were obtained also with the p53-deleted HCT116 cells.

<Example A3>

A construct that expresses partially deleted 5' UTR or mutant 5' UTR was produced, and an element sequence of vegf 5' UTR involving down regulation of p53 was determined.

(1) Plasmid construct

A DNA fragment corresponding to exon 1 of a human vegf gene and the following various plasmid constructs were provided based on the aforementioned documents in the same manner as in Example A2. The plasmid constructs, which were produced according to the following method, are shown in FIG. 3A.

<u>Construct A (pVC) (vegf 1-1203)</u>

A construct A (pVC) was produced by making reference to the method mentioned in the document (Huez, I. et al. Mol. Endocrinol. 15 2197-2210 (2001)). In the construct A, chimera DNA of a CAT coding sequence and a DNA sequence having exon 1, exon 2, and a part of exon 3 in a vegf gene is inserted into a pSCT plasmid vector. The DNA sequence here corresponds to exon 1 (from base 1 to base 1104 in SEQ ID NO: 1), exon 2 (from base 1105 to base 1154 in SEQ ID NO: 1), and a partial sequence of exon 3 (from base 1155 to base 1203 in SEQ ID NO: 1) in vegf mRNA. This construct A is the same as the construct D in Example A2.

<u>Construct B (Δ 555-1012)</u>

A construct B in which exon 1 is partially deleted was produced. In the construct B, chimera DNA of a CAT coding sequence and a DNA sequence having a partial sequence of exon 1 in which a region from base 555 to base 1012 in SEQ ID NO: 1 is deleted, exon 2 (from base 1105 to base 1154 in SEQ ID NO: 1), and a partial sequence of exon 3 (from base 1155 to base 1203 in SEQ ID NO: 1) is inserted into a pSCT plasmid vector. Specifically, the construct A (pVC) was treated with NgOMIV and ligated again to obtain the construct B.

Construct C (Δ 1-744)

A construct C in which exon 1 is partially deleted was produced. In the construct C, chimera DNA of a CAT coding sequence and a DNA sequence having a partial sequence of exon 1 in which a region from base 1 to base 744 in SEQ ID NO: 1 is deleted, exon 2 (from base 1105 to base 1154 in SEQ ID NO: 1), and a partial sequence of exon 3 (from base 1155 to base 1203 in SEQ ID NO: 1) is inserted into a pSCT plasmid vector. Specifically, the construct A (pVC) was treated with XbaI and NheI, and ligated again to obtain the construct C.

Construct D (Δ 1-658)

A construct D in which exon 1 is partially deleted was produced. In the construct D, chimera DNA of a CAT coding sequence and a DNA sequence having a partial sequence of exon 1 in which a region from base 1 to base 658 in SEQ ID NO: 1 is deleted, exon 2 (from base 1105 to base 1154 in SEQ ID NO: 1), and a partial sequence of exon 3 (from base 1155 to base 1203 in SEQ ID NO: 1) is inserted into a pSCT plasmid vector. Specifically, the construct A (pVC) was treated with XbaI and XmnI, blunt-ended by a ligase treatment, and ligated again to obtain the construct D.

Construct E (Δ 1-475, Δ 918-1175)

A construct E in which exon 1 is partially deleted was produced. In the construct E, chimera DNA of a CAT coding sequence and a DNA sequence having a partial sequence of exon 1 in which a region from base 1 to base 475 and a region from base 918 to base 1175 in SEQ ID NO: 1 are deleted and a partial sequence of exon 3 (from base 1176 to base 1203 in SEQ ID NO: 1) in which a 5' region is deleted is inserted into a pSCT plasmid vector. Specifically, the construct A (pVC) was treated with BamHI, a BamHI fragments was removed, and then ligated again (pVC-Δ1-474). Then, a SmaI-BsaBI fragment of the pVC-Δ1-474 was removed, and ligated again to obtain the construct E.

Construct F (Δ 1-475, Δ 746-1012)

A construct F in which exon 1 is partially deleted was produced. In the construct F, chimera DNA of a CAT coding sequence and a DNA sequence having a partial sequence of exon 1 in which a region from base 1 to base 475 and a region from base 746 to base 1012 in SEQ ID NO: 1 are deleted, exon 2 (from base 1105 to base 1154 in SEQ ID NO: 1), and a partial sequence of exon 3 (from base 1155 to base 1203 in SEQ ID NO: 1) is inserted into a pSCT plasmid vector. Specifically, a NheI-NgOMIV fragment was removed from the construct pVC-Δ 1-474, and the construct was blunt-ended by a ligase treatment, and ligated again to obtain the construct F.

Construct G (pVCTTT) (mutation of 499-501: CTG→TTT)

A construct G (pVCTTT) having mutant exon 1 that expresses mutant 5' UTR was produced by making reference to the method mentioned in the document (Huez, I. et al. Mol. Endocrinol. 15 2197-2210 (2001)). In the construct G, chimera DNA of a CAT coding sequence and a DNA sequence having a sequence of the mutant exon 1 and a partial sequence of exon 2 (from base 1105 to base 1205 in SEQ ID NO: 1) is inserted into a pSCT plasmid vector. Specifically, in a DNA sequence coding 5' UTR, a CTG codon (corresponding to base 499 to base 501 in SEQ ID NO: 1) was mutated to TTT. Due to this mutation introduction, mRNA transcribed from pVCTTT cannot produce protein from the CTG codon (from base 499 to base 501 in SEQ ID NO: 1).

Construct H (pVCmut5') (mutant 5' UTR)

A construct H having chimera DNA of a CAT coding sequence and a sequence of mutant exon 1 that expresses mutant 5' UTR and a partial sequence of exon 2 (from base 1105 to base 1205 in SEQ ID NO: 1) was produced. Specifically, mutation was introduced into a sequence of exon 1 in such a manner that an amino acid sequence of long VEGF protein (hereinafter, referred to as "L-VEGF") in which translation is started from a CTG codon (from base 499 to base 501 in SEQ ID NO: 1), was not affected. The mutation was introduced into a DNA region corresponding to a region from base 594 to base 915 in SEQ ID NO: 1 in a DNA sequence coding 5' UTR.

First, in a DNA sequence coding 5' UTR, four types of double-stranded oligonucleotides each having mutation in a DNA region corresponding to a region from base 594 to base 915 in SEQ ID NO: 1 were synthesized. Sense strand sequences of the double-stranded oligonucleotides are as follows. In the following sequences, non capital letters indicate mutant parts.

Fragment 1 (SEQ ID NO: 33)

5'-GCG AGC CGC GGG CAa GGa CCa GAa CCa GCa CCa GGg GGa GGa GTa GAa GGa GTa GGG GCT C-3'

Fragment 2 (SEQ ID NO: 34)

5'-GG GCT CGa GGa GTa GCa tta AAg CTa TTt GTa CAg tta tta GGa TGc TCa CGa TTt GGg GGg GCa GTa GTa aga GCa GGa Gag GCa GAa CCa AGt GGg GCa GCa AGg AGT GCT AGC-3'

Fragment 3 (SEQ ID NO: 35)

5'-GCT AGC TCa GGa aga GAa GAa CCa CAa CCa GAa GAa GGa GAa GAa Gag Gag GAa AAa GAg GAa GAa cgt GGa CCa CAa TGG agg tta GGa GCa aga AAa CCG GGC TC-3'

Fragment 4 (SEQ ID NO: 36)

5'-G GGC TCA TGG ACa GGa GAa GCa GCa GTa TGt GCc Gat AGc GCa CCt GCa GCa aga GCa CCa CAa GCa tta GCC CGG GCC TCG-3'

These four types of double-stranded oligonucleotides were connected to the construct A (pVC) treated with SacII and XmaI. The Fragments 1 to 4, in this order, were inserted into the construct A (pVC) from the upstream 5' side. In the plasmid construct into which four types of fragments having mutations were inserted, inserted fragments were identified by a DNA sequence. The plasmid construct obtained in this manner is referred to as a construct H (pVCmut5').

(2) Expression of p53 mRNA and p53 protein

The aforementioned p53 expression vectors and each type of the plasmid constructs as effectors were cotransfected into H1299 cells (p53 null). Then, in the same manner as in Example A2, each type of the transfection cells was cultured, and the expression level of exogenous p53 mRNA and the expression level of exogenous p53 protein were analyzed. In addition, the expression level of L-VEGF was analyzed by the Western immunoblotting using anti L-VEGF antibodies. The results are shown in FIGs. 3B and 3C.
FIGs. 3B and 3C show the results of the Northern blotting. The first row from the top indicates expression of p53 mRNA, and the second row indicates expression of gapdh mRNA. In FIG. 3C, the third row from the top indicates expression of p53 protein, the fourth row indicates expression of L-VEGF protein, and the fifth row indicates expression of β-actin protein. In FIG. 3B, the lane 1 "A" indicates the cells transfected with the construct A, the lane 2 "B" indicates the cells transfected with the construct B, the lane 3 "C" indicates the cells transfected with the construct C, the lane 4 "D" indicates the cells transfected with the construct D, the lane 5 "E" indicates the cells transfected with the construct E, and the lane 6 "F" indicates the cells transfected with the construct F. In FIG. 3C, the lane 1 "NT" indicates the cells not transfected with the vectors, the lane 2 "mock" indicates the cells transfected with the construct mock, the lane 3 "G" indicates the cells transfected with the construct G, the lane 4 "A" indicates the cells transfected with the construct A, and the lane "H" indicates the cells transfected with the construct H. "p53+" indicates that the transfection of p53 expression vectors was conducted. In FIG. 3C, "ns" indicates nonspecific bands detected by anti L-VEGF antibodies.
As shown in FIG. 3B, as same as the cells transfected with the construct A that expresses full length exon 1 RNA including 5' UTR (lane 1), the constructs D (lane 4) and E (lane 5) had the ability to conduct down regulation of expression of p53 mRNA. The construct D has a region downstream from base 659 of exon 1, and the construct E has a region from base 476 to base 917 and a region downstream from base 1176. This showed that a molecule of 259 nucleotide (nt), which is a region from base 659 to base 917 of vegf 5'UTR, was involved in the down regulation of p53. In contrast, in the cells transfected with the construct B (lane 2), the cells transfected with the construct C (lane 3), and the cells transfected with the construct F (lane 6), in which expression of p53 was not suppressed, a region from base 555 to base 1012 of exon 1, a region from base 1 to base 744 of exon 1, and a region from base 746 to base 1012 of exon 1 were deleted, respectively. These results meet the results of the constructs D and E in which expression of p53 was suppressed.

The aforementioned region from base 659 to base 917 includes a part of a unique structural sequence, which is called an internal ribosome entry site (IRES) (Huez, I. et al. Mol. Cell. Biol. 18, 6178-6190 (1998), Akiri, G. et al. Oncogene 17, 227-236 (1998)). Thus, with respect to IRES-containing 5' UTR in other genes (gf-2, pdgf, and c-myc), the influence on expression of p53 was checked. The results are shown in FIG. 4. FIG. 4 shows the results of the Northern blotting, which indicate expression of p53 mRNA in the H1299 cells cotransfected with the p53 expression vectors and the plasmid vectors that express chimera mRNA of CAT mRNA and 5' UTR RNA of each type of the genes. As shown in FIG. 4, it was ascertained that expression of p53 was specifically suppressed only by 5' UTR.

In the document (Huez, I. et al. Mol. Endocrinol. 15, 2197-2210 (2001)), it was demonstrated that there is a case in which 5' UTR of human vegf mRNA is translated into protein (L-VEGF), VEGF protein having an elongated N-terminal, in frame, from a CUG codon (499-501) positioned upstream from an initiation codon AUG (1039-1041). The element sequence (from base 659 to base 917) of RNA involving suppression of p53 expression is localized in a region downstream from the CUG codon to be translated into L-VEGF Therefore, there is a possibility that vegf 5' UTR RNA or L-VEGF protein is involved in suppression of p53 expression. Thus, there was an examination of which one of vegf 5' UTR RNA and L-VEGF protein induces suppression of p53 expression using the constructs G and H that express mutant 5' UTR. In the construct G, as described above, the DNA sequence was changed so that an initiation codon CTG became a non initiation codon TTT (CUG to UUU in mRNA). In the construct H, a 5' UTR coding DNA sequence was substituted so as to produce L-VEGF protein without changing an amino acid sequence as well as sufficiently to impair the function of RNA by changing the RNA structure. As a result, as shown in FIG. 3C, with respect to the construct G (lane 3), although L-VEGF protein was not detected because a non initiation codon was changed to TTT, expression of p53 mRNA still was suppressed. In contrast, the construct H (lane 5) did not show the ability to suppress expression of p53 mRNA although L-VEGF protein was produced in the same manner as the construct A (lane 4) having full length 5' UTR. These results show that not L-VEGF protein translated from 5' UTR but the 5' UTR has the ability to suppress expression of p53 at the mRNA level. Therefore, it can be said that the down regulation of p53 can be released by suppressing expression of 5' UTR in vegf mRNA or degrading expressed 5' UTR (mRNA). Further, as can be seen from the aforementioned results, suppression of expression of 5' UTR may be conducted in a complete sequence or only in an element sequence (from base 659 to base 917). When suppression of p53 expression can be released in this manner, growth of cancer cells is suppressed and apoptosis can be induced by expression of p53. As a result, this may allow suppression of canceration of cells and growth of cancer cells and also may allow treatment of cancer.

<Example A4>

In Example A4, the influence of 5' UTR in vegf mRNA on endogenous p53 expression and p53-dependent apoptosis response was examined. In Example A4, an adenovirus backbone vector that expresses chimera mRNA in which vegf 5' UTR is fused to mRNA coding yellow fluorescent protein (YFP) was used.

(1) Construction of recombinant adenovirus vector

The construct D (pVC) in Example A2 was treated with EcoRI and NcoI, and thus obtained EcoRI-NcoI fragment was connected to pd2EYFP-N1 (Clontech). The thus obtained plasmid construct is referred to as pVY. Further, the construct H (pVCmut5') in Example A3 was treated with EcoRI and NcoI, and the thus obtained EcoRI-NcoI fragment was connected to pd2EYFP-N1 (Clontech). The thus obtained plasmid construct is referred to as pVYmut5'.

The pVY, the pVCmut5', and the pd2EYFP-N1 (control) were treated with EcoRI and NotI, and cleaved fragments were blunted by the klenow fragment treatment. Then each blunted fragment was cloned to a pShuttleCMV vector. The thus obtained plasmid vectors are referred to as pShuttleCMV-VY pShuttleCMV-VYmut5', and pShuttleCMV-YFP (control). The construction of these plasmid vectors was identified by a DNA sequence. Subsequently, in BJ5183 bacteria, homologous recombination was conducted between an adenovirus backbone plasmid pAdEasy-1 and each of plasmids, pShuttleCMV-VY, pShuttleCMV-VYmut5', and pShuttleCMV-YFP treated with PmeI to produce recombinant adenovirus vectors, Ad-VY, Ad-VYmut5', and Ad-YFP. Then, these recombinant adenovirus vectors were treated further with PacI and transfected into HEK293 cells (ATCC) using JetPEI (Polyplus-transfection). The replicated virus was purified by a CsCl equilibrium density-gradient centrifugation method, and stored at -80°C until use. The function titration with respect to a plaque forming unit of virus was verified by end-point dilution infection in the HEK293 cells. The following test was carried out with respect to a single batch of each type of the recombinant adenovirus vectors, Ad-VY, Ad-VYmut5', and Ad-YFP.

Ad-VY is a recombinant vector that expresses chimera mRNA in which full length 5' UTR of vegf mRNA was fused in mRNA coding YFP (hereinafter, also referred to as "Ad-VEGF5'"). Ad-VEGF5' is a vector having a chimera gene in which mutant 5' UTR shown in the construct H (pVCmut5') in FIG. 3A was fused in mRNA coding YFP. Ad-YFP is a control vector into which 5' UTR of a vegf gene was not inserted and expresses mRNA coding YFP.

(2) Expression of endogenous p53

Expression of endogenous p53 mRNA

HCT116 cells (parent cells, wild-type p53) were infected with each type of the recombinant adenovirus vectors, Ad-VY, Ad-VYmut5', and Ad-YFP, for 12 hours according to a conventionally known method. Then, each type of the infected cells was treated with 90 μmol/L of 5-FU for a predetermined time (0, 6, and 12 hours). With respect to the treated cells, the expression level of endogenous p53 mRNA, exogenous vegf-YFP mRNA, and exogenous YFP mRNA was analyzed by the Northern blotting. The HCT116 was cultured using McCoy's 5A medium containing 10% fetal bovine serum and 100 μg/ml (100 units/ml) of streptomycin under the condition of 37°C and 5% $CO_2$ (hereinafter, the same applies). The results are shown in FIG. 5A. FIG. 5A shows the results of the Northern blotting, which indicate expression of endogenous p53 mRNA in each type of the cells. In FIG. 5A, the first row from the top indicates expression of endogenous p53 mRNA, the second row indicates expression of vegf-YFP chimera mRNA or YFP mRNA, and the third row indicates expression of gapdh mRNA. In FIG. 5A, the lanes 1 to 3 "Ad-control" indicates the result obtained regarding the cells transfected with the control Ad-YFP, the lanes 4 to 6 "Ad-VEGF mut5'" indicates the result obtained regarding the cells transfected with the Ad-VYmut5', and the lanes 7 to 9 "Ad-VEGF 5'" indicates the cells transfected with the Ad-VY. Further, the numeric values regarding "5-FU" indicate the time (h) of the culture in the presence of 5-FU. In this state, "0h" means a condition not treated with 5-FU.

Production of endogenous p53 protein

In the same manner as described above, cells infected with each type of the recombinant adenovirus vectors for 12 hours were treated with 120 μmol/L of 5-FU for predetermined time (0, 9, and 18 hours). With respect to the treated cells, the expression level of endogenous p53 protein was analyzed by the Western immunoblotting. The results are shown in FIG. 5B. In FIG. 5B, the first row from the top indicates expression of endogenous p53 protein and the second row indicates expression of β-actin protein. Definitions of the lanes in FIG. 5B are the same as those of FIG. 5A.

Expression of mRNA of each gene induced in p53 pathway

In the same manner as described above, cells infected with each type of the recombinant adenovirus vectors were treated with 120 μmol/L of 5-FU for a predetermined time (0, 9, and 18 hours). With respect to the treated cells, the expression level of noxa mRNA, bax mRNA, and p21 mRNA was analyzed by the Northern blotting. The results are shown in FIG. 5C. In FIG. 5C, the first row from the top indicates expression of noxa mRNA, the second row indicates expression of bax mRNA, the third row indicates expression of p21 mRNA, and the fourth row indicates expression of gapdh mRNA. Definitions of the lanes in FIG. 5C are the same as those of FIG. 5A.

As shown in the lanes 7 to 9 in FIG. 5A, in the cells (Ad-VY) overexpressing vegf 5' UTR (unmutated), expression of endogenous p53 mRNA was markedly decreased. Further, as shown in the lanes 7 to 9 in FIG. 5B, accumulation of p53 protein in response to 5-FU was suppressed markedly by vegf 5' UTR. As a result, as shown in the lanes 7 to 9 in FIG. 5C, also in mRNA of noxa, bax, and p21, which are target genes of p53, expression induction was suppressed. In contrast, as shown in FIG. 5A, in the cells (Ad-VYmut5') expressing vegf mutant 5' UTR shown in the lanes 4 to 6 and the control cells (Ad-control) shown in the lanes 1 to 3, expression of endogenous p53 mRNA was observed. Further, in response to the treating time with 5-FU, increase in the expression amount of p53 mRNA was observed. Further, as shown in FIG. 5B, accumulation of p53 protein in response to 5-FU was increased in accordance with the treating time with 5-FU. As a result, as shown in FIG. 5C, induction of expression of mRNA of noxa, bax, and p21, target genes of p53, was ascertained.

(3) p53-dependent apoptosis response

In the same manner as described above, cells infected with each type of the recombinant adenovirus vectors for 12 hours were treated with 5-FU of predetermined concentration (0, 30, 60, and 120 μmol/L) for 60 hours. With respect to the treated cells, apoptosis (%) was examined in the same manner as in Example A1 (means ± SD, n = 3). The results are shown in FIG. 6D.

FIG. 6D is a graph showing proportion (%) of apoptosis of each type of the cells against change in 5-FU concentration. In FIG. 6D, the white circle indicates the result obtained regarding the cells (Ad-control) transfected with the control Ad-YFP, the white square indicates the cells (Ad-VYmut5') transfected with the Ad-VYmut5', and the black square indicates the cells (Ad-VEGF 5') transfected with the Ad-VY. Definitions of the white circle, the white square, and the black square in FIG. 6E are the same as those of FIG. 6D.

Further, in the same manner as described above, cells infected with each type of the recombinant adenovirus vectors for 12 hours were treated with 120 μmol/L of 5-FU for predetermined time (0, 12, 24, 48, and 60 hours), and apoptosis (%) was examined (means ± SD, n = 3). The results are shown in FIG. 6E. FIG. 6E is a graph showing the change in proportion (%) of apoptosis with time in each type of the cells treated with 5-FU.

As shown in FIGs. 6D and 6E, the cells (Ad-VY, black square) expressing vegf 5' UTR had marked tolerance to apoptosis induced by 5-FU. Particularly, as shown in FIG. 6D, when the concentration of 5-FU was 30 μmol/L or 60 μmol/L, apoptosis was completely inhibited by 5' UTR. As shown in FIG. 6E, even when the treating time with 5-FU was lengthened, a great increase in apoptosis was not observed. In contrast, the cells (Ad-VYmut5', white square) expressing vegf mutant 5' UTR did not have tolerance to apoptosis. Further, depending on the concentration of 5-FU as shown in FIG. 6D or depending on the treating time with 5-FU as shown in FIG. 6E, the proportion of apoptosis was increased. This result was similar to that of the cells (Ad-control, white circle) expressing no vegf 5' UTR, and meets the result of activation of p53 pathway shown in FIGs. 5B and 5C.

<Example A5>

Possibility of degradation of expressed endogenous p53 mRNA due to vegf 5' UTR was examined.

(1) Induction of degradation of p53 mRNA due to vegf 5' UTR

In the same manner as in Example A4, the p53 expression vectors and each type of the recombinant plasmid vectors, pVC and pVCmut5', or empty vectors (mock) were cotransfected into H1299 cells (p53 null), and the cells were cultured for 12 hours. RNA was collected from each type of the transfection cells, the RNA was separated by a denatured agarose gel electrophoresis, and exogenous p53 mRNA and its degradation product were detected by the Northern blotting. The results are shown in FIG. 7A. FIG 7A is a photograph showing exogenous p53 mRNA and its degradation product in each type of the cells. In FIG. 7A, the lane 1 "mock" indicates the result obtained regarding the cells transfected with the empty vectors, the lane 2 "VEGF mut5'" indicates the result obtained regarding the cells transfected with pVCmut5', and the lane 3 "VEGF 5'" indicates the result obtained regarding the cells transfected with pVC. The size (nt) of an RNA marker (Ambion, Inc.) was shown on the left of the photograph of blotting.

As shown in the lane 3 in FIG. 7A, it was ascertained that expressed p53 mRNA was degraded by expression of vegf 5' UTR. In contrast, when vegf 5' UTR was not expressed (lane 1) or mutant vegf 5' UTR was expressed (lane 2), although p53 mRNA was expressed, its degradation product was not observed. This result shows that vegf 5' UTR RNA suppresses expression of p53 mRNA at the posttranscriptional level.

(2) Influence of translation inhibitor, cycloheximide, in degradation of p53 mRNA mediated by 5' UTR

First, the p53 expression vectors and each type of the recombinant plasmid vectors, pVC and pVCmut5', were cotransfected into H1299 cells (p53 null), and the cells were cultured for 8 hours. Then, each type of the transfection cells was treated in the presence or absence of cycloheximide having the final concentration of 50 μg/ml for 16 hours. With respect to the treated cells, the expression level of p53 mRNA was analyzed by the Northern blotting. The results are shown in FIG. 7B. FIG. 7B shows the results of the Northern blotting, which indicate expression of p53 mRNA in each type of the cells. In FIG. 7B, the first row from the top indicates expression of p53 mRNA and the second row indicates expression of gapdh mRNA. In FIG. 7B, the lanes 1 and 3 "VEGF 5'" indicates the pVC-transfected cells expressing vegf 5' UTR and the lanes 2 and 4 "VEGF mut5'" indicates the pVCmut5'-transfected cells expressing denatured 5' UTR. Further, "-" indicates the treatment in the absence of cycloheximide (CHX) and "+" indicates the treatment in the presence of cycloheximide (CHX).

As shown in FIG. 7B, marked inhibition of silencing of p53 mRNA due to vegf 5' UTR was verified by the cells treated with cycloheximide (lanes 3 and 4) as compared to the cells to which cycloheximide was not added. These results showed that a translation process was involved in silencing of p53 mRNA mediated by vegf 5' UTR.

(3) Direct interaction between vegf 5' UTR RNA and p53 mRNA *in vitro*

As a mechanism of gene silencing mediated by RNA, direct interaction between target RNA and control RNA by base pairing has been known. Thus, the direct interaction between vegf 5' UTR and p53 mRNA was analyzed.

The plasmid vector D (pVC) in Example A2, the plasmid vector H (pVCmutS') in Example A3, and the pc53 expression vectors (pcDNA3.1-p53) were treated with BsaBI and XbaI to be linearized. RNAs having capping 5'-ends were synthesized by an *in vitro* transcription method using a mMESSAGEmMACHINE kit (Ambion, Inc.) with the linearized DNAs being templates. The synthesized RNAs were quantified by ethidium bromide staining with an agarose gel at the absorbance of 260 nm. RNAs were each dissolved in RNase-free water, and these solutions were heated at 90°C for 3 minutes and then gradually cooled to 25°C. The RNAs each were mixed with a $Mg^{2+}$-containing buffer in equal amount (1 pmol) so as to be the combinations shown in Fig. 7C, and incubated at 37°C for 60 minutes. The buffer was a hypertonic solution, in which molecular association occurs in RNA, comprising 10mmol/L of Tris-HCl (pH7.5), 50mmol/L of KCl, 5mmol/L of $MgCl_2$, and 40U/ml of RNasin® (Promega Corporation). These samples of after incubation were each subjected to undenatured agarose gel of 1.8%, and stained with ethidium bromide.

The results are shown in FIG. 7C. FIG. 7C is a photograph showing association of vegf 5' UTR RNA and p53 mRNA *in vitro*. In FIG. 7C, "VEGF mut5' UTR RNA" is mutant 5' UTR RNA, "VEGF 5' UTR RNA" is 5' UTR RNA (wild-type), and "p53 RNA" is p53 mRNA. "+" and "-" on the upper side of each lane indicate the combinations of RNAs incubated in the $Mg^{2+}$-containing buffer. Further, in FIG. 7C, the arrow indicates heteroduplex of 5' UTR RNA and p53 mRNA.

As shown in the lane 3 in FIG. 7C, it was verified that vegf 5' UTR RNA involving attenuation of p53 mRNA formed a heteroduplex with p53 mRNA. In contrast, as shown in the lane 4, it was verified that mutant vegf 5' UTR RNA not involving attenuation of p53 mRNA did not form heteroduplex with mRNA.

(4) Relevance of translation process to attenuation of p53 mRNA mediated by vegf 5' UTR RNA

After the formation of a heteroduplex by vegf 5' UTR and p53 mRNA, a cell-free protein synthesis reaction (cell-free translation reaction) was performed and the influence of a translation process on degradation of p53 mRNA was examined.

(5) Detection of p53 mRNA and its degradation product

In the same manner as in the above-described item (3), mutant 5' UTR RNA or 5' UTR was incubated with p53 mRNA. As described above, mutant 5' UTR RNA and p53 mRNA form a heteroduplex by incubation. Then, using the treated RNA samples, in the presence or absence of cycloheximide (final concentration of 50 $\mu$l/ml) or in the presence of purified ribosome prepared by a sucrose density-gradient centrifugation method, a cell-free protein synthesis reaction was performed. The cell-free protein synthesis was carried out by making reference to the research paper (Huez, I. et al. Mol. Cell. Biol. 18, 6178-6190 (1998)) or using Wako PURE system (trade name, Wako Chemical) following the specifications thereof.

With respect to the reaction solutions, p53 mRNA and its degradation product were analyzed by the Northern blotting. The results are shown in FIG. 8D. FIG. 8D shows the results of the Northern blotting, which indicate p53 mRNA and its degradation product in a reaction solution of cell-free protein synthesis in the presence of vegf 5' UTR RNA. In FIG. 8D, the lanes 2, 5, and 8 "VEGF mut5'" indicate the result obtained regarding the samples in which mutant 5' UTR RNA and p53 mRNA were incubated, the lanes 3, 6, and 9 "VEGF 5'" indicate the result obtained regarding the samples in which 5' UTR RNA (wild-type) and p53 were incubated, and the lanes 1, 4, and 7 "-" indicate the result obtained regarding the samples in which only p53 mRNA was incubated. Further, "+ cycloheximide" in the lanes 4 to 6 means that the cell-free protein synthesis was carried out in the presence of cycloheximide and "+ ribosome" in the lanes 7 to 8 means that the cell-free protein synthesis was carried out in the presence of purified ribosome.

As a result, as shown in FIG. 8D, in the system in which p53 mRNA and 5' UTR RNA formed a heteroduplex (lane 3), partial degradation of p53 mRNA was observed in the absence of cycloheximide. Further, in this system, the partial degradation of p53 mRNA was completely blocked by addition of a translation inhibitor, cycloheximide. Further, partial degradation of p53 mRNA was observed also in a case where only purified ribosome was added to the system in which a heteroduplex was formed. These results show that the interaction between heteroduplex RNA and ribosome started degradation of p53 mRNA mediated by vegf 5' UTR. From this, it is speculated that partial degradation of p53 mRNA triggers further attenuation in cells. In contrast, in the systems with the absence of 5' UTR RNA (lanes 1, 4, and 7), and the systems with the presence of mutant 5' UTR and p53 (lanes 2, 5, and 8), degradation of p53 mRNA did not occur in the absence of cycloheximide.

(6) Detection of p53 protein

Subsequently, the influence of partial degradation of p53 mRNA mediated by 5' UTR on a translation of p53 protein was examined.

In the same manner as in the above-described item (4), RNAs each were incubated in the combination shown in FIG. 8E, and cell-free protein synthesis was carried out. In this state, [$^{35}$S] methionine coexisted in a reaction solution of cell-free protein synthesis. Then, p53 protein synthesized *in vitro* was separated by SDS-PAGE using a polyacrylamide gel of 12%, and a dried gel was subjected to an autoradiography. The results are shown in FIG. 8E. FIG. 8E is an autoradiography showing synthesis of p53 protein in a reaction solution of cell-free protein in the presence of vegf 5' UTR. In FIG. 8E, "VEGF mut5' RNA" indicates mutant 5' UTR, "VEGF 5' RNA" indicates "5' UTR (wild-type)", and "p53RNA" indicates p53 mRNA.

As shown in FIG. 8E, in the system in which p53 mRNA and vegf 5' UTR RNA formed a heteroduplex (lane 3), p53 protein synthesis was markedly suppressed as compared to the system with the absence of vegf 5' UTR (lane 1) and the system with the presence of mutant vegf 5' UTR (lane 2).

<Example A6>

In Example A3, it was found that a molecule of 259 nucleotide (nt), which is a region from base 659 to base 917 of vegf 5' UTR, was required for down regulation of p53. Thus, an element sequence in vegf 5' UTR involving down regulation of p53 was narrowed down further.

(1) Plasmid construct

Exon 1 of a coding gene (vegf gene) of human $VEGF_{165}$ and the following various plasmid constructs were produced based on the aforementioned documents in the same manner as in Example A2. Regions of vegf mRNA to be expressed by the following constructs are shown in FIG. 9.

Construct vegf mRNA (p165 mSPHA3') (full length vegf mRNA)

DNA coding full length vegf mRNA excluding intron was inserted into an XbaI-XhoI site of a plasmid vector pSCT (Bornes, S. et al. J. Biol. Chem. 279, 18717-18726 (2004)). The obtained plasmid construct was referred to as construct vegf mRNA (p165mSPHA3'). In this state, in the cells transfected with this plasmid construct, full length mRNA of a $vegf_{165}$ gene is expressed.

Construct BC019867RNA (16-780)

IMAGE Clone ("Clone ID 4153053", Open Biosystems) was obtained. In the IMAGE Clone, a DNA fragment coding a predetermined region (region from base 16 to base 780) in 5' UTR of exon 1 of a vegf gene is inserted into a NotI-SalI site of a plasmid vector pCMV-SPORT6. This plasmid construct was referred to as a construct BC019867RNA according to NCBI accession No. BC019867. In the cells transfected with this plasmid construct, a region from base 16 to base 780 of vegf mRNA 5' UTR is expressed.

Construct BC011177RNA (526-1216)

IMAGE Clone ("Clone ID 4150451", Open Biosystems) was obtained. In the IMAGE Clone, a DNA fragment coding a region from base 526 to base 1104 of exon 1 of a vegf gene and a region from base 1 to base 112 of intron 1 of the vegf gene is inserted into a NotI-SalI site of a plasmid vector pCMV-SPORT6. This plasmid construct was referred to as a construct BC011177RNA according to NCBI accession No. BC019867. In the cells transfected with this plasmid vector, a region from base 526 to base 1216 of vegf precursor mRNA shown in SEQ ID NO: 45 is expressed.

Construct vegf IresRNA (600-879)

The region from base 659 to base 917 of vegf mRNA 5' UTR specified in Example A3 was analyzed by the Northern blotting using an antisense oligoDNA probe. The results are shown in FIG. 10. FIG. 10 shows the results of the Northern blotting, which indicate RNA fragments of vegf in various human cancer cell strains. As shown in FIG. 10, it was found that small molecule RNA having the chain length of about 200 to 300 bases exists in the various cancer cell strains, and the small molecule RNA is expressed very little in human normal fibroblast. Thus, a fraction including the small molecule RNA was separated by a denatured agarose gel electrophoresis, and the small molecule RNA was cloned by an ordinary method (reference: Kawano, M. et al. Nucleic Acid Res. 33(3):1040-1050 (2005)). When the base sequence of the obtained RNA was determined, it was an RNA product having a region from base 600 to base 879 in 5' UTR of vegf mRNA. This small molecule RNA was named as IRES-related small RNA (IresRNA). Then, the cDNA obtained by the cloning was cloned again to an XhoI-HindIII site of a plasmid vector pcDNA3.1. The thus obtained plasmid construct was referred to as construct vegf IresRNA. In the cells transfected with this plasmid construct, a region from base 600 to base 879 in 5' UTR of vegf mRNA is expressed.

Construct vegf mRNA mutation 1

As shown in FIG. 9, a construct that expresses mRNA having mutation in a region from base 594 to base 745 in 5' UTR of vegf mRNA was produced. Specifically, the following double-stranded fragments 1 and 2, in this order, were connected to a SacII-NheI site of the construct vegf mRNA (p165mSPHA3') from the 5' side. In the following sense strands, non capital letters indicate mutant bases. Thus obtained plasmid construct is referred to as a construct vegf mRNA mutation 1. In the cells transfected with this plasmid, a region from base 594 to base 741 in vegf 5' UTR is mutated.

Fragment 1 (from base 589 to base 639 of vegf 5' UTR)

Sense strand (SEQ ID NO: 37)

5'-GGG CAa GGa CCa GAa CCa GCa CCa GGg GGa GGa GTa GAa GGa GTa GGG GCT-3'

Antisense strand (SEQ ID NO: 38)

5'-CCTACTCCTTCTACTCCTCCCCCTGGTGCTGGTTCTGGTCCTTGCCCGC-3'

Fragment 2 (from base 640 to base 745 of vegf 5' UTR)
Sense strand (SEQ ID NO: 39)

5'-CGa GGa GTa GCa tta AAg CTa TTt GTa CAg tta tta GGa TGc TCa CGa TTt GGg GGg GCa GTa GTa aga GCa GGa Gag GCa GAa CCa AGt GGg GCa GCa AGg AGT G-3'

Antisense strand (SEQ ID NO: 40)

5'-CTAGCACTCCTTGCTGCCCCACTTGGTTCTGCCTCTCCTGCTCTTACTACTGCCCCCCCAAATCGTGAGCATCCTAATAACTGTACAAATAGCTTTAATGCTACTCCTCGAGCC-3'

Construct vegf mRNA mutation 2

As shown in FIG. 9, a construct that expresses mRNA having mutation in a region from base 746 to base 915 in 5' UTR of vegf mRNA was produced. Specifically, the following double-stranded fragments 3 and 4, in this order, were connected to a NheI-XmaI site of the construct vegf mRNA (p165mSPHA3') from the 5' side. In the following sense strands, non capital letters indicate mutant bases. Thus obtained plasmid construct is referred to as a construct vegf mRNA mutation 2. In the cells transfected with this plasmid, a region from base 753 to base 915 in vegf 5' UTR is mutated.

Fragment 3 (from base 746 to base 850 of vegf 5' UTR)

Sense strand (SEQ ID NO: 41)

5'-CT AGC TCa GGa aga GAa GAa CCa CAa CCa GAa GAa GGa GAa GAa GAgGAg GAa AAa Gag GAa GAa cgt GGa CCa CAa TGG agg tta GGa GCa aga AAa CCG GGC T-3'

Antisense strand (SEQ ID NO: 42)

5'-CGGTTTTCTTGCTCCTAACCTCCATTGTGGTCCACGTTCTTCCTCTTTT TCCTCCTCTTCTTCTCCTTCTTCTGGTTGTGGTTCTTCTCTTCCTGAG-3'

Fragment 4 (from base 851 to base 917 of vegf 5' UTR)

Sense strand (SEQ ID NO: 43)

5'-CA TGG ACa GGa GAa GCa GCa GTa TGt GCc Gat AGc GCa CCt GCa GCaaga GCa CCa CAa GCa tta GC-3'

Antisense strand (SEQ ID NO: 44)

5'-CCGGGCTAATGCTTGTGGTGCTCTTGCTGCAGGTGCGCTATCGGCACA TACTGCTGCTTCTCCTGTCCATGAGCC-3'

(2) Expression of p53 mRNA

The p53 expression vectors and each type of the plasmid constructs were cotransfected into H1299 cells (p53 null). In the same manner as in Example A2, each type of transfection cells was cultured, and the expression level of p53 mRNA was analyzed. The results are shown in FIGs. 11A and 11B. In FIGs. 11A and 11B, the first row from the top shows the result of the northern blottin, which indicates expression of p53 mRNA and the second row shows the result of the Northern blotting, which indicates expression of gapdh mRNA. In FIGs. 11A and 11B, "-" indicates the cells transfected with the p53 expression vectors only, which are control for p53 mRNA expression.

As shown in FIG. 11A, in the cells expressing mRNA from base 600 to base 879 (lane 5), as same as in the cells expressing full length vegf mRNA (lane2), expression of p53 mRNA was not observed. Accordingly, together with the results determined in Example A2 (from base 659 to base 917), the sequence involving silencing of p53 expression could be narrowed down to a region from base 659 to 879 of vegf mRNA.

Further, as shown in FIG. 11A, in the cells expressing mRNA from base 16 to base 780 (lane 3) and in the cells expressing mRNA from base 526 to base 1216 in SEQ ID NO: 45 (lane 4), the same as in the cells expressing full length vegf mRNA (lane2), expression of p53 mRNA was not observed. Each of these mRNAs commonly has a region from base 600 to base 780. Accordingly, together with the results determined in Example A2 (from base 659 to base 917), the sequence involving silencing of p53 expression was narrowed down to a region from base 659 to 780 of vegf mRNA.

Moreover, as shown in FIG. 11B, in the vegf mRNA mutation 2 in which a region from base 753 to base 915 was mutated (lane 3), as same as in the cells expressing full length vegf mRNA (lane4), expression of p53 mRNA was not observed. However, in the vegf mRNA mutation 1 in which a region from base 594 to 741 was mutated (lane 2), expression of p53 mRNA was observed at the position similar to that in the control of p53 mRNA (lane 1). Accordingly, together with the results of Example A2 and the results shown in FIG. 11A, it was found that a region from base 659 to base 741 was involved in silencing of p53 expression.

Therefore, for example, by degrading RNA sequences including sequences, for example, a region from base 659 to base 879, a region from base 659 to base 780, and a region from base 659 to base 741 in vegf mRNA, or inhibiting transcription to the aforementioned RNA sequences, suppression of p53 mRNA expression can be avoided. For example, this makes it possible to prevent cancer development and to treat cancers by suppressing growth and survival of cancer cells.

<Example 7A>

vegf 5' UTR-expressing cells were xenografted (transplanted) to a mouse and the influence on growth of cancer cells *in vivo* was examined.

The plasmid construct pVY produced in Example A4 and a newly produced plasmid construct pVY mutation were used. The pVY mutation was produced by cleaving a SacII-NheI site of the construct vegf mRNA mutation 1 produced in Example A6 and replacing with a SacII-NheI site of the pVY. These plasmid constructs were transfected into HCT116 cells (parent cells, wild-type p53) and a clone having tolerance to antibiotic G418 was established by two strains each. The thus obtained each type of the cell strains was referred to as "pVY cell strain (G3 strain and G5 strain)" and "pVY mutant cell strain (D5 strain and E4 strain)". $5 \times 10^6$ of each type of cell strains were xenografted subcutaneously to femoral regions of nude mice using a 27-gauge injection needle. The length and the width of tumors formed after 5, 7, 9, 12, 14, 16, 19, 21, 26, and 28 days from the grafting were measured, and the volume of the tumors was calculated with the following mathematical expression. As a control, cells transfected with pd2EYFP (constructs that only express YFP) were xenografted to mice and the measurement was conducted in the same manner as described above.

$$\text{Volume} = (\text{length} \times \text{width}^{2}) \times 0.5$$

The results are shown in FIGs. 12 and 13. FIG. 12A is a photographs of a mouse to which the pVY cell strain was xenografted, and FIG. 12B is a photographs of a mouse to which the pVY mutant cell strain was xenografted (after 28 days from the grafting). FIG. 12A shows mice to which transfection cells expressing wild-type vegf 5' UTR were xenografted. The photograph on the left indicates the result obtained regarding the pVY cell strain G3 and the photograph on the right indicates the result obtained regarding the pVY cell strain G5. FIG. 12B shows mice to which transfection cells expressing mutant vegf 5' UTR were xenografted. The photograph on the left indicates the result of the pVY mutant cell strain D4 and the photograph on the right indicates the result of the pVY mutant cell strain E4. FIG. 13 is a graph showing change in volume of tumor with time in mice to which these four types of cell strains were xenografted. As shown in FIG. 12A, with respect to the mouse to which the pVY cell strain expressing wild-type vegf 5' UTR was xenografted, tumor of a graft site was obviously enlarged. In contrast, with respect to the mouse to which the pVY mutant cell strain expressing mutant vegf 5' UTR was xenografted, little growth of tumor was seen as shown in FIG. 12B. As obvious from the graph in FIG. 13, increase in change in volume of tumor with time was observed with mice (black circle and black square) to which the pVY cell strains expressing wild-type 5' UTR were xenografted. In contrast, mice (white circle and white square) to which the pVY mutant cell strains expressing mutant 5' UTR were xenografted showed results rarely different from the result of a control mouse (white triangle). These results demonstrate that vegf 5' UTR RNA markedly enhances formation of tumor *in vivo.*

<Example A8>

Tolerance to anticancer agents that is given to cells by vegf 5' UTR associated RNA was examined.

In the same manner as in Example A6, the construct vegf mRNA, the construct BC019867 RNA(16-780), construct BC011177 RNA(526-1216), and construct vegf IresRNA (600-879) were transfected into HCT116 cells (parent cells, wild-type p53). Then, these cells were treated with various anticancer agents having the final concentrations as described below for 60 hours. With respect to the treated cells, the proportion (%) of apoptosis was quantified in the same manner as in Example A1. As the anticancer agent, camptotecin (40 nmol/L), cisplatin (2 μg/ml), doxorubicin (160 nM), etoposide (60 μmol/L), 5-FU (80 μmol/L), and the combination of leucovorin (5 μmol/L) and 5-FU (10 μmol/L) were used. Further, as a control cell, a cell transfected with pcDNA3.1, an empty vector, was used.

The results are shown in FIG. 14. FIG. 14 indicates proportion of apoptosis in cells expressing various vegf 5' UTR associated RNA, treated with various anticancer agents. In FIG. 14, each bar, from the left, indicates the result obtained regarding the control and transfection with the construct vegf mRNA, the construct BC019867 RNA, the construct BC011177 RNA, and the construct vegf IresRNA (600-879). Since apoptosis normally is induced by anticancer agents, these results show whether or not the effects of anticancer agents were suppressed by vegf 5' UTR associated RNA expressed in cells, i.e., whether or not tolerance to anticancer agents was given to the cells. As shown in FIG. 14, with respect to all cells, apoptosis (%) lower than that of the control cell was observed. In other words, it was found that the cells show tolerance to anticancer agents by vegf 5' UTR associated RNA expressed in the cells. Accordingly, by suppressing the expression of vegf 5' UTR associated RNA in cancer cells or by degrading expressed vegf 5' UTR associated RNA, anticancer agent sensitivity of cells can be enhanced.

<Example A9>

In Example A8, it was found that vegf 5' UTR RNA give anticancer agent tolerance to cells. Thus, expressed vegf 5' UTR RNA was degraded by vegf 5' UTR-targeting siRNA to ascertain enhancement of the anticancer agent sensitivity in cells.

The recombinant vectors that express vegf siRNA#1 and #2 and the vectors that express control siRNA were transfected into HCT116 cells (parent cells, wild-type p53). As a control cell, an HCT116 cell not transfected with a recombinant vector that expresses siRNA was used. These four types of cells were treated in the presence or absence of various anticancer agents having the final concentrations as described below for 60 hours. As the anticancer agent, etoposide (20 μmol/L), doxorubicin (170 nmol/L), and 5-FU (40 μmol/L) were used. With respect to the treated cells, apoptosis (%) was quantified in the same manner as in Example A1.

When vegf mRNA is knocked down by expression of vegf siRNA#1 or siRNA #2, VEGF protein also is not synthesized. It has been known that VEGF protein acts as survival factors for cancer cells, and leads anticancer agent tolerance. In other words, vegf siRNA blocks both function of vegf 5' UTR RNA and function of VEGF protein. With the aim of clarifying an effect of blocking the function of vegf 5' UTR RNA, recombinant VEGF protein (R&D systems, hereinafter referred to as "rVEGF') was added to a culture supernatant of cells transfected with vegf siRNA in order to add VEGF protein that is decreased due to siRNA. The experiment showed that the level of VEGF protein constantly secreted by the HCT116 cells was 0.7 to 1 ng/ml. Therefore, in Example A9, 1 ng/ml of rVEGF protein was added to the four types of cells. Thereby, the enhancement of anticancer agent sensitivity due to knockdown of vegf 5' UTR RNA truly caused by siRNA was ascertained. Then, these transfection cells were treated with anticancer agents in the same manner as described above.

The results are shown in FIG. 15. FIG. 15 is graphs showing apoptosis (%) in each type of the cells, treated with various anticancer agents. It can be judged that the higher the proportion of apoptosis is, the more anticancer agent sensitivity is enhanced. FIG. 15A shows the result of apoptosis indicating anticancer agent etoposide sensitivity, FIG. 15B shows the result of apoptosis, which indicates anticancer agent doxorubicin sensitivity, and FIG. 15C shows the result of apoptosis, which indicates anticancer agent 5-FU sensitivity. In FIGs. 15A to 15C, "NT" indicates the cells not transfected with recombinant vectors that express siRNA, "control siRNA" indicates the cells expressing control siRNA, "VEGF siRNA#1" indicates the cells expressing siRNA#1, and "VEGF siRNA#2" indicates the cells expressing siRNA#2. Further, in FIGs. 15A to 15C, open bars (white rectangle) indicate the cells not treated with anticancer agents, solid bars (black rectangle) indicate the cells treated with anticancer agents, and shaded bars indicate the cells treated with rVEGF and anticancer agents.

In any of FIGs. 15A to 15C, in the cells not expressing siRNA(NT) and in the cells expressing control siRNA (control siRNA), significant enhancement of apoptosis was not induced even when etoposide (20 μmol/L), doxorubicin (170 nmol/L), and 5-FU (40 μmol/L) of low concentrations were added. These results show that cancer cells, in which vegf 5' UTR RNAs are not knocked down, have anticancer agent tolerance. In contrast, in the cells expressing siRNA#1 or the cells expressing siRNA#2, as compared to the cells not treated with anticancer agents (white bars), apoptosis was enhanced markedly by treatments with anticancer agents of low concentrations. Further, this enhancement of apoptosis was affected very little even when cells were treated with rVEGF protein (shaded bars). In other words, it can be considered that enhancement of sensitivity to anticancer agents was caused not by decrease in endogenous VEGF protein due to siRNA but mainly by knockdown of vegf 5' UTR RNA (including vegf mRNA) due to siRNA. These results showed importance of considering vegf 5' UTR associated RNA as a treatment target in addition to VEGF protein that has been considered as an important target in cancer treatments.

<Example A10>

Enhancement of anticancer agent sensitivity of cells by each type of the siRNAs was examined.

The aforementioned vegf siRNA#1 vectors to vegf siRNA#5 vectors and the control siRNA vectors were transfected into HCT116 cells (parent cells, wild-type p53). Then, the transfection cells were treated with various anticancer agents having the final concentrations as described below for 60 hours. As an anticancer agent, doxorubicin (170 nmol/L); etoposide (20 μmol/L); 5-FU (40 μmol/L); the combination of leucovorin (5 μmol/L) and 5-FU (10 μmol/L); mitomycin C (1 μg/mL); paclitaxel (5 nmol/L); vincritine (12.5 nmol/L); and cisplatin(1 μg/ml) were used. Then, with respect to the treated cells, the relative value of the survival rate (%) was calculated. The relative value was obtained with the number of viable cells transfected with the recombinant vectors that express control siRNA being considered as 100%.

The results are shown in FIG. 16. FIG. 16 is a graph showing a relative survival rate (%) of cells transfected with each type of the recombinant vectors, treated with anticancer agents. In FIG. 16, each bar, from the left, indicates the result obtained regarding transfection with the control siRNAvectors, and the vegf siRNA#1 vectors to the vegf siRNA#5 vectors. As shown in FIG. 16, as compared to the cells expressing control siRNA, in the cells expressing siRNA#1 to siRNA#5, the relative value of viable cells was sufficiently and reliably decreased. These results showed that the anticancer agent sensitivity of cells also can be increased by any one of siRNA#1 to siRNA#5. From this result, it can be said that growth and survival of cancer cells can efficiently be inhibited by attacking vegf 5' UTR RNA with siRNA #1 to siRNA #5, and these siRNAs are very useful as anticancer agents or concomitant drugs of known anticancer agents.

<Example A11>

A construct that expresses partially deleted p53 mRNA was produced, and an element sequence interacting with vegf 5' UTR was determined.

FIG. 17A schematically shows the produced constructs. CDS of a p53 gene, cDNA (Δ 5' p53) in which the 5' region of the CDS was deleted, and cDNA in which the 3' region of the CDS was deleted were produced by PCR. The CDS corresponds to RNA from base 252 to base 1434 in a complete sequence of human p53 mature mRNA shown in SEQ ID NO: 2. The Δ 5' p53 is cDNA corresponding to RNA in which a region from base 252 to base 551 is deleted from a region from base 252 to 1434 in SEQ ID NO: 2. The Δ 3' p53 is cDNA corresponding to RNA in which a region from base 1133 to base 1433 is deleted from a region from base 252 to base 1434 in SEQ ID NO: 2. These cDNAs each were inserted into a BamHI/EcoRI site of a pcDNA3.1 vector (Invitrogen). These plasmid constructs were coexpressed with a plasmid construct A (pVC) or a plasmid construct H (pVCmut5') in H1299 cells. Then, with respect to these cells, the expression level of exogenous p53 mRNA was detected by an RT-PCR method. The results are shown in a photograph in FIG. 17B. In FIG. 17B, "pVC" indicates a construct A and "mut5'" indicates a construct H.

As shown in FIG. 17B, Δ 5' p53 in which 5'-end is deleted was not silenced by vegf 5' UTR RNA. In contrast, Δ 3' p53 in which 3'-end is deleted was silenced by vegf 5' UTR RNA in the same manner as wild-type p53 mRNA. This result showed that a region interacting with vegf 5' UTR in p53 mRNA was RNA including a region from base 252 to base 551 in SEQ ID NO: 2.

[Example B1]

Malignant transformation of cells expressing vegf IresRNA

In order to clarify whether or not vegf IresRNA promotes the malignant progression of tumor cells, the following experiment was conducted.

(1) Expression of vegf IresRNA

Full length cDNAs of vegf IresRNA having a sequence from base 600 to base 879 in 5' UTR of vegf mRNA of SEQ ID NO. 1were inserted into plasmid vectors pcDNA3.1 (Invitrogen). Thus, vegf IresRNA expression vectors (pcDNA-vegf Ires) were produced. These vegf IresRNA expression vectors (pcDNA-vegf Ires) were transfected into HCT116 (parent cells, wild-type p53), and two types of cell clones that stably overexpress vegf IresRNAwere selected with an antibiotic G418. The thus-established two types of vegf IresRNA-expressing cells are hereinafter referred to as vegf Ires-#1 and vegf Ires-#2. Furthermore, as a mock treatment, plasmid vectors pcDNA3.1 lacking the above-described insert were transfected into HCT116 cells, and the transfect cell clones were selected with the antibiotic G418. These cell clones were hereinafter referred to as a mock. Then, expression of vegf IresRNA in these cell clones was examined by Northern blotting.

The results are shown in FIG. 18A. FIG. 18A shows the results of the Northern blotting, which indicate the RNA expression in each type of the cells. The first row from the top indicates the expression of vegf IresRNA, and the second row indicates the expression of gapdh mRNA as a loading control. As can be seen from FIG. 18A, little expression of vegf IresRNA was seen in the mock. In contrast, in each of the vegf Ires-#1 and vegf Ires-#2 expressing vegf IresRNA, expression of vegf IresRNA was seen.

(2) Influence of vegf IresRNA on p53 mRNA expression-inducing ability of cisplatin

Subsequently, each of these vegf IresRNA-stably expressing cell strains was treated in the presence or absence of 5 μg/mL of an anticancer agent cisplatinin for a predetermined time (0, 6, 8, 12, 24 h). Thereafter, the expression level of p53 mRNA was analyzed by quantitative RT-PCR, and the expression level of p53 protein was analyzed by Western immunoblotting. Also, with respect to the mock cells, the same treatment and analyses of the expression levels were conducted.

The results are shown in FIGs. 18B and 18C. FIG. 18B shows the result indicating the amount of the p53 mRNA expressed in each type of the cells, and the vertical axis represents the ratio between the amount of the expressed p53 mRNA and the amount of the expressed gapdh mRNA as a control. FIG. 18C shows the result indicating the amount of the p53 protein expressed in each type of the cells. As can be seen from FIGs. 18B and 18C, as compared with the mock not treated with the cisplatin (0 h), a significant increase in the expressions of p53 mRNA and p53 protein was observed in the mocks treated with the cisplatin (6 h, 12h), because the expression of p53 mRNA was induced by the cisplatin. In contrast, in the vegf IresRNA-#1 and vegf Ires-#2 not treated with the cisplatin (0 h), a decrease in the basal expression level of endogenous p53 mRNA and p53 protein was seen as compared with the mock. Furthermore, in the vegf IresRNA-#1 (6 h, 12 h) and the vegf Ires-#2 (6 h, 12 h), induction of the expressions of p53 mRNA and p53 protein was not observed, as opposed to the case of the mocks (6 h, 12 h). As described above, the p53 mRNA expression-inducing ability of the cisplatin, which was demonstrated in the mocks, was lowered markedly by the expression of the vegf IresRNA. This demonstrates that vegf IresRNA inhibits the induction of p53 expression.

(3) Influence of vegf IresRNA on apoptosis caused by various anticancer agents

Resistance of various types of cells to apoptosis imparted by anticancer agents 5-FU, etopside, mitomycin C (MMC), and cisplatin, and the influence of vegf IresRNA on apoptosis were examined. The vegf Ires-#1, the vegf Ires-#2, the mocks, and the HCT116 (the parent cells) not transfected with the vectors in the above item (2) were treated for 60 hours with each of 5-FU (120 μmol/L), etoposide (10 μmol/L), MMC (6 μg/ml), and cisplatin (5 μg/ml), thereby inducing apoptosis. The cell apoptosis after the induction was detected by the method described in "Apoptotic evaluation 2" above (means ± SD, n = 4).

The results are shown in FIG. 18D. FIG. 18D is a graph showing the proportion of apoptosis positive cells in each type of the cells. In FIG. 18D, "parental" indicates the result of the parent cells HCT116. As can be seen from FIG. 18D, in the HCT116 (parental) and the mock, apoptosis was induced in around 30% of the cells by the stimulation of any of the anticancer agents and no resistance was shown to any of the anticancer agents. In contrast, in the vegf IresRNA-#1 and the vegf Ires-#2, apoptosis was induced only in about 10% of the cells by the stimulation of any of the anticancer agents. This proportion was significantly low as compared with that in the HCT116 (parental) and the mock. From this result, it was found that vegf IresRNA imparts apoptosis resistance to cells.

(4) Malignant transformation of cells *in vivo* by vegf IresRNA

Subsequently, the vegf Ires-#1, the vegf Ires-#2, and the mock ($5 \times 10^6$ cells) described in the above item (2) respectively were xenografted subcutaneously to femoral regions of athymic nude mice using a 27-gauge injection needle. After a lapse of a predetermined time (0, 5, 9, 13, 17, 21 days) from the grafting, the volume of the formed tumor was measured in the same manner as in Example A7 (means ± SEM, n = 5). As a control, HCT116 cells not transfected with expression vectors were xenografted to mice and the volume of the tumor was measured in the same manner. The results are shown in FIG. 18E. FIG. 18E is a graph showing the change in the tumor volume with time in the mice xenografted with these cell strains. Also, the vegf Ires-#1 and the control HCT116 cells not transfected with expression vectors ($1 \times 10^6$) were respectively xenografted intraperitoneally to nude mice using a 27-gauge injection needle, and the formed tumor was observed by opening the abdomen on the 35th day after the grafting. The results are shown in FIG. 19F. FIG. 19F shows photographs of the opened abdomens of the mice, and the arrow heads point the formed tumor. In FIG. 19F, the photograph on the left shows the result obtained when the cells expressing vegf IresRNA were xenografted, and the photograph on the right shows the result obtained when the control cells were xenografted.

As can be seen from FIG. 18E, in each of the vegf Ires-#1 and vegf Ires-#2, the tumor obviously larger than that in the mock was formed in a short time. Furthermore, as can be seen from FIG. 19F, also from the intraperitoneally-xenografted models allowing the evaluation of the morphology of highly malignant tumor cells, it became clear that the cells expressing vegf IresRNA exhibits remarkable tumorigenicity as compared with the control cells. From the above results, it was revealed that vegf IresRNA markedly enhances tumor formation *in vivo*.

[Example B2]

<u>Effect of suppressing tumorigenicity of cells by vegf siRNA</u>

With regard to cells expressing siRNA against vegf, the effect thereof on the tumorigenicity *in vivo* was examined. Vegf siRNA#1 vectors expressing the above-described siRNA#1 were transfected into a HCT116 cell strain (wild-type p53) derived from human large bowel cancer, and the transfected cells were selected with an antibiotic blasticidin. The thus-obtained cell clone hereinafter is referred to as a si-vegf #1 cell strain. As a control, pcDNA6.2-GW/EmGFP-miR vectors (Invitrogen) to which a double-stranded oligonucleotide with a sequence (SEQ ID NO. 46: 5'-gaaatgtactgcgcgtggagacgttttggccactgactgacgtctccacgcagtacattt-3') having no homology to known vertebrate genes had been inserted were used (control siRNA vectors) as in the above. The HCT16 cell strain was transfected with these control siRNA vectors in the same manner as in the above, and the transfected cells were selected with the antibiotic. The thus-obtained cell clone hereinafter is referred to as a si-Control cell strain. Then, the si-vegf #1 cell strain and the si-Control cell strain respectively were xenografted subcutaneously to femoral regions of nude mice, and the volume of the formed tumor was measured after the predetermined time (each n = 5) in the same manner as in Example C2.

The results are shown in FIG. 20. FIG. 20 is a graph showing the change in the tumor volume with time in the mice xenografted with these cell strains. In FIG. 20, #1 to #5 si-vegf #1 represented by open symbols indicate the results obtained when the si-vegf #1 cells expressing the siRNA #1 were xenografted, and #1 to #5 controls represented by solid symbols indicate the results obtained when the si-Control cells were xenografted.

As can be seen from FIG. 20, in the mice xenografted with the si-Control cells (control), the growth of the tumor with time was observed. In contrast, in the mice xenografted with the si-vegf #1 cells expressing the siRNA#1 (si-vegf #1), significant suppression of the tumor formation and the tumor growth were observed. These results demonstrate that, by siRNA that targets exon 1 in vegf mRNA, tumor formation and growth of the formed tumor can be suppressed significantly.

[Example B3]

<u>Promotion of apoptosis sensitivity by vegf siRNA</u>

(1) Suppression of expression of vegf mRNA or vegf IresRNA by vegf siRNA

Whether or not vegf siRNA promotes the apoptosis sensitivity of cells was examined. In the same manner as in Example A1 except that HCT116 cells (parent cells, wild-type p53) were used instead of the HepG2 cells (wild-type p53), the vegf siRNA#1 vectors expressing siRNA#1, the vegf siRNA #2 vectors expressing siRNA #2, and the control siRNA vectors described above were respectively transfected into the HCT116 cells (parent cells, wild-type p53). Then, with regard to the cells after 18 hours from the transfection, the expression levels of vegf mRNA and gapdh mRNA were analyzed by quantitative RT-PCR (means $\pm$ SD, n = 4) and the expression levels of vegf IresRNA and gapdh mRNA were analyzed by Northern blotting. Also, with regard to HCT116 cells not transfected with the above-described recombinant vectors, the analyses were conducted in the same manner. The quantitative RT-PCR was conducted in the same manner as in Example B1, except that a primer set for vegf mRNA was used.

The results are shown in FIG. 21A. In FIG. 21A, the graph on the left shows the result of the analysis of the amount of the vegf mRNA expressed in each type of the cells, and the vertical axis represents the ratio between the vegf mRNA and the gapdh mRNA. In FIG. 21A, photographs on the right show the result of the Northern blotting, which indicate the expression of the vegf IresRNA in each type of the cells. The upper row shows the result obtained regarding the vegf IresRNA and the lower row shows the result obtained regarding the gapdh mRNA. In FIG. 21A, "none" indicates the result obtained regarding the cells not transfected with the recombinant vectors, "si-Control" indicates the result obtained regarding the cells transfected with the control siRNA vectors, "si-vegf #1" indicates the result obtained regarding the cells transfected with the vegf siRNA#1 vectors, and "si-vegf #2" indicates the result obtained regarding the cells transfected with the vegf siRNA #2 vectors (hereinafter the same).

As can be seen from the graph on the left of FIG. 21A, by the transfection of the vegf siRNA#1 vectors or the vegf siRNA#2 vectors, the amount of the vegf mRNA expressed in HCT 116 was decreased markedly. Furthermore, as can be seen from the photographs on the right of FIG. 21A, by the transfection of the vegf siRNA#1 vectors or the vegf siRNA#2 vectors, the amount of the expressed vegf IresRNA was decreased as compared with that in the control cells. In particular, in the cells transfected with the vegf siRNA #2 vectors, the expression of the vegf IresRNA was decreased markedly. The above-described results demonstrate that knockdown of vegf IresRNA can be achieved by the siRNA #1 and the siRNA #2.

(2) Promotion of apoptosis sensitivity by vegf siRNA

Various types of cells were treated with 5-FU, which is an anticancer agent inducing apoptosis in a p53-dependent manner, and the sensitivity to apoptosis was analyzed. The vegf siRNA#1 vectors expressing siRNA#1, the vegf siRNA #2 vectors expressing siRNA #2, and the control siRNA vectors described above respectively were transfected into HCT 116 (parent cells) and p53-deleted cells HCT116. Then, the cells after 18 hours from the transfection was treated for 60 hours in the presence of 80 $\mu$mol/L of 5-FU (+) or in the absence of 5-FU (-). The proportion (%) of apoptosis positive cells in the thus-treated cells was calculated based on "Apoptotic evaluation 2" above (means $\pm$ SD, n = 4).

The results are shown in FIG. 21B. FIG. 21B is a graph showing the apoptosis (%) in each type of the cells. In FIG. 21B, "parental HCT116" indicates the parent cells HCT116 expressing p53, "p53-KO HCT116" indicates the p53-deleted HCT116 cells, "no transfection" indicates the cells not transfected with the recombinant vectors, and "si-Control", "si-veg#1", and "si-vegf #2" are the same as those in FIG. 21A. With regard to "5-FU", "+" indicates the treatment in the presence of 5-FU and "-" indicates the treatment in the absence of 5-FU (hereinafter the same). As can be seen from FIG. 21B, the proportion of apoptosis in the p53-deleted cells HCT116 was increased slightly by the treatment with 5-FU. However, little increase was observed even if the siRNA#1 or the siRNA#2 further was caused to be expressed. In contrast, in the parent cells HCT116, the proportion of apoptosis was increased markedly by causing the siRNA#1 or the siRNA#2 to be expressed and conducting the treatment with 5-FU. These results demonstrate that the improvement in apoptosis sensitivity by the siRNA #1 and the siRNA #2 depends on p53.

(3) Promotion of expressions of p53 mRNA and p53 protein by vegf siRNA

From the results obtained in the above (1) and (2), it is interpreted that vegf IresRNA exerts an adverse influence in the p53 cancer suppression pathway. Thus, the expression level of endogenous p53 in cells transfected with vegf siRNA was examined. Specifically, the same transfected parent cells HCT166 as in the above were treated for 8 hours in the presence of 80 μmol/L of 5-FU (+) or in the absence of 5-FU (-), after which expression of p53 mRNA was analyzed by quantitative RT-PCR (means ± SD, n = 4). Also, the transfected cells were treated in the presence of 80 μmol/L of 5-FU (+) for a predetermined time (0, 8, 16, 24 hours), after which p53 protein was detected by the Western immunoblotting described above.

The results are shown in FIGs. 21C and 21D. FIG. 21C is a graph showing the amount of the p53 mRNA expressed in each type of the cells, and the vertical axis represents the ratio between the p53 mRNA and the gapdh mRNA. In FIG. 21C, with regard to "5-FU", "+" indicates the treatment in the presence of 5-FU and "-" indicates the treatment in the absence of 5-FU. FIG. 21D shows the result of the Western immunoblotting, which indicates the expression of p53 protein in each type of the cells. In FIG. 21D, each value of 5-FU indicates the time (hour) of the treatment with 5-FU. In FIGs. 21C and 21D, "si-Control", "vegf #1", and "si-vegf #2" are the same as those in FIG. 21A. As can be seen from FIG. 21C, in the cells caused to express the siRNA#1 or the siRNA#2, the amount of the expressed mRNA was increased as compared with the control by the decrease in vegf IresRNA. By further treating these cells with 5-FU, the amount of the expressed p53 mRNA could be increased markedly. Furthermore, as can be seen from FIG. 21D, in the cells caused to express siRNA#1 or siRNA#2, the amount of the expressed p53 protein also was increased similarly as compared with the control. By further treating these cells with 5-FU, the amount of the expressed p53 protein could be increased markedly. From the above results, it was found that the expressions of p53 mRNA and p53 protein induced by 5-FU are increased by the knockdown of vegf IresRNA by vegf siRNA.

(4) Promotion of expression of mRNA induced via p53 by vegf siRNA

Whether or not the increase in the 5-FU-induced expression of p53 mRNA caused by the knockdown of vegf IresRNA by vegf siRNA induces expression of mRNA of a bax gene or a noxa gene, which are both p53 target genes, was examined. Cells obtained by transfecting the same control siRNA vectors, vegf siRNA#1 vectors, or vegf siRNA #2 vectors as in the above into parent cells HCT116 were treated for 24 hours in the presence of 80 μmol/L of 5-FU (+) or in the absence of 5-FU (-). Thereafter, expressions of bax mRNA and noxa mRNA were analyzed by quantitative RT-PCR (means ± SD, n = 4).

The results are shown in FIG. 22E. FIG. 22E shows graphs each indicating the amount of the expressed mRNA of the p53 target gene. In FIG. 22E, the graph on the left shows the result regarding the bax mRNA, and the vertical axis represents the ratio between the bax mRNA and gapdh mRNA. In FIG. 22E, the graph on the right shows the result regarding the noxa mRNA, and the vertical axis represents the ratio between the noxa mRNA and gapdh mRNA. As can be seen from FIG. 22E, induction of the expressions of both the bax mRNA and the noxa mRNA by the treatment with 5-FU was promoted markedly as compared with the control by the expression of the siRNA #1 or the siRNA #2. From this result, it can be said that, by the expression of the siRNA #1 or the siRNA #2, the amount of expressed apoptosis-related gene such as bax, noxa, or the like is increased, resulting in the improvement in apoptosis sensitivity.

(5) The relationship among p53 mRNA, vegf IresRNA, and vegf mRNA

HCT116 cells (parent cells) were exposed to genotoxic stress that upregulates the expression of p53 mRNA. Specifically, the HCT116 cells were treated with 5-FU (80 μmol/L), mitomycin C (MMC, 6 μg/ml), or cisplatin (5 μg/ml) for a predetermined time (0, 6, 12 hours), after which the expressions of p53 mRNA, vegf IresRNA, and vegf mRNA were examined by Northern blotting. Also, HCT116 cells were exposed to UV-B for 1 minute. Then, after placing them under non-exposed conditions for a predetermined time (6, 12 hours), the expressions were examined in the same manner as in the above.

The results are shown in FIG. 22F. As can be seen from FIG. 22F, in the cells subjected to the stress with 5-FU, MMC, or UV irradiation, both the vegf IresRNA and the vegf mRNA increased markedly, whereby the expression of the p53 mRNA was suppressed. In contrast, cisplatin increased the expression of the p53 mRNA and decreased the expression of the vegf IresRNA. This result supports the conclusion that cisplatin inhibited the expression of vegf mRNA and the subsequent generation of vegf IresRNA. This inverse correlation supports the fact that vegf IresRNA acts as a repressor of p53 expression. Also, it has been reported that p53 suppresses VEGF expression at the transcriptional level. Thus, it is considered that vegf IresRNA provides a positive feedback loop for VEGF production through the silencing of the p53 expression.

[Example B4]

Identification of interaction region with p53 mRNA in vegf IresRNA

(1) Influence of vegf IresRNA on expression of p53 mRNA

Vegf IresRNA expression vectors (pcDNA-vegf Ires) were produced in the same manner as in Example B1. The p53 expression vectors of Example A2 and the vegf IresRNA expression vectors (pcDNA-vegf Ires) were cotransfected into p53-deleted HCT116 cells (p53 null). After 18 hours from the cotransfection, expressions of p53 mRNA and vegf IresRNA were examined by Northern blotting. Furthermore, as a mock treatment, expression plasmid vectors pcDNA3.1 lacking an insert and the p53 expression vectors were cotransfected into p53-deleted HCT116 cells. This cell clone hereinafter is referred to as a mock. With regard to the mock, expressions of the RNA were checked in the same manner.

The results are shown in FIG. 23A. In FIG. 23A, the photograph on the left shows the result of the Northern blotting, which indicates the expression of p53 mRNA in each type of the cells, and the photograph on the right shows the result of the Northern blotting, which indicates the expression of vegf IresRNA in each type of the cells. In FIG. 23A, "mock" indicates the cells subjected to the mock treatment, "vegf IresRNA" indicates the cells transfected with the vegf IresRNA expression vector, p53 "+" indicates that the transfection of p53 expression vectors was conducted, and "-" indicates that the transfection of the same was not conducted. As can be seen from FIG. 23A, in the cells not expressing vegf IresRNA indicated as "mock", p53 mRNA was detected strongly. In contrast, in the cells expressing vegf IresRNA indicated as "vegf IresRNA", degradation of p53 mRNA was inducted. These results suggest that vegf 5' UTR, especially vegf IresRNA, suppresses expression of p53 after the transcription.

(2) Identification of interaction region with p53 mRNA in vegf IresRNA

From the above-described results, it became clear that vegf IresRNA negatively-controls the amount of expressed p53 mRNA after the transcription. Thus, the interaction region with p53 mRNA in vegf IresRNA further was identified.

The secondary structures of vegf IresRNA and p53 mRNA were predicted using mFold 3.2, which is a program for predicting the secondary structures of nucleic acids. Then, based on the predicted secondary structures of them, the regions in vegf IresRNA and p53 mRNA complementary to each other are predicted using the Vienna RNAfold algorithm. The results are shown in FIGs. 23B and 23C. FIGs. 23B and 23C show the alignment of the complementary regions of the vegf IresRNA and the p53 mRNA, respectively. As can be seen from FIG. 23B, the complementarity was found between a region from base 694 and base 714 of vegf IresRNA in SEQ ID NO. 1 and a region from base 462 to base 481 of p53 mRNA in SEQ ID NO. 2. Also, as can be seen from FIG. 23C, the complementarity was found between a region from base 693 to base 714 of vegf IresRNA in SEQ ID NO. 1 and a region from base 462 to base 491 of p53 mRNA in SEQ ID NO. 2. It was found that the region from base 693 to base 714 in SEQ ID NO. 1 forms a stem-loop in the secondary structure of the vegf IresRNA. FIG. 24D schematically shows the secondary structure (the stem-loop structure) of vegf IresRNA.

(3) Influence of mutation of stem-loop in vegf IresRNA

Whether or not the stem-loop structure in vegf IresRNA is involved in the silencing of p53 mRNA was examined by the following method. Expression vectors (del-1) and (del-2) respectively expressing vegf IresRNA (del-1) and vegf IresRNA (del-2) in which the stem-loop of vegf IresRNA is partially deleted were produced. The deleted part in the vegf IresRNA is shown in FIG. 24E. FIG. 24E shows only the region that is in the square in the secondary structure of vegf IresRNA shown in FIG. 24D. In FIG. 24D, the vegf IresRNA (del-1) is shown on the left and the vegf IresRNA (del-2) is shown on the right. In the vegf IresRNA (del-1), the region from base 693 to base 714 in vegf IresRNA is partially deleted. Specifically, as can be seen from FIG. 24E, the region from base 691 to base 703 forming the basal portion of the stem-loop is missing. In the vegf IresRNA (del-2), the region from base 711 to base 718 and the region from base 721 to base 723 in vegf IresRNA are deleted. Specifically, as can be seen from FIG. 24E, it forms a short stem-loop without the head loop portion.

The expression vectors (del-1) and (del-2) were produced in the following manner. First, the vegf IresRNA expression vectors (pcDNA-vegf Ires) of Example B1 were cleaved by NheI, and oligonucleotides coding vegf IresRNA (del-1) and vegf IresRNA (del-2) were inserted thereto. The oligonucleotide coding the vegf IresRNA (del-1) and the oligonucleotide coding the vegf IresRNA (del-2) are shown below.

del-1 oligonucleotide (SEQ ID NO. 91)

5'-gctagcggagcccgcgcccggaggcggggtggagggggtcggggctcgcggcgtcgcactgaaacttttc
gtccaacttctgggctgttctcgcttctccgcgcgggggaagccgagccgagcggagccgcgagaagtgctagc
-3'

del-2 oligonucleotide (SEQ ID NO. 92)

5'-gctagcggagcccgcgcccggaggcggggtggagggggtcggggctcgcggcgtcgcactgaaacttttc
gtccaacttctgggctgttctcgcttcggaggagccgtggtccgcgcccccgagcggagccgcgagaagtgcta
gc-3'

Then, one type of vectors selected from the expression vectors (del-1), the expression vectors (del-2), and the vegf IresRNA expression vectors, and the p53 expression vectors of Example A2 were cotransfected into p53-deleted HCT116. After 18 hours, expressions of p53 mRNA and vegf IresRNA were examined by Northern blotting. Furthermore, as a mock treatment, expression plasmid vectors pcDNA3.1 lacking the insert and the p53 expression vectors were cotransfected into p53-deleted HCT116, and the expressions were examined in the same manner. The culture of the HCT116 (p53 null) was carried out using McCoy's 5A medium containing 10% fetal bovine serum and 100 μg/ml (100 units/ml) of streptomycin under the conditions of 37°C and 5% $CO_2$ (hereinafter the same).

The results are shown in FIG. 24F. FIG. 24F shows the result of the Northern blotting, which indicates the RNA expression in each type of the cells. The first row from the top indicates the expression of p53 mRNA, the second row indicates the expression of vegf IresRNA, and the third row indicates the expression of gapdh mRNA as a loading control. Furthermore, p53 "+" means that the transfection of p53 expression vectors was conducted. In FIG. 24F, "vegf IresRNA (wt)" indicates the cells transfected with the expression vectors vegf IresRNA, "vegf IresRNA (del-1)" indicates the cells transfected with the expression vectors vegf IresRNA (del-1), and "vegf Ires (del-2)" indicates the cells transfected with the expression vectors (del-2). As can be seen from "vegf IresRNA (wt)" in FIG. 24F, when the full-length vegf IresRNA was expressed together with p53 mRNA, silencing of the p53 mRNA and the vegf IresRNA was observed. On the other hand, as can be seen from "vegf IresRNA (del-1)" and "the vegf IresRNA (del-2)", in the case where the partial deletion mutant of the vegf IresRNA was expressed together with p53, the amount of the expressed p53 mRNA was the same as that in the mock, and the degradation of the vegf IresRNA was not observed. Thus, it is demonstrated that the vegf IresRNA (del-1) and the vegf IresRNA (del-2) do not have p53-silencing ability. From the above-described results, it was found that the stem-loop structure in the vicinity of the region from base 693 to base 714 in vegf IresRNA plays an important role in the silencing of p53 mRNA by vegf IresRNA.

(4) Binding with p53 mRNA via stem-loop of vegf Ires mRNA

The possibility that vegf IresRNA might bind to p53 mRNA via the above-described stem-loop structure was examined. First, in the same manner as in the above, the p53 expression vectors described in the section of "RNA pull-down assay" and each types of the various vegf IresRNA expression vectors were cotransfected into p53-deleted HCT116. Then, by the RNA pull-down assay described above, the presence or absence of amplification of the PCR products was examined.

The results are shown in FIG. 24G. In FIG. 24G, "vegf IresRNA (wt)" indicates the cells transfected with the vegf IresRNA expression vectors, "vegf IresRNA (del-1)" indicates the cells transfected with the expression vectors vegf IresRNA (del-1), and "vegf IresRNA (del-2)" indicates the cells transfected with the expression vectors vegf IresRNA (del-2). "RT (-)" indicates the negative control lacking transcriptase, and "vegf IresRNA cDNA" and "p53 cDNA" indicate the PCR positive controls. As can be seen from "vegf IresRNA (wt)" in FIG. 24G, in the case where the wild-type full-length vegf IresRNA and p53 were expressed, the amplification of p53 was observed. From this result, it was found that the p53 mRNA is bound to the wild-type full-length vegf IresRNA. On the other hand, as can be seen from "vegf IresRNA (del-1)" and "vegf IresRNA (del-2)", the partial deletion mutant of vegf IresRNA was expressed together with p53, amplification of p53 was not observed. From this result, it was found that the p53 mRNA does not bind to the vegf IresRNA (del-1) and the vegf IresRNA (del-2). Thus, it was suggested that p53 mRNA and vegf IresRNA bind to each other via the stem-loop structure in the vicinity of the region from base 693 to base 714 of the vegf IresRNA. From the above-described results, it is presumed that not only the sequence of vegf IresRNA complementary to p53 but also the stem-loop structure of vegf IresRNA is important for the induction of degradation of p53 mRNA.

<Example C1>

Effect of p53 decoy LNA-DNA

A region of p53 mRNA interacting with vegf IresRNA was used as decoy DNA, and the effect of vegf IresRNA on p53 mRNA silencing and cell death of cancer cells was examined.

From the result of Example B4, it can be considered that a region of p53 mRNA interacting with vegf IresRNA is a region including 462nd to 491st bases. Thus, as a wild-type p53 decoy, LNA-DNA corresponding to a sequence from base 462 to base 482 of p53 mRNA was produced. This is referred to as a wild-type p53 decoy. In addition, LNA-DNA corresponding to the aforementioned sequence introduced with mutation was produced. This is referred to as a mutant-type p53 decoy. The sequences thereof are as follows. In the mutant-type p53 decoy, bases written in capital letters are mutant sites, which are different from the wild-type p53 decoy.
Wild-type p53 decoy (SEQ ID NO: 93)
5'-ccccgcgtggcccctgcacca-3'
Mutant-type p53 decoy (SEQ ID NO: 94)
5'-cccGCcCACCcGGctgGTcca-3'

Then, the p53 expression vectors of Example A2, the vegf IresRNA expression vectors of Example B1, and the wild-type or mutant-type p53 decoys were cotransfected into p53-deleted HCT116, and expression of p53 mRNA was analyzed by the Northern blotting. Further, with respect to p53-deleted HCT116 cells (vegf IresRNA) cotransfected with the vegf IresRNA expression vectors and the p53 expression vectors and not transfected with the decoys, p53-deleted HCT116 cells (mock) cotransfected with plasmid vector pcDNA3.1 lacking insert and the p53 expression vectors and not transfected with the decoys, and p53-deleted HCT116 cells not transfected with the expression vectors or the decoys, expression of p53 mRNA was analyzed in the same manner as described above.

The results are shown in FIG. 25A. FIG. 25A shows the results of the Northern blotting, which indicate expression of p53 mRNA in each type of the cells. In FIG. 25A, "no transfection" indicates the result obtained regarding the cells not transfected with the expression vectors, "vegf IresRNA" indicates the result obtained regarding the cells transfected with the vegf IresRNA expression vectors, "mock" indicates the result obtained regarding the cells transfected with pcDNA3.1, "vegf IresRNA + p53 decoy mutant" indicates the result obtained regarding the cells cotransfected with the mutant-type p53 decoys and the vegf IresRNA expression vectors, and "vegf IresRNA + p53 decoy" indicates the result obtained regarding the cells cotransfected with the wild-type p53 decoys and the vegf IresRNA expression vectors. Further, p53 "+" indicates that the transfection of p53 expression vectors was conducted, and "-" indicates that the transfection of the same was not conducted. As shown in FIG. 25A, with respect to "vegf IresRNA" not transfected with the decoys, the expression amount of p53 mRNA was very little and it is interpreted that "vegf IresRNA" was silenced. Further, also with respect to "vegf IresRNA + p53 decoy mutant" transfected with the mutant-type p53 decoys, the expression amount of p53RNA was small and it is interpreted that "vegf IresRNA+ p53 decoy mutant" was silenced. In contrast, with respect to "vegf IresRNA + p53 decoy" transfected with the wild-type p53 decoys, the expression amount higher than that of "vegf IresRNA" was observed, and the expression amount of p53 mRNA similar to that of mock was observed. This showed that the p53 decoy suppressed the silencing effect of vegf IresRNA to endogenous p53 mRNA and increased the expression amount of p53 mRNA.

Further, with respect to wild-type and mutant-type p53 decoys, the influence on the apoptosis sensitivity of cancer cells was examined.

The wild-type and mutant-type p53 decoy LNA-DNAwere transfected into HCT116 cells (wild-type p53). The cells transfected with the decoys were cultured in McCoy's 5A medium containing 10% fetal bovine serum and 100 μg/ml (100 units/ml) of streptomycin, and the relative value of the survival rate (%) was calculated according to "Apoptosis evaluation 2". The relative value was obtained with the number of viable cells of 5-FU untreated cells being considered as 100%. In addition, the survival rate of HCT cells (p53) not transfected with the decoys also was calculated in the same manner as described above.

The results are shown in FIG. 25B. FIG. 25B is a graph showing the survival rate (%) of each type of the cells. In FIG. 25B, "none" indicates the result obtained regarding the cells not transfected with the decoys, "p53 decoy" indicates the result obtained regarding the cells transfected with the wild-type p53 decoys, and "p53 decoy mutant" indicates the result obtained regarding the cells transfected with the mutant-type p53 decoys. As shown in FIG. 25B, with respect to the cells transfected with the wild-type p53 decoys, the survival rate of cells after treated with 5-FU (40 μmol/L) was decreased as compared to the cells not transfected with the decoys and the cells transfected with the mutant-type p53 decoys. These results showed that a p53 mRNA region interacting with vegf IresRNA was useful for preventing degradation of p53 mRNA and inducing cell death when it was used as the decoy. The decoy can be used as a p53 binding inhibitor or a p53 expression promoting agent of the present invention.

<Example C2>

Effect of vegf antisense DNA

With respect to antisense DNA complementary to a region of vegf IresRNA interacting with p53 mRNA, the effect of vegf IresRNA on p53 mRNA silencing and cell death of cancer cells was examined.

From the results of Example B4, it can be considered that a region of vegf IresRNA interacting with p53 mRNA is a region including 693rd to 714th bases. Thus, antisense LNA-DNA complementary to this region was produced. This is referred to as wild-type vegf antisense. In addition, antisense LNA-DNA having the sequence introduced with mutation was produced. This is referred to as mutant-type vegf antisense. The sequences thereof are as follows. In the mutant-type antisense, bases written in capital letters are mutant sites, which are different from the wild-type antisense. Wild-type vegf antisense (SEQ ID NO: 74)
5'-ccccgcgcggaccacggctcct-3'
Mutant-type vegf antisense (SEQ ID NO: 95)
5'-cccGCcCGggTcGTcCgcAccA-3'

The results are shown in FIG. 26A. FIG. 26A shows results of the Northern blotting, which indicate expression of p53 mRNA in each type of the cells. In FIG. 26A, "vegf IresRNA" indicates the result obtained regarding the cells transfected with the vegf IresRNA expression vectors, "vegf IresRNA + AS mutant" indicates the result obtained regarding the cells cotransfected with the mutant-type vegf antisenses and the vegf IresRNA expression vectors, and "vegf IresRNA + AS" indicates the result obtained regarding the cells cotransfected with the wild-type vegf antisenses and the vegf IresRNA expression vectors. Further, p53 "+" indicates that the transfection of p53 expression vectors was conducted. As shown in FIG. 26A, with respect to "vegf IresRNA" not transfected with the antisenses, the expression amount of p53 mRNA was very little and it is interpreted that "vegf IresRNA" was silenced. Further, also with respect to "vegf IresRNA + AS mutant" transfected with the mutant-type antisenses, the expression amount of p53RNA was small and it is interpreted that "vegf IresRNA+AS mutant" was silenced. In contrast, with respect to "vegf IresRNA + AS" cotransfected with the wild-type vegf antisenses, marked expression amount was observed as compared to "vegf IresRNA". This showed that the vegf antisense suppressed the silencing effect of vegf IresRNA to endogenous p53 mRNA and increased the expression amount of p53 mRNA.

Further, with respect to wild-type and mutant-type vegf antisense, the influence on apoptosis sensitivity of cancer cells was examined.

The wild-type and mutant-type vegf antisenses were transfected into HCT116 cells (wild-type p53). With respect to the cells transfected with the antisenses, the relative value of the survival rate (%) was calculated in the same manner as in Example C4. The relative value was obtained with the number of viable cells of 5-FU untreated cells being considered as 100%. In addition, the survival rate of HCT cells (wild-type p53) not transfected with the vegf antisenses was also calculated in the same manner as described above.

The results are shown in FIG. 26B. FIG. 26B is a graph showing the survival rate (%) of each type of the cells. In FIG. 26B, "none" indicates the result obtained regarding the cells not transfected with the vegf antisenses, "vegf 5'-AS" indicates the result obtained regarding the cells transfected with the wild-type vegf antisenses, and "vegf 5'-AS mutant" indicates the result obtained regarding the cells transfected with the mutant-type vegf antisenses. As shown in FIG. 26B, with respect to the cells transfected with the wild-type vegf antisenses, the survival rate of cells after treated with 5-FU (40 μmol/L) was markedly decreased as compared to the cells (none) not transfected with the vegf antisenses. These results showed that a region of vegf IresRNA interacting with p53 mRNA was useful for preventing degradation of p53 mRNA and inducing cell death by anticancer agents when it was used as antisense. The antisense can be used as a p53 binding inhibitor or a p53 expression promoting agent of the present invention.

Industrial Applicability

As described above, according to the present invention, by inhibiting the binding between RNA transcribed from exon 1 of the vegf-A gene and p53 mRNA, the expression of p53 can be promoted, thereby allowing the transcription of genes that are involved in apoptosis and transcribed in a p53-dependent manner to be promoted. Accordingly, it is possible to promote the progress of the apoptosis of a target cell in the end. In particular, in the medical field, cell apoptosis is utilized in prevention of malignant progression of tumors and treatments for cancers, and hence, it can be said that the present invention can serve as useful means in treatments for cancers etc. Furthermore, it has been known that, in a tumor cell in which the amount of the expressed p53 is decreased, the sensitivity to an anticancer agent is lowered, and the tumor cell shows resistance to the anticancer agent. However, according to the present invention, it is possible to enhance the sensitivity of a cell to an anticancer agent, so that it becomes possible to allow conventionally known anticancer agents to act efficiency. As specifically described above, it can be said that the present invention is very beneficial technology in the medical field including treatments for cancers etc.

## Claims

1. A method for promoting expression of a p53 gene in a cell, wherein the expression of the p53 gene is promoted by inhibiting binding between RNA transcribed from exon 1 of a vegf-A gene and mRNA transcribed from the p53 gene.

2. The p53 expression promoting method according to claim 1, which comprises a method for inhibiting the binding between the RNA transcribed from the exon 1 of the vegf-A gene and the mRNA transcribed from the p53 gene, the inhibition method comprising at least one step selected from the group consisting of the following steps (a1) to (a3):

   (a1) the step of degrading the RNA transcribed from the exon 1 of the vegf-A gene;
   (a2) the step of inhibiting transcription of RNA from the exon 1 of the vegf-A gene; and
   (a3) the step of causing a nucleic acid different from the mRNA transcribed from the p53 gene to bind to the RNA transcribed from the exon 1 of the vegf-A gene, thereby inhibiting the binding between the RNA and the mRNA transcribed from the p53 gene.

3. The p53 expression promoting method according to claim 2, wherein a target RNA to be degraded in the step (a1) and a target RNA to be subjected to the inhibition of the transcription in the step (a2) each have at least a whole or a part of a sequence from base 600 to base 879 in SEQ ID NO. 1.

4. The promoting method according to claim 3, wherein the target RNAs each are at least one RNA selected from the group consisting of the following (1) to (16):

   (1) RNA having a sequence from base 600 to base 879 in SEQ ID NO. 1
   (2) RNA having a sequence from base 659 to base 879 in SEQ ID NO. 1
   (3) RNA having a sequence from base 659 to base 780 in SEQ ID NO. 1
   (4) RNA having a sequence from base 659 to base 752 in SEQ ID NO. 1
   (5) RNA having a sequence from base 659 to base 745 in SEQ ID NO. 1
   (6) RNA having a sequence from base 694 to base 714 in SEQ ID NO. 1
   (7) RNA having a sequence from base 693 to base 714 in SEQ ID NO. 1
   (8) RNA having a sequence from base 691 to base 703 in SEQ ID NO. 1
   (9) RNA having a sequence from base 711 to base 723 in SEQ ID NO. 1
   (10) RNA having a sequence from base 16 to base 780 in SEQ ID NO. 1
   (11) RNA having a sequence from base 659 to base 917 in SEQ ID NO. 1
   (12) RNA having a sequence from base 526 to base 1104 in SEQ ID NO. 1

(13) RNA having a sequence from base 526 to base 1216 in SEQ ID NO. 45
(14) 5' UTR RNA in vegf mRNA
(15) exon 1 RNA in vegf mRNA
(16) vegf mRNA

**5.** The p53 expression promoting method according to claim 3, wherein the method comprises at least one of the step (a1) and the step (a2),
in the step (a1), the target RNA is degraded by at least one binding inhibitor selected from the group consisting of siRNA, a siRNA precursor that releases siRNA when degraded by ribonuclease, an antisense, and ribozyme, and in the step (a2), the transcription of the target RNA is inhibited by at least one binding inhibitor selected from the group consisting of siRNA, a siRNA precursor that releases siRNA when degraded by ribonuclease, an antisense, ribozyme, and a decoy nucleic acid.

**6.** The p53 expression promoting method according to claim 5, wherein, in the step (a1), the binding inhibitor is siRNA, and an antisense strand of the siRNA is at least one selected from the group consisting of the following (#1) to (#17):

#1 5' -agcaaggcaaggcuccaaugcnn-3' (SEQ ID NO. 4)
#2 5' -agagcagcaaggcaaggcuccnn-3' (SEQ ID NO. 6)
#3 5' -cacgaccgcuuaccuuggcaunn-3' (SEQ ID NO. 8)
#4 5' -uagcacuucucgcggcuccgcnn-3' (SEQ ID NO. 10)
#5 5' -cgagcuagcacuucucgcggcnn-3' (SEQ ID NO. 12)
#6 5' -uggacgaaaaguuucagugcgacgcnn-3' (SEQ ID NO. 14)
#7 5' -agaaguuggacgaaaaguuucagugnn-3' (SEQ ID NO. 16)
#8 5' -gaaguuggacgaaaaguuucagugcnn-3' (SEQ ID NO. 18)
#9 5' -ggacgaaaaguuucagugcgacgccnn-3' (SEQ ID NO. 20)
#10 5' -uuggacgaaaaguuucagugcgacgnn-3' (SEQ ID NO. 22)
#11 5' -agaaguuggacgaaaaguuucagugnn-3' (SEQ ID NO. 61)
#12 5' -aguuucagugcgacgccgcgagcccnn-3' (SEQ ID NO. 63)
#13 5' -gaagcgagaacagcccagaaguuggnn-3' (SEQ ID NO. 65)
#14 5' -agcaaggcaaggcuccaaugcacccnn-3' (SEQ ID NO. 67)
#15 5' -agagcagcaaggcaaggcuccaaugnn-3' (SEQ ID NO. 69)
#16 5' -cgagcuagcacuucucgcggcuccgnn-3' (SEQ ID NO. 71)
#17 5' -cgcggaccacggcuccuccgaagcgnn-3' (SEQ ID NO. 73).

**7.** The p53 expression promoting method according to claim 2, wherein, in the step (a3), the different nucleic acid is at least one nucleic acid selected from the group consisting of the following (D1) to (D8):

(D1) RNA having a sequence from base 462 to base 481 in SEQ ID NO. 2, or DNA having a sequence corresponding to the sequence of this RNA in which base u is substituted with base t;
(D2) RNA having a sequence from base 462 to base 482 in SEQ ID NO. 2, or DNA having a sequence corresponding to the sequence of this RNA in which the base u is substituted with base t
(D3) RNA having a sequence from base 462 to base 491 in SEQ ID NO. 2, or DNA having a sequence corresponding to the sequence of this RNA in which the base u is substituted with base t
(D4) RNA having a sequence from base 462 to base 505 in SEQ ID NO. 2, or DNA having a sequence corresponding to the sequence of this RNA in which the base u is substituted with base t
(D5) RNA having a sequence from base 252 to base 551 in SEQ ID NO. 2, or DNA having a sequence corresponding to the sequence of this RNA in which the base u is substituted with base t
(D6) RNA having a sequence complementary to a sequence from base 694 to base 714 in SEQ ID NO. 1, or DNA having a sequence corresponding to the sequence of this RNA in which the base u is substituted with base t
(D7) RNA having a sequence complementary to a sequence from base 693 to base 714 in SEQ ID NO. 1, or DNA having a sequence corresponding to the sequence of this RNA in which the base u is substituted with base t
(D8) a nucleic acid that has a base sequence corresponding to the sequence of at lease one nucleic acid selected from the group consisting of (D1) to (D7), in which one or more bases are substituted, deleted, inserted, or added, and can bind to the RNA transcribed from the exon 1 of the vegf-A gene.

**8.** The p53 expression promoting method according to claim 1, wherein the cell is a tumor cell.

**9.** A p53 expression promoting agent for use in the p53 expression promoting method according to claim 1, comprising:

at least one binding inhibitor that inhibits binding between RNA transcribed from exon 1 of a vegf-A gene and mRNA transcribed from a p53 gene,
the binding inhibitor being selected from the group consisting of the following (A1) to (A4):

(A1) at least one binding inhibitor selected from the group consisting of siRNA, a siRNA precursor that releases siRNA when degraded by ribonuclease, an antisense, and ribozyme, each of which degrades the RNA transcribed from the exon 1 of the vegf-A gene;
(A2) at least one binding inhibitor selected from the group consisting of siRNA, a siRNA precursor that releases siRNA when degraded by ribonuclease, an antisense, ribozyme, and a decoy nucleic acid, each of which inhibits transcription of RNA from the exon 1 of the vegf-A gene;
(A3) a binding inhibitor that contains a nucleic acid different from the mRNA transcribed from the p53 gene and binds to the RNA transcribed from the exon 1 of the vegf-A gene; and
(A4) a binding inhibitor that contains an expression vector in which DNA coding the at least one binding inhibitor selected from the group consisting of (A1) to (A3) is inserted.

**10.** The p53 expression promoting agent according to claim 8, wherein a target RNA to be degraded by the binding inhibitor of (A1) and a target RNA to be subjected to the inhibition of the transcription by the binding inhibitor of (A2) each have at least a whole or a part of a sequence from base 600 to base 879 in SEQ ID NO. 1.

**11.** The p53 expression promoting agent according to claim 9, wherein the target RNAs each are at least one RNA selected from the group consisting of the following (1) to (16):

(1) RNA having a sequence from base 600 to base 879 in SEQ ID NO. 1
(2) RNA having a sequence from base 659 to base 879 in SEQ ID NO. 1
(3) RNA having a sequence from base 659 to base 780 in SEQ ID NO. 1
(4) RNA having a sequence from base 659 to base 752 in SEQ ID NO. 1
(5) RNA having a sequence from base 659 to base 745 in SEQ ID NO. 1
(6) RNA having a sequence from base 694 to base 714 in SEQ ID NO. 1
(7) RNA having a sequence from base 693 to base 714 in SEQ ID NO. 1
(8) RNA having a sequence from base 691 to base 703 in SEQ ID NO. 1
(9) RNA having.a sequence from base 711 to base 723 in SEQ ID NO. 1
(10) RNA having a sequence from base 16 to base 780 in SEQ ID NO. 1
(11) RNA having a sequence from base 659 to base 917 in SEQ ID NO. 1
(12) RNA having a sequence from base 526 to base 1104 in SEQ ID NO. 1
(13) RNA having a sequence from base 526 to base 1216 in SEQ ID NO. 45
(14) 5' UTR RNA in vegf mRNA
(15) exon 1 RNA in vegf mRNA
(16) vegf mRNA

**12.** The p53 expression promoting agent according to claim 9, wherein the binding inhibitor of (A1) is siRNA, and an antisense strand of the siRNA is at least one selected from the group consisting of the following (#1) to (#17):

#1 5' -agcaaggcaaggcuccaaugcnn-3' (SEQ ID NO. 4)
#2 5' -agagcagcaaggcaaggcucccnn-3' (SEQ ID NO. 6)
#3 5' -cacgaccgcuuaccuuggcaunn-3' (SEQ ID NO. 8)
#4 5' -uagcacuucucgcggcuccgcnn-3' (SEQ ID NO. 10)
#5 5' -cgagcuagcacuucucgcggcnn-3' (SEQ ID NO. 12)
#6 5' -uggacgaaaaguuucagugcgacgcnn-3' (SEQ ID NO. 14)
#7 5' -agaaguuggacgaaaaguuucagugnn-3' (SEQ ID NO. 16)
#8 5' -gaaguuggacgaaaaguuucagugcnn-3' (SEQ ID NO. 18)
#9 5' -ggacgaaaaguuucagugcgacgccnn-3' (SEQ ID NO. 20)
#10 5' -uuggacgaaaaguuucagugcgacgnn-3' (SEQ ID NO. 22)
#11 5' -agaaguuggacgaaaaguuucagugnn-3' (SEQ ID NO. 61)
#12 5' -aguuucagugcgacgccgcgagcccnn-3' (SEQ ID NO. 63)
#13 5' -gaagcgagaacagcccagaaguuggnn-3' (SEQ ID NO. 65)
#14 5' -agcaaggcaaggcuccaaugcacccnn-3' (SEQ ID NO. 67)
#15 5' -agagcagcaaggcaaggcuccaaugnn-3' (SEQ ID NO. 69)
#16 5' -cgagcuagcacuucucgcggcuccgnn-3' (SEQ ID NO. 71)

#17 5' -cgcggaccacggcuccuccgaagcgnn-3' (SEQ ID NO. 73).

**13.** The p53 expression promoting agent according to claim 9, wherein the different nucleic acid in the binding inhibitor of (A3) is at least one nucleic acid selected from the group consisting of the following (D1) to (D8):

(D1) RNA having a sequence from base 462 to base 481 in SEQ ID NO. 2, or DNA having a sequence corresponding to the sequence of this RNA in which base u is substituted with base t
(D2) RNA having a sequence from base 462 to base 482 in SEQ ID NO. 2, or DNA having a sequence corresponding to the sequence of this RNA in which the base u is substituted with base t
(D3) RNA having a sequence from base 462 to base 491 in SEQ ID NO. 2, or DNA having a sequence corresponding to the sequence of this RNA in which the base u is substituted with base t
(D4) RNA having a sequence from base 462 to base 505 in SEQ ID NO. 2, or DNA having a sequence corresponding to the sequence of this RNA in which the base u is substituted with base t
(D5) RNA having a sequence from base 252 to base 551 in SEQ ID NO. 2, or DNA having a sequence corresponding to the sequence of this RNA in which the base u is substituted with base t
(D6) RNA having a sequence complementary to a sequence from base 694 to base 714 in SEQ ID NO. 1, or DNA having a sequence corresponding to the sequence of this RNA in which the base u is substituted with base t
(D7) RNA having a sequence complementary to a sequence from base 693 to base 714 in SEQ ID NO. 1, or DNA having a sequence corresponding to the sequence of this RNA in which the base u is substituted with base t
(D8) a nucleic acid that has a base sequence corresponding to the sequence of at lease one nucleic acid selected from the group consisting of (D1) to (D7), in which one or more bases are substituted, deleted, inserted, or added, and can bind to the RNA transcribed from the exon 1 of the vegf-A gene.

**14.** The p53 expression promoting agent according to claim 9, wherein the different nucleic acid in the binding inhibitor of (A3) is either one of DNA having a sequence of SEQ ID NO. 74 and DNA having a sequence of SEQ ID NO. 93.

**15.** A method for promoting apoptosis of a cell, wherein expression of p53 in the cell is promoted by the p53 expression promoting method according to claim 1.

**16.** An apoptosis promoting agent for promoting apoptosis of a cell, comprising the p53 expression promoting agent according to claim 9.

**17.** The apoptosis promoting agent according to claim 16, further comprising an anticancer agent.

**18.** A method for enhancing sensitivity of a cell to an anticancer agent, wherein expression of p53 in the cell is promoted by the p53 expression promoting method according to claim 1.

**19.** An enhancer for enhancing sensitivity of a cell to an anticancer agent, the enhancer comprising the p53 expression promoting agent according to claim 9.

**20.** A method for preventing or treating cancer, comprising the step of
administering the p53 expression promoting agent according to claim 9 to a cancer cell that is *in vivo.*

**21.** An anticancer agent comprising the p53 expression promoting agent according to claim 9.

**22.** A p53 mRNA binding agent that can bind to p53 mRNA, comprising:

a nucleic acid,

wherein the nucleic acid comprises RNA having a sequence from base 694 to base 714 in vegf-A mRNA of SEQ ID NO. 1.

**23.** A vegf-A mRNA binding agent that can bind to vegf-A mRNA, comprising:

a nucleic acid,
wherein the nucleic acid comprises RNA having a sequence from base 462 to base 481 in p53 mRNA of SEQ ID NO. 2.

**24.** A method for screening an expression promoting agent for promoting expression of p53, the method comprising the following steps (A) to (C):

(A) bringing the p53 mRNA binding agent according to claim 22 and the vegf-A RNA binding agent according to claim 23 into contact with each other in the presence of a candidate substance;
(B) detecting whether binding between the p53 mRNA binding agent and the vegf-A RNA binding agent is inhibited by the candidate substance; and
(C) selecting the candidate substance that inhibited the binding between the p53 mRNA binding agent and the vegf-A RNA binding agent as a p53 expression promoting agent.

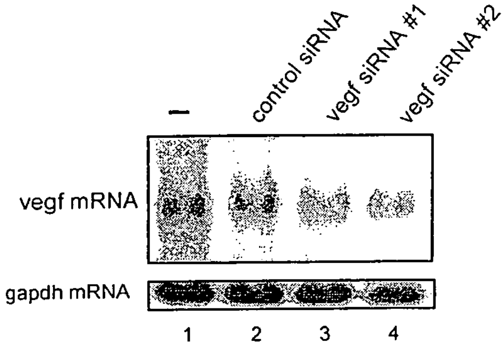


FIG. 1A

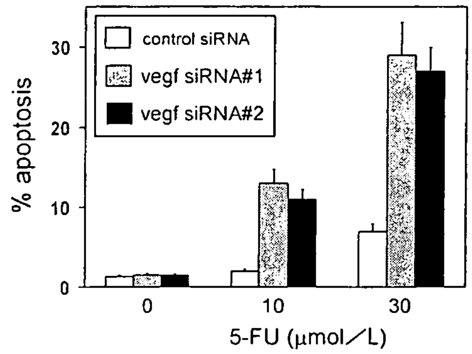


FIG. 1B

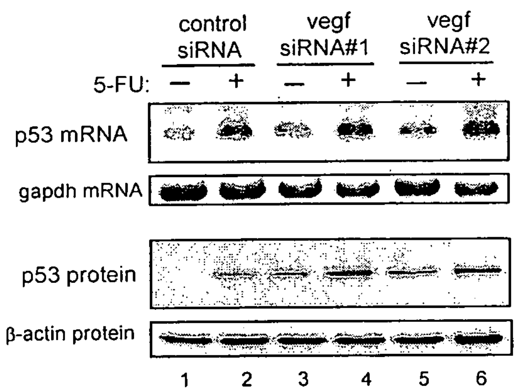


FIG. 1C

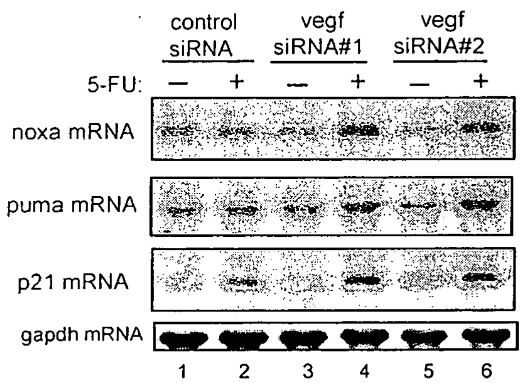


FIG. 1D

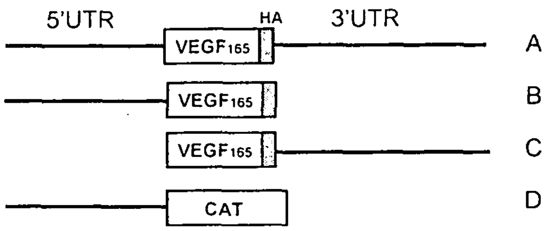


FIG. 2A

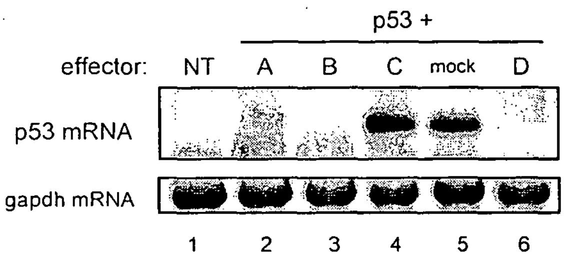


FIG. 2B

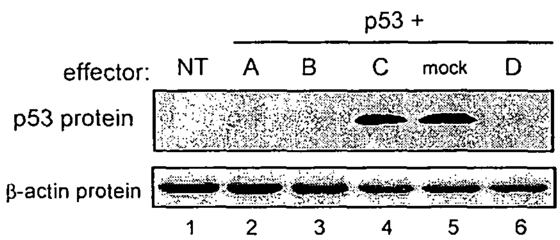


FIG. 2C

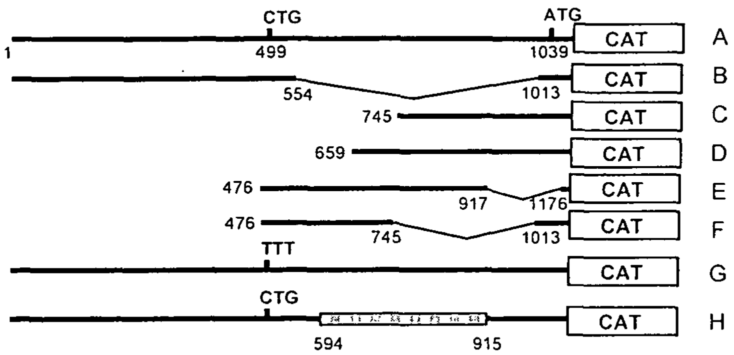


FIG. 3A

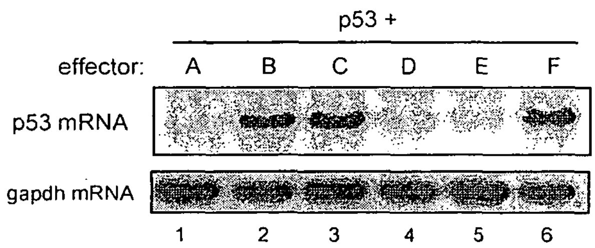


FIG. 3B

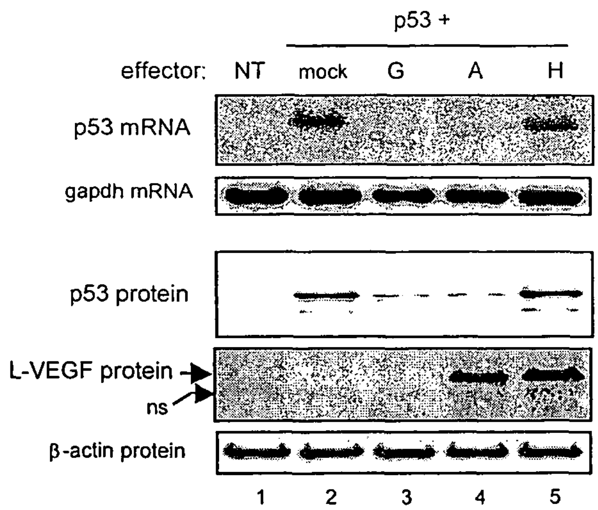


FIG. 3C

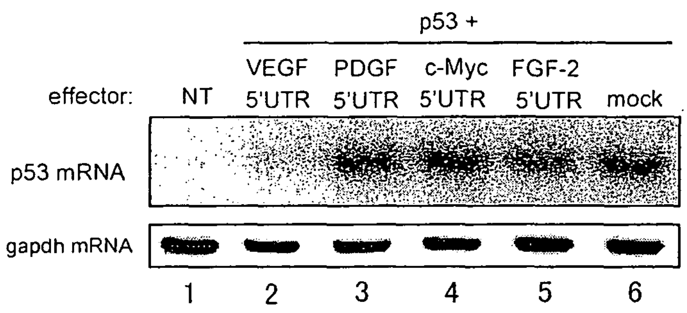
p53 +
effector:
NT
VEGF 5'UTR
PDGF 5'UTR
c-Myc 5'UTR
FGF-2 5'UTR
mock
p53 mRNA
gapdh mRNA
1
2
3
4
5
6

FIG. 4

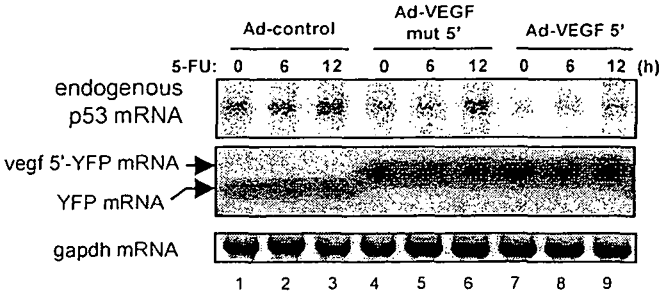


FIG. 5A

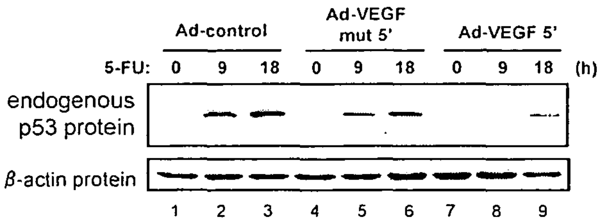


FIG. 5B

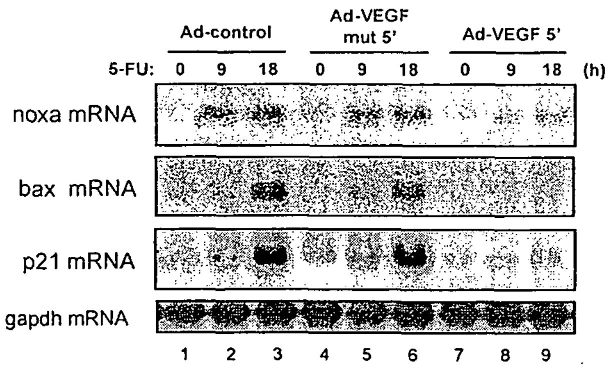


FIG. 5C

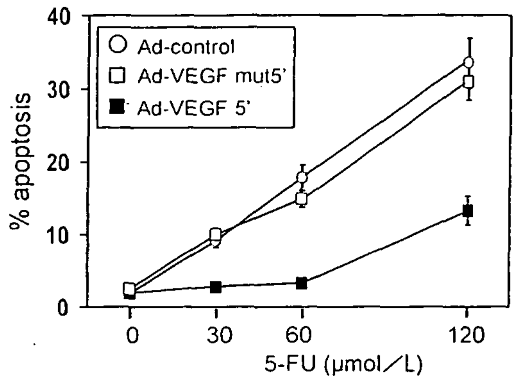
40
30
20
10
0
% apoptosis
Ad-control
Ad-VEGF mut5'
Ad-VEGF 5'
0
30
60
120
5-FU (μmol/L)

FIG. 6D

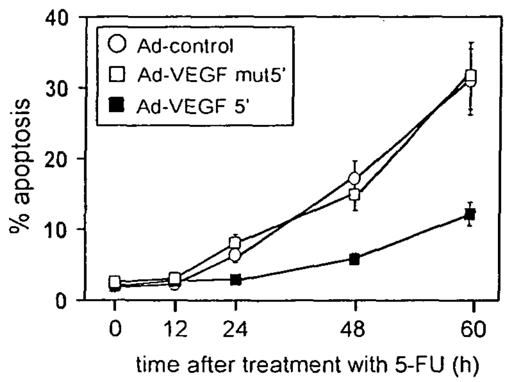
40
30
20
10
0
% apoptosis
Ad-control
Ad-VEGF mut5'
Ad-VEGF 5'
0
12
24
48
60
time after treatment with 5-FU (h)

FIG. 6E

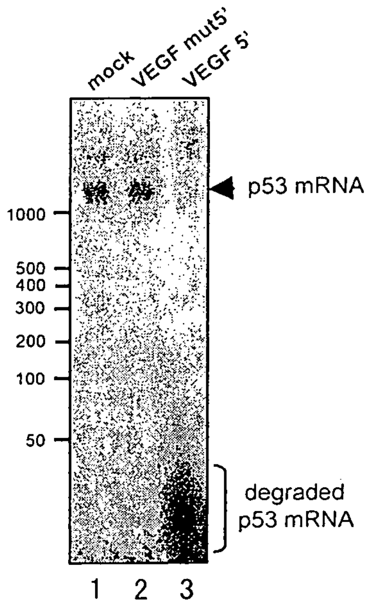


FIG. 7A

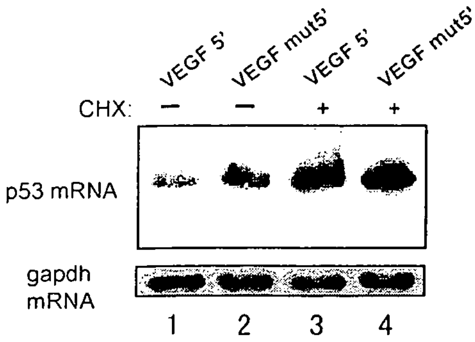


FIG. 7B

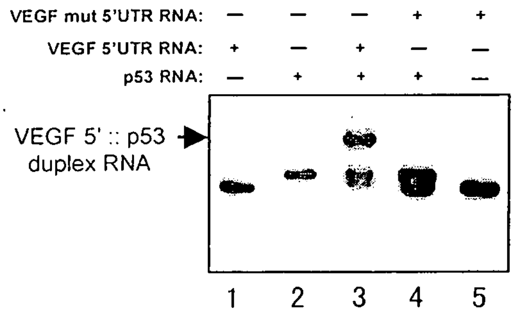


FIG. 7C

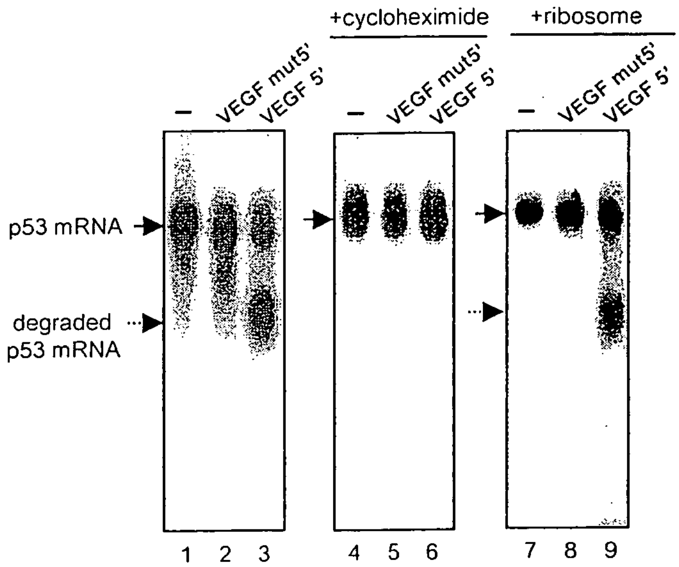
+cycloheximide
+ribosome
–
VEGF mut5'
VEGF 5'
p53 mRNA
degraded
p53 mRNA
1 2 3
4 5 6
7 8 9

FIG. 8D

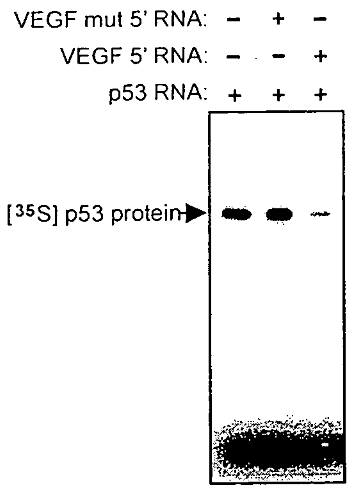
VEGF mut 5' RNA: – + –
VEGF 5' RNA: – – +
p53 RNA: + + +
[35S] p53 protein

FIG. 8E

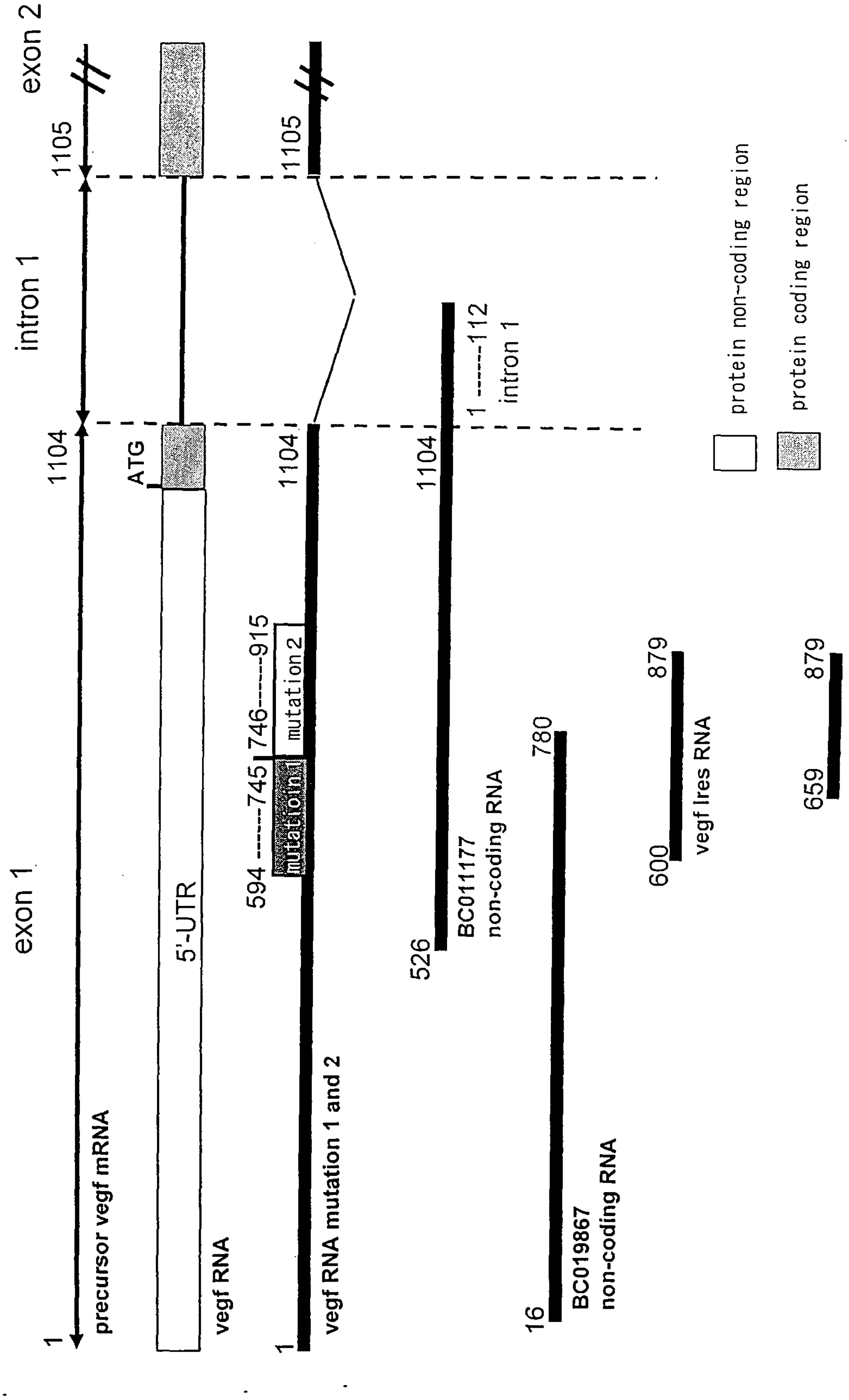

exon 1
intron 1
exon 2
1
1104
1105
precursor vegf mRNA
5'-UTR
ATG
vegf RNA
594 ------745
746------915
mutation1
mutation2
1104
1105
vegf RNA mutation 1 and 2
526
1104
BC011177
non-coding RNA
1 ------112
intron 1
16
780
BC019867
non-coding RNA
600
879
vegf Ires RNA
659
879
protein non-coding region
protein coding region


FIG. 9

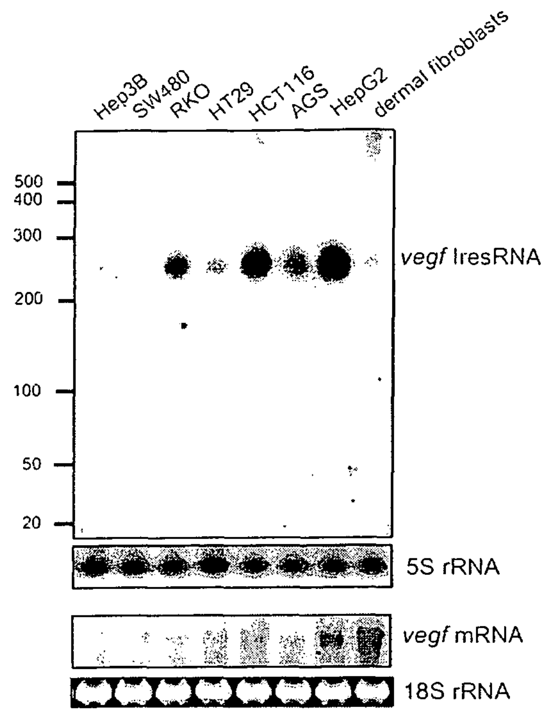
Hep3B
SW480
RKO
HT29
HCT116
AGS
HepG2
dermal fibroblasts
500
400
300
200
100
50
20
vegf IresRNA
5S rRNA
vegf mRNA
18S rRNA

FIG. 10

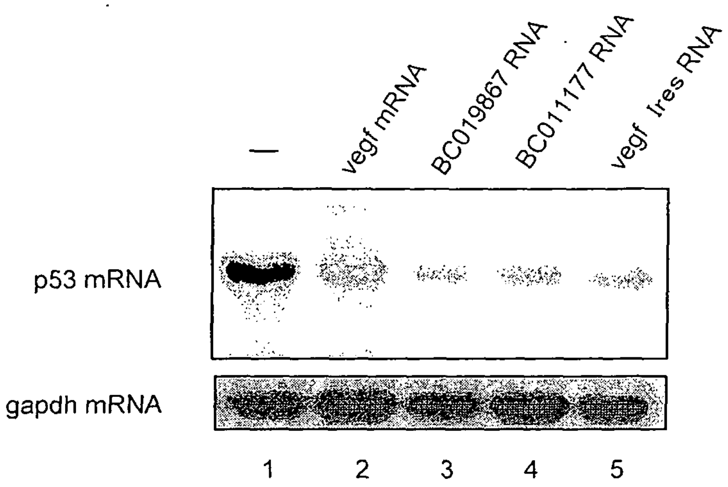


FIG. 11A

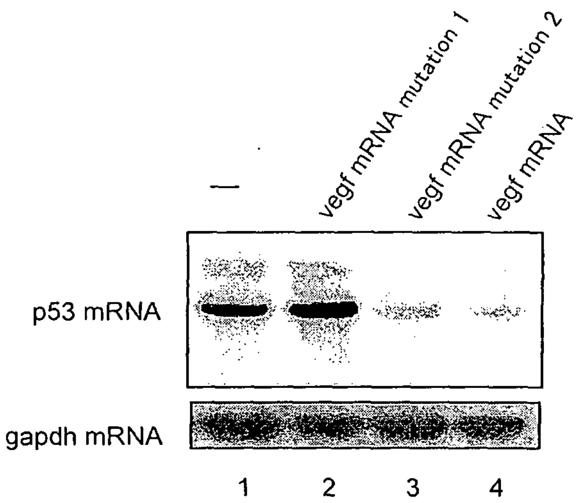


FIG. 11B

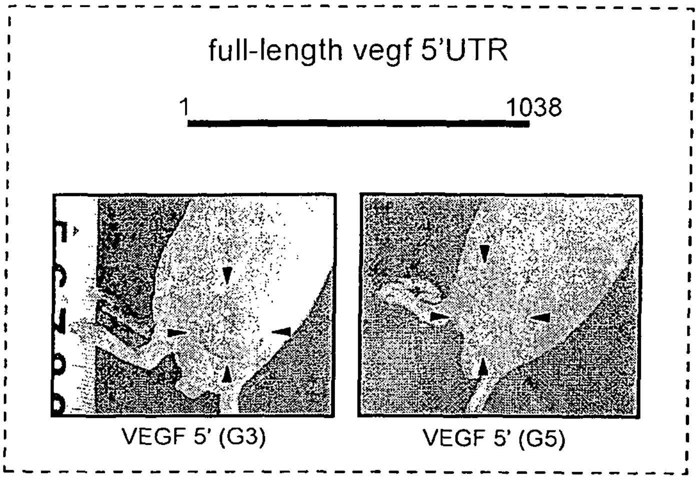


FIG. 12A

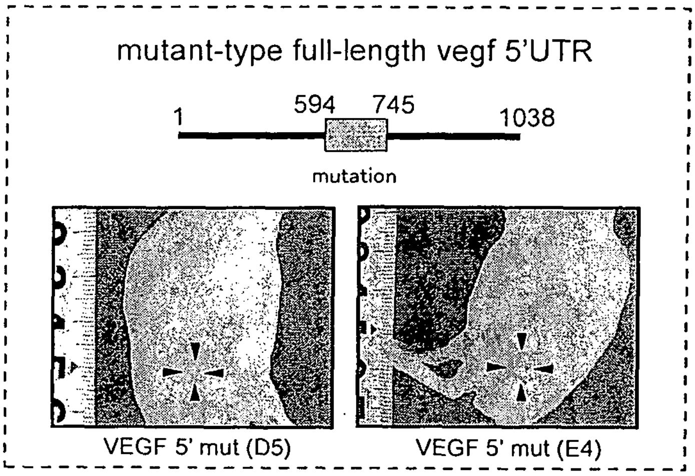


FIG. 12B

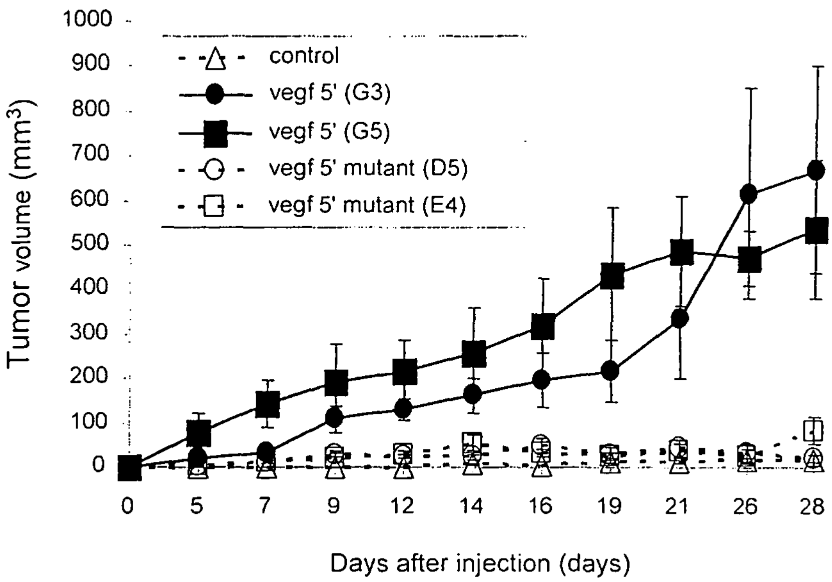
1000
900
800
700
600
500
400
300
200
100
0
Tumor volume (mm3)
- -Δ- - control
vegf 5' (G3)
vegf 5' (G5)
- -O- - vegf 5' mutant (D5)
- -□- - vegf 5' mutant (E4)
0
5
7
9
12
14
16
19
21
26
28
Days after injection (days)

FIG. 13

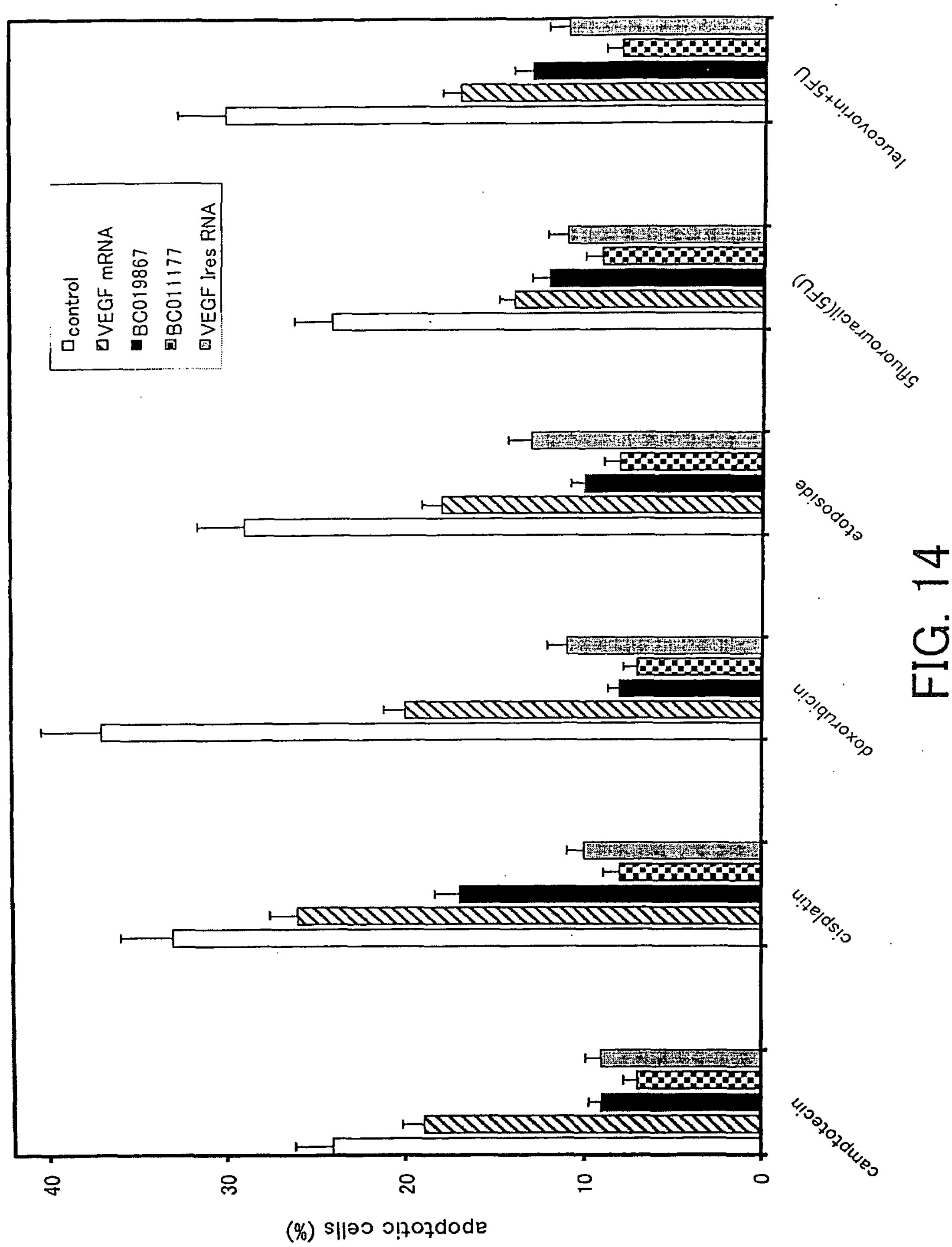
control
VEGF mRNA
BC019867
BC011177
VEGF Ires RNA
apoptotic cells (%)
0
10
20
30
40
camptotecin
cisplatin
doxorubicin
etoposide
5fluorouracil(5FU)
leucovorin+5FU

FIG. 14

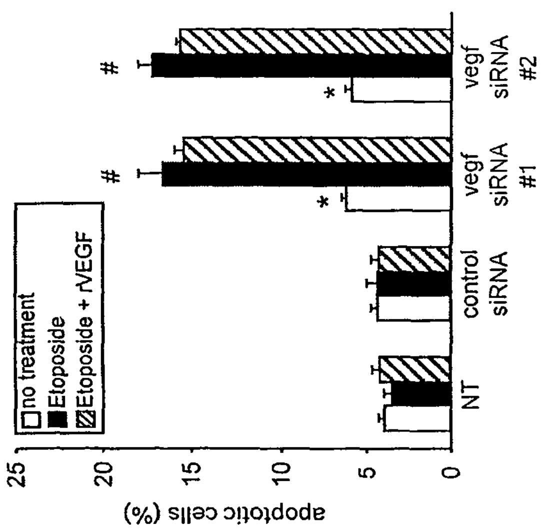


FIG. 15A

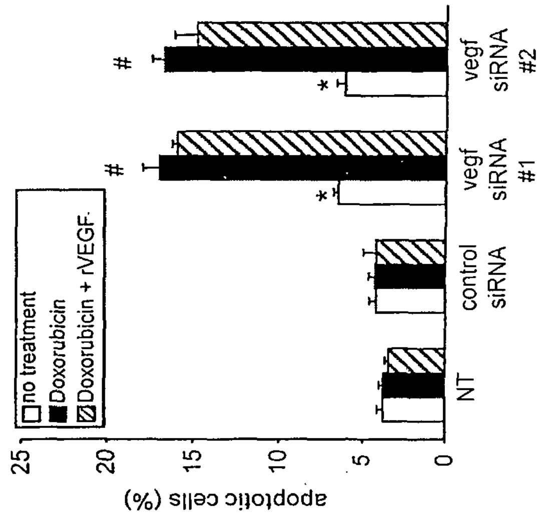


FIG. 15B

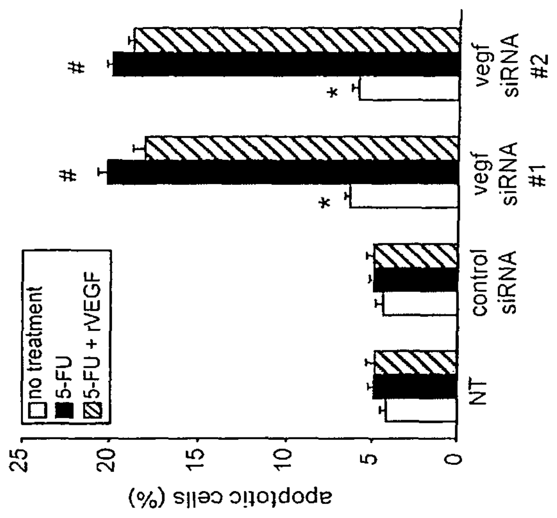


FIG. 15C

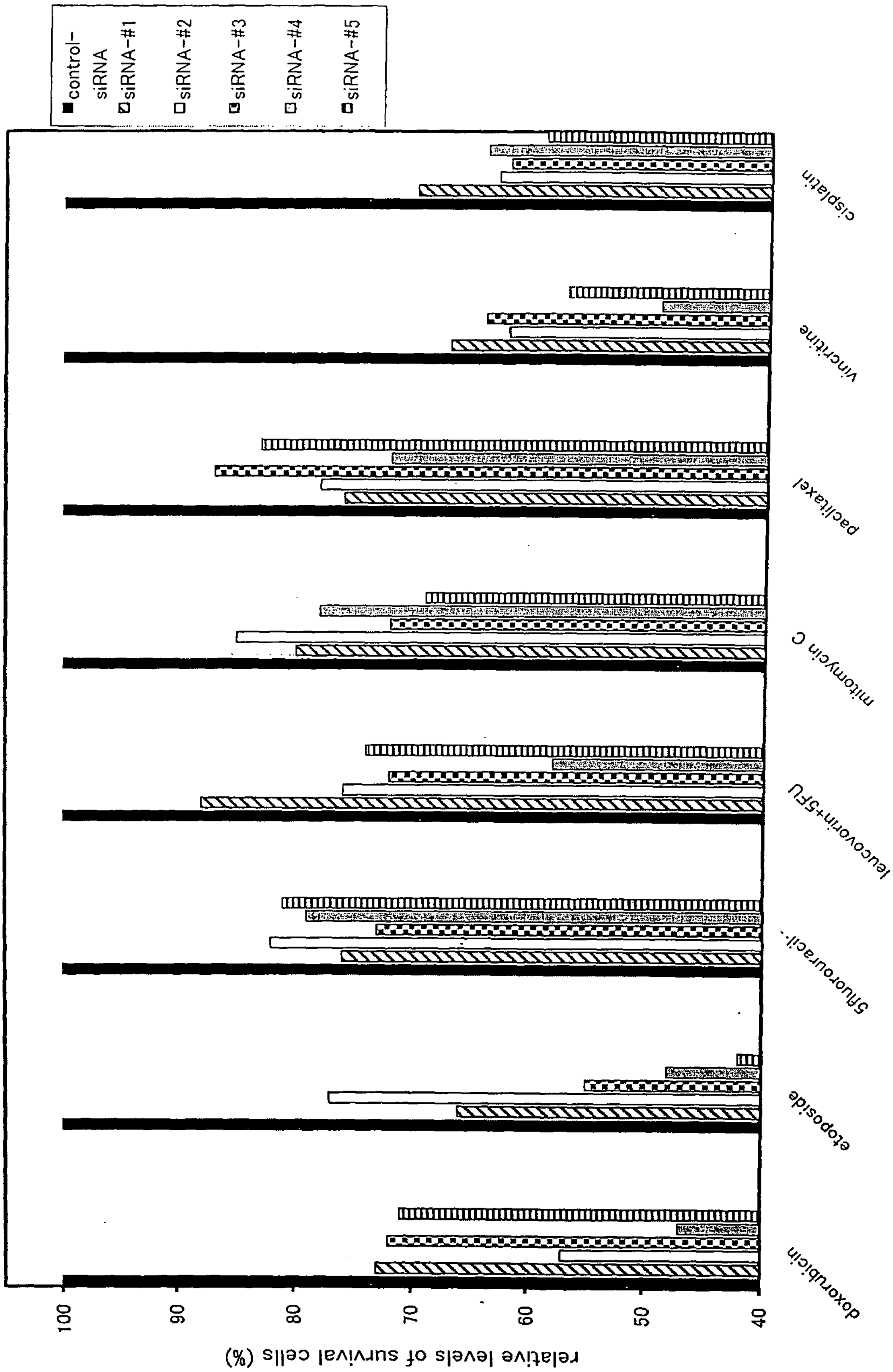

control-siRNA
siRNA-#1
siRNA-#2
siRNA-#3
siRNA-#4
siRNA-#5
relative levels of survival cells (%)
100
90
80
70
60
50
40
doxorubicin
etoposide
5fluorouracil
leucovorin+5FU
mitomycin C
paclitaxel
vincritine
cisplatin


FIG. 16

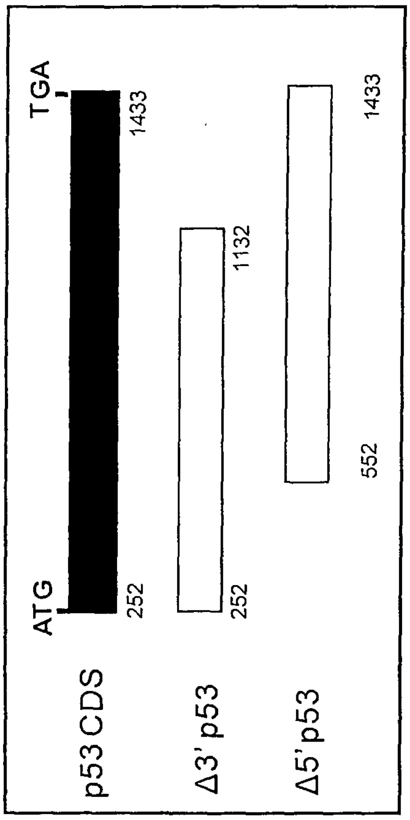


FIG. 17A

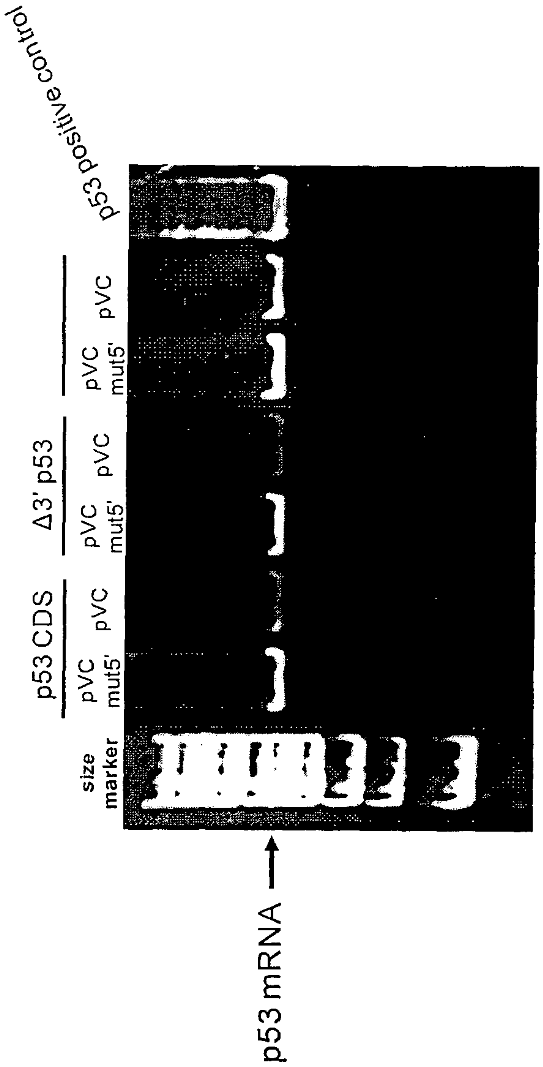


FIG. 17B

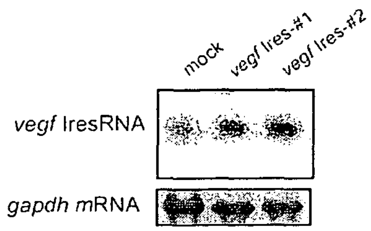


FIG. 18A

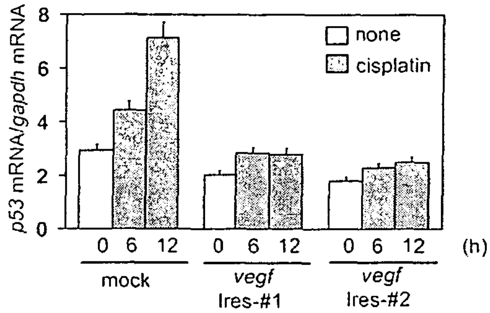


FIG. 18B

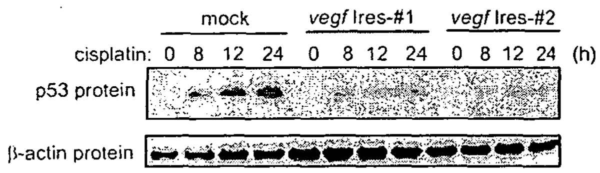


FIG. 18C

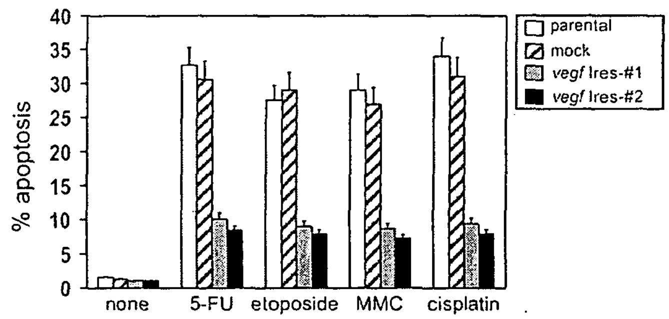


FIG. 18D

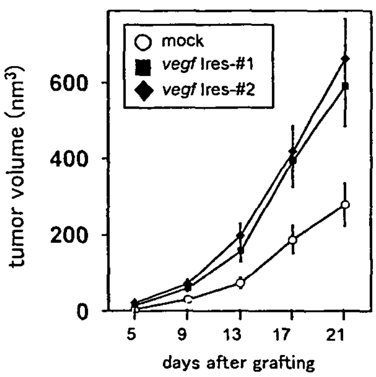


FIG. 18E

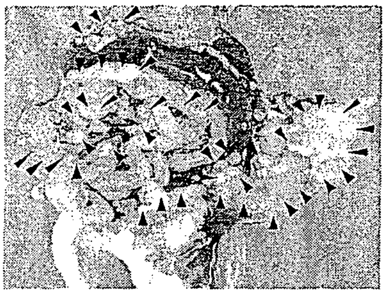

grafting of vegf IresRNA-expressing cells

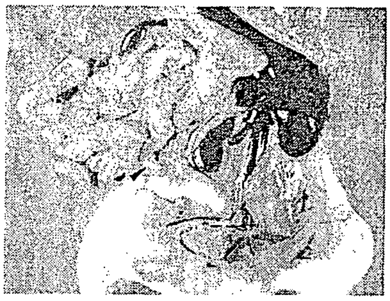

grafting of control cells

FIG. 19F

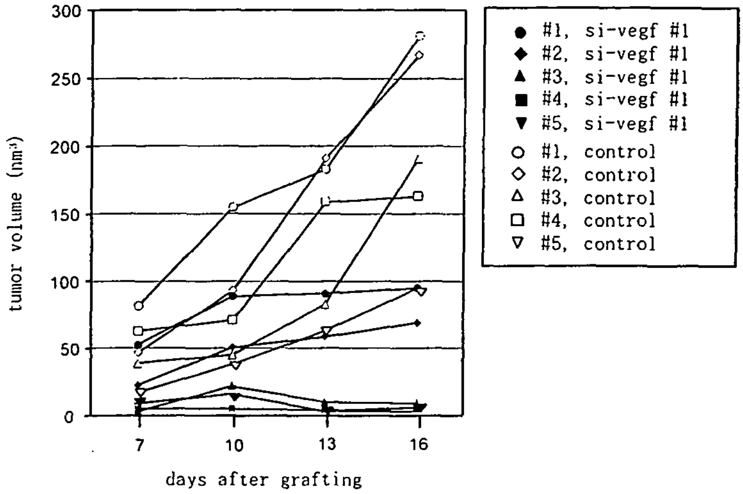
300
250
200
150
100
50
0
tumor volume (nm³)
7
10
13
16
days after grafting
#1, si-vegf #1
#2, si-vegf #1
#3, si-vegf #1
#4, si-vegf #1
#5, si-vegf #1
#1, control
#2, control
#3, control
#4, control
#5, control

FIG. 20

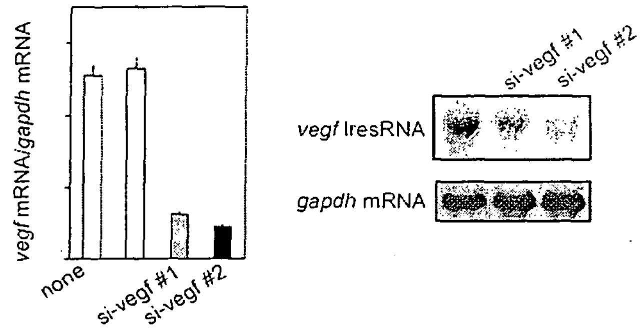


FIG. 21A

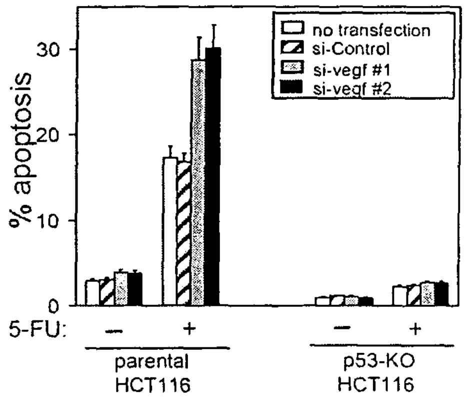


FIG. 21B

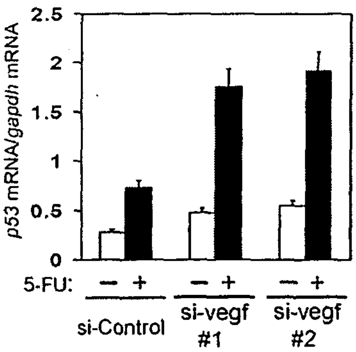


FIG. 21C

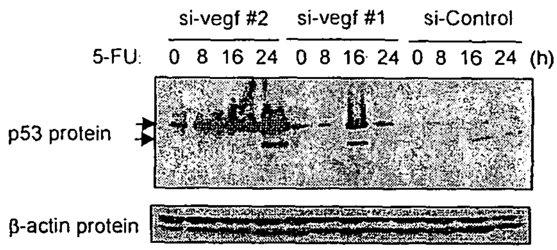


FIG. 21D

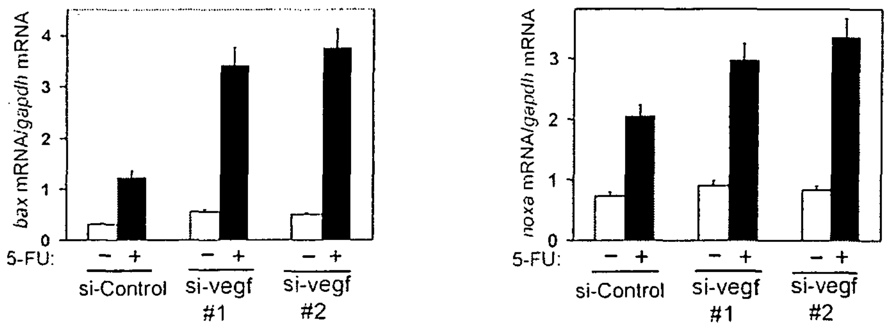


FIG. 22E

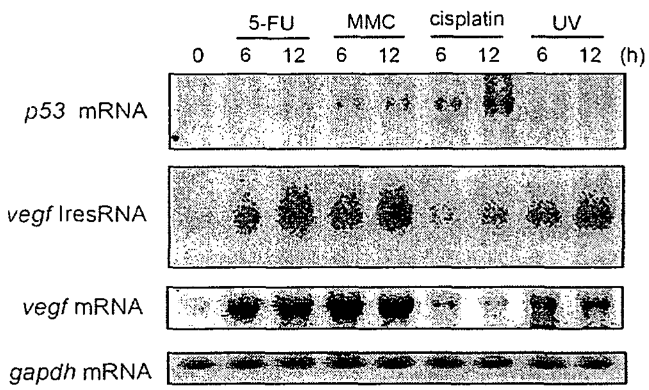


FIG. 22F

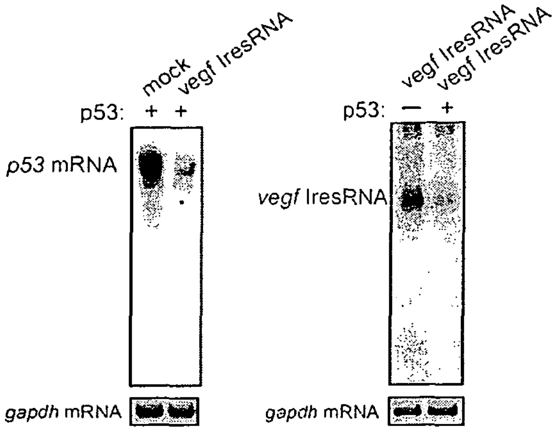


FIG. 23A

```
                   694                      714
vegf IresRNA 5'- GGAGCCGUGGUCCGCGCGGGG -3'
                 || || | || |||||||||| 
p53 mRNA     3'- CCACGUC-CCCGGUGCGCCCC -5'
                 481                      462
```

FIG. 23B

```
                   693                                714
vegf IresRNA 5'- AGGAGCCG-UGGU-------CCGCGCGGGG -3'
                 |||||| | ||||        ||||||||||
p53 mRNA     3'- UCCUCGACGACCACGUCCCCGGUGCGCCCC -5'
                 491                                462
```

FIG. 23C

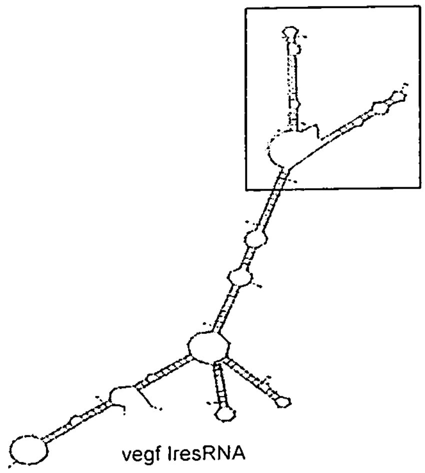


FIG. 24D

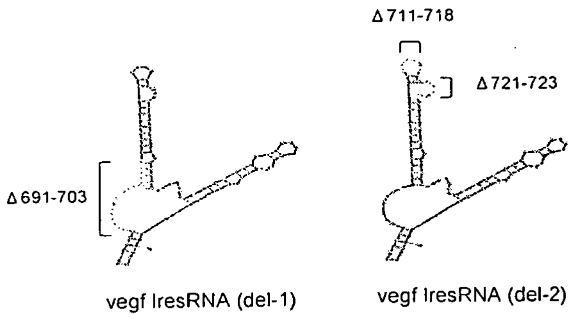


FIG. 24E

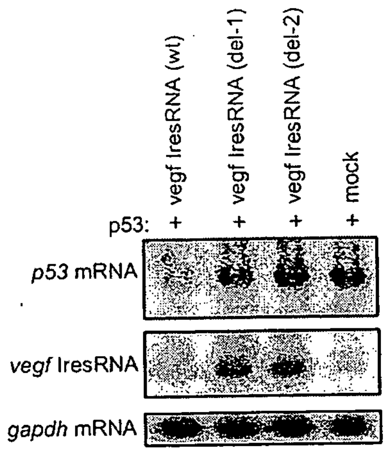


FIG. 24F

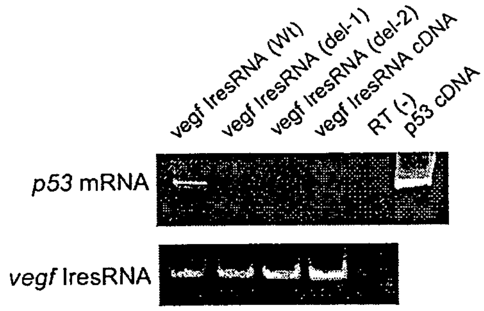


FIG. 24G

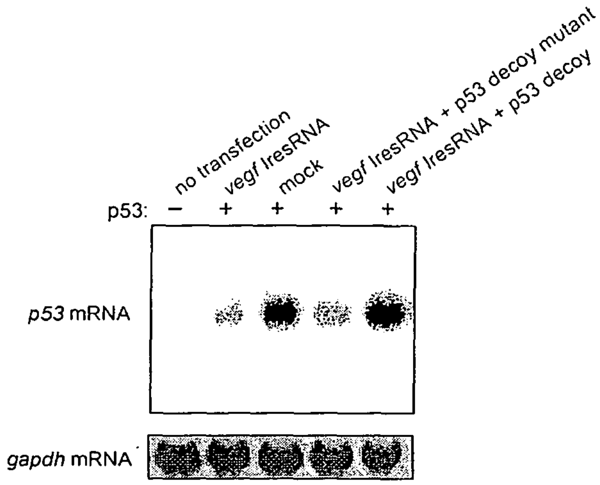


FIG. 25A

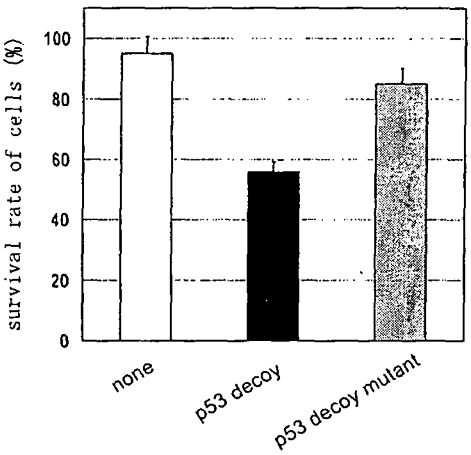


FIG. 25B

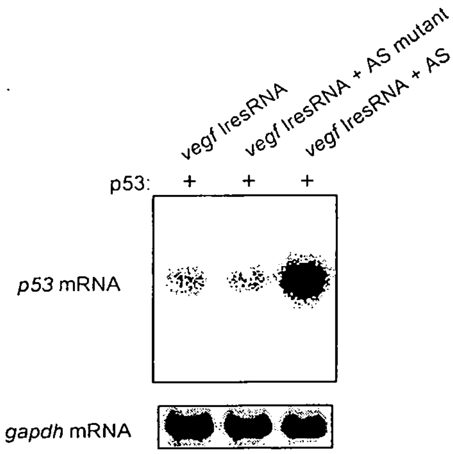


FIG. 26A

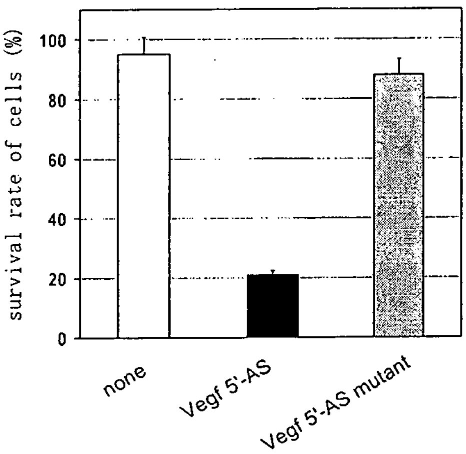


FIG. 26B

**INTERNATIONAL SEARCH REPORT**

International application No.
PCT/JP2008/066127

A. CLASSIFICATION OF SUBJECT MATTER
*A61K48/00*(2006.01)i, *A61K31/337*(2006.01)i, *A61K31/7105*(2006.01)i, *A61K31/711*(2006.01)i, *A61P35/00*(2006.01)i, *A61P43/00*(2006.01)i

According to International Patent Classification (IPC) or to both national classification and IPC

B. FIELDS SEARCHED

Minimum documentation searched (classification system followed by classification symbols)
A61K48/00, A61K31/337, A61K31/7105, A61K31/711, A61P35/00, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2008 |
| Kokai Jitsuyo Shinan Koho | 1971-2008 | Toroku Jitsuyo Shinan Koho | 1994-2008 |

Electronic data base consulted during the international search (name of data base and, where practicable, search terms used)
BIOSIS(STN), CAplus(STN), MEDLINE(STN), GenBank/EMBL/DDBJ/GeneSeq, JSTPlus(JDreamII), JMEDPlus(JDreamII), JST7580(JDreamII)

C. DOCUMENTS CONSIDERED TO BE RELEVANT

| Category* | Citation of document, with indication, where appropriate, of the relevant passages | Relevant to claim No. |
|---|---|---|
| X | MORINO, F. et al, Hammerhead ribozyme specifically inhibits vascular endothelial growth factor gene expression in a human hepatocellular carcinoma cell line, International Journal of Oncology, 2000, Vol.17, No.3, p.495-499 | 21 |
| A | IM, S.A. et al, Antiangiogenesis treatment for gliomas: transfer of antisense-vascular endothelial growth factor inhibits tumor growth in vivo, Cancer Res, 1999, Vol.59, No.4, p.895-900 | 9-17,19, 21-24 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 11 November, 2008 (11.11.08) | 25 November, 2008 (25.11.08) |
| Name and mailing address of the ISA/ Japanese Patent Office | Authorized officer |
| Facsimile No. | Telephone No. |

Form PCT/ISA/210 (second sheet) (April 2007)

**INTERNATIONAL SEARCH REPORT**

International application No.
PCT/JP2008/066127

C (Continuation). DOCUMENTS CONSIDERED TO BE RELEVANT

| Category* | Citation of document, with indication, where appropriate, of the relevant passages | Relevant to claim No. |
|---|---|---|
| A | YAMANAKA, K. et al, Synergistic antitumor effect of combined use of adenoviral-mediated p53 gene transfer and antisense oligodeoxynucleotide targeting clusterin gene in an androgen-independent human prostate cancer model, Mol Cancer Ther, 2005, Vol.4, No.2, p.187-95 | 9-17,19, 21-24 |
| A | BIROCCIO, A. et al, The future of antisense therapy: combination with anticancer treatments, Oncogene, 2003, Vol.22, No.42, p.6579-88 | 9-17,19, 21-24 |
| A | KONISHI, T. et al, The K-ras gene regulates vascular endothelial growth factor gene expression in non-small cell lung cancers, International Journal of Oncology, 2000, Vol.16, No.3, p.501-511 | 9-17,19, 21-24 |
| A | HASSAN, I. et al, Antisense p53 decreases production of VEGF in follicular thyroid cancer cells, Endocrine, 2006, Vol.29, No.3, p.409-12 | 9-17,19, 21-24 |
| P,X | MASUDA, K. et al, A novel tumor-promoting function residing in the 5' non-coding region of vascular endothelial growth factor mRNA, PLoS Med, 2008.05.20, Vol.5, No.5, p.e94 | 9-17,19, 21-24 |

Form PCT/ISA/210 (continuation of second sheet) (April 2007)

**INTERNATIONAL SEARCH REPORT**

International application No.
PCT/JP2008/066127

**Box No. II Observations where certain claims were found unsearchable (Continuation of item 2 of first sheet)**

This international search report has not been established in respect of certain claims under Article 17(2)(a) for the following reasons:

1. [X] Claims Nos.: 1-8, 18, 20
because they relate to subject matter not required to be searched by this Authority, namely:
The invention of the above claims pertains to "a method for treatment of a human body by therapy" and thus relates to a subject matter which this International Searching Authority is not required, under the provisions of PCT Rule 39.1(iv), to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.

2. [ ] As all searchable claims could be searched without effort justifying additional fees, this Authority did not invite payment of additional fees.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the

[ ] The additional search fees were accompanied by the applicant's protest and, where applicable, payment of a protest fee.

[ ] The additional search fees were accompanied by the applicant's protest but the applicable protest fee was not paid within the time limit specified in the invitation.

[ ] No protest accompanied the payment of additional search fees.

Form PCT/ISA/210 (continuation of first sheet (2)) (April 2007)

INTERNATIONAL SEARCH REPORT

International application No.
PCT/JP2008/066127

<Subject of search>

Claim 15 of the present application refers to the method claimed in claim 1, and relates to "an apoptosis promoter characterized by being capable of promoting the expression of p53" through "the inhibition of the binding of RNA that is transcribed from exon-1 in vegf-A gene to mRNA that is transcribed from p53 gene". Claim 15 includes all of the compounds having that function. However, a substance which is disclosed in the meaning within PCT Article 5 is only siRNA that targets a part of specific mRNA, which is an extremely small part of the compounds included in claim 15. Therefore, it cannot be considered that this claim is supported by the disclosure of the description in the meaning within PCT Article 6.

Further, with regard to the compound "capable of inhibiting the binding of RNA that is transcribed from exon-1 in vegf-A gene to mRNA that is transcribed from p53 gene", even though the common technical knowledge at the time of filing the present application is taken into the consideration, it is impossible to specify the scope of the compound having the property. Therefore, claim 15 does not comply with the requirement of clearness under PCT Article 6.

Thus, the search was made on, as the compound "capable of inhibiting the binding of RNA that is transcribed from exon-1 in vegf-A gene to mRNA that is transcribed from p53 gene", nucleic acid substances such as an antisense, siRNA or a ribozyme for exon-1 or a 5'-UTR region in vegf-A or a specific part of p53 mRNA, which is a substance that can be presumed tentatively from the contents of the description.

With regard to claims 9-14, 16, 17, 19 and 21-24 (excluding claim 15), the search was made completely.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Steele, R. et al.** *Br. J. Surg.,* 1998, vol. 85, 1460-7
- **Levine, A. et al.** *Cell,* 1997, vol. 88, 323-331
- **Vogelstein, B. et al.** *Nature,* 2000, vol. 408, 307-310
- **Pharoah, P. et al.** *Br. J. Cancer,* 1999, vol. 80, 1968-1973
- **Raman, V. et al.** *Nature,* 2000, vol. 405, 974-978
- **Hirashima ; Inoue.** Shin Seikagaku Jikken Koza 2, Kakusan IV, Idenshi no Fukusei to Hatsugen. Tokyo Kagaku Dojin, 1993, 319-347
- *Gene,* 1991, vol. 108, 193-200
- **Huez, I. et al.** *Mol. Endocrinol.,* 2001, vol. 15, 2197-2210
- **Masuda, K. et al.** *PLoS Med.,* 2008, vol. 5, e94
- **Bunz, F. et al.** *J. Clin. Invest.,* 1999, vol. 104, 263-269
- **J. Abraham et al.** *J. Biol. Chem.,* 1991, vol. 266 (18), 11947-11954
- **Bornes, S. et al.** *J. Biol. Chem.,* 2004, vol. 279, 18717-18726
- **Huez, I. et al.** *Mol. Cell. Biol.,* 1998, vol. 18, 6178-6190
- **Akiri, G. et al.** *Oncogene,* 1998, vol. 17, 227-236
- **Kawano, M. et al.** *Nucleic Acid Res.,* 2005, vol. 33 (3), 1040-1050

SEQUENCE LISTING

<110> The University of Tokushima

<120> Method for promotion of p53 expression

<130> TF08024-01

<150> JP 2007-233316
<151> 2007-09-07

<160> 95

<170> PatentIn version 3.1

<210> 1
<211> 3621
<212> RNA
<213> Homo sapiens

<220>
<221> 5'UTR
<222> (1)..(1038)
<223>

<400> 1
ucgcggaggc uuggggcagc cggguagcuc ggaggucgug gcgcuggggg cuagcaccag 60

cgcucugucg ggaggcgcag cgguuaggug gaccggucag cggacucacc ggccagggcg 120

cucggugcug gaauuugaua uucauugauc cggguuuuau cccucuucuu uuuucuuaaa 180

cauuuuuuuu uaaaacugua uuguuucucg uuuuaauuua uuuuugcuug ccauucccca 240

cuugaaucgg gccgacggcu uggggagauu gcucuacuuc cccaaaucac uguggauuuu 300

ggaaaccagc agaaagagga aagagguagc aagagcucca gagagaaguc gaggaagaga 360

gagacggggu cagagagagc gcgcgggcgu gcgagcagcg aaagcgacag gggcaaagug 420

agugaccugc uuuuggggu gaccgccgga gcgcggcgug agcccucccc cuugggaucc 480

cgcagcugac cagucgcgcu gacggacaga cagacagaca ccgcccccag ccccagcuac 540

caccuccucc ccggccggcg gcggacagug gacgcggcgg cgagccgcgg gcaggggccg 600

gagcccgcgc ccggaggcgg gguggagggg gucggggcuc gcggcgucgc acugaaacuu 660

uucguccaac uucugggcug uucucgcuuc ggaggagccg ugguccgcgc gggggaagcc 720

gagccgagcg gagccgcgag aagugcuagc ucgggccggg aggagccgca gccggaggag 780

ggggaggagg aagaagagaa ggaagaggag agggggccgc aguggcgacu cggcgcucgg 840

aagccgggcu cauggacggg ugaggcggcg gugugcgcag acagugcucc agccgcgcgc 900

gcuccccagg cccuggcccg ggccucgggc cggggaggaa gaguagcucg ccgaggcgcc 960

gaggagagcg ggccgcccca cagcccgagc cggagaggga gcgcgagccg cgccggcccc 1020

ggucgggccu ccgaaaccau gaacuuucug cugucuuggg ugcauuggag ccuugccuug 1080

cugcucuacc uccaccaugc caaguggucc caggcugcac ccauggcaga aggaggaggg 1140

cagaaucauc acgaaguggu gaaguucaug gaugucuauc agcgcagcua cugccaucca 1200

aucgagaccc ugguggacau cuuccaggag uacccugaug agaucgagua caucuucaag 1260

ccauccugug ugccccugau gcgaugcggg ggcugcugca augacgaggg ccuggagugu 1320
gugcccacug aggaguccaa caucaccaug cagauuaugc ggaucaaacc ucaccaaggc 1380
cagcacauag gagagaugag cuuccuacag cacaacaaau gugaaugcag accaaagaaa 1440
gauagagcaa gacaagaaaa aaaaucaguu cgaggaaagg gaaaggggca aaaacgaaag 1500
cgcaagaaau cccgguauaa guccuggagc guucccugug ggccuugcuc agagcggaga 1560
aagcauuugu uuguacaaga uccgcagacg uguaaauguu ccugcaaaaa cacagacucg 1620
cguugcaagg cgaggcagcu ugaguuaaac gaacguacuu gcagauguga caagccgagg 1680
cggugagccg ggcaggagga aggagccucc cucaggguuu cgggaaccag aucucucacc 1740
aggaaagacu gauacagaac gaucgauaca gaaaccacgc ugccgccacc acaccaucac 1800
caucgacaga acaguccuua auccagaaac cugaaaugaa ggaagaggag acucugcgca 1860
gagcacuuug gguccggagg gcgagacucc ggcggaagca uucccgggcg ggugacccag 1920
cacggucccu cuuggaauug gauucgccau uuuauuuuuc uugcugcuaa aucaccgagc 1980
ccggaagauu agagaguuuu auuucuggga uuccuguaga cacacccacc cacauacaua 2040
cauuuauaua uauauauauu auauauauau aaaaauaaau aucucuauuu uauauauaua 2100
aaauauauau auucuuuuuu uaaauuaaca gugcuaaugu uauuggugucc uucacuggau 2160
guauuugacu gcuguggacu ugaguuggga ggggaauguu cccacucaga uccugacagg 2220
gaagaggagg agaugagaga cucuggcaug aucuuuuuuu ugucccacuu gguggggcca 2280
ggguccucuc cccugcccag gaaugugcaa ggccagggca ugggggcaaa uaugacccag 2340
uuuugggaac accgacaaac ccagcccugg cgcugagccu cucuacccca ggucagacgg 2400
acagaaagac agaucacagg uacagggaug aggacaccgg cucugaccag gaguuugggg 2460
agcuucagga cauugcugug cuuuggggau ucccuccaca ugcugcacgc gcaucucgcc 2520
cccaggggca cugccuggaa gauucaggag ccugggcggc cuucgcuuac ucucaccugc 2580
uucugaguug cccaggagac cacuggcaga ugucccggcg aagagaagag acacauuguu 2640
ggaagaagca gcccaugaca gcuccccuuc cugggacucg cccucauccu cuuccugcuc 2700
cccuuccugg ggugcagccu aaaaggaccu auguccucac accauugaaa ccacuaguuc 2760
uguccccccca ggagaccugg uugugugugu gugagugguu gaccuucccuc cauccccugg 2820
uccuucccuu cccuucccga ggcacagaga gacagggcag gauccacgug cccauugugg 2880
aggcagagaa aagagaaagu guuuuauaua cgguacuuau uuaauauccc uuuuuaauua 2940
gaaauuaaaa caguuaauuu aauuaaagag uaggguuuuu uuucaguauu cuugguuaau 3000
auuuaauuuc aacuauuuau gagauguauc uuuugcucuc ucuugcucuc uuauuuguac 3060
cgguuuuugu auauaaaauu cauguuucca aucucucucu cccugaucgg ugacagucac 3120
uagcuuaucu ugaacagaua uuuaauuuug cuaacacuca gcucugcccu ccccgauccc 3180
cuggcucccc agcacacauu ccuuugaaau aagguuucaa uauacaucua cauacuauau 3240
auauauuugg caacuuguau uuguguguau auauauauau auauguuuau guauauaugu 3300

gauucugaua aaauagacau ugcuauucug uuuuuuauau guaaaaacaa aacaagaaaa 3360
aauagagaau ucuacauacu aaaucucucu ccuuuuuuaa uuuuaauauu uguuaucauu 3420
uauuuauugg ugcuacuguu uauccguaau aauugugggg aaaagauauu aacaucacgu 3480
cuuugucucu agugcaguuu uucgagauau uccguaguac auauuuauuu uuaaacaacg 3540
acaaagaaau acagauauau cuuaaaaaaa aaaaagcauu uuguauuaaa gaauuuaauu 3600
cugaucucaa aaaaaaaaaa a 3621

<210> 2
<211> 2640
<212> RNA
<213> Homo sapiens

<400> 2
acuugucaug gcgacugucc agcuuugugc caggagccuc gcagggguug augggauugg 60
gguuuucccc ucccaugugc ucaagacugg cgcuaaaagu uuugagcuuc ucaaaagucu 120
agagccaccg uccagggagc agguagcugc ugggcuccgg ggacacuuug cguucgggcu 180
gggagcgugc uuuccacgac ggugacacgc uucccuggau uggcagccag acugccuucc 240
gggucacugc cauggaggag ccgcagucag auccuagcgu cgagccccu cugagucagg 300
aaacauuuuc agaccuaugg aaacuacuuc cugaaaacaa cguucugucc cccuugccgu 360
cccaagcaau ggaugauuug augcuguccc cggacgauau ugaacaaugg uucacugaag 420
acccaggucc agaugaagcu cccagaaugc cagaggcugc uccccccgug gccccugcac 480
cagcagcucc uacaccggcg gccccugcac cagccccuc cuggccccug ucaucuucug 540
ucccuuccca gaaaaccuac cagggcagcu acgguuuccg ucugggcuuc uugcauucug 600
ggacagccaa gucugugacu ugcacguacu ccccugcccu caacaagaug uuuugccaac 660
uggccaagac cugcccugug cagcuguggg uugauuccac acccccgccc ggcacccgcg 720
uccgcgccau ggccaucuac aagcagucac agcacaugac ggagguugug aggcgcugcc 780
cccaccauga gcgcugcuca gauagcgaug gucuggcccc uccucagcau cuuauccgag 840
uggaaggaaa uuugcgugug gaguauuugg augacagaaa cacuuuucga cauagugugg 900
uggugcccua ugagccgccu gagguuggcu cugacuguac caccauccac uacaacuaca 960
uguguaacag uuccugcaug ggcggcauga accggaggcc cauccucacc aucaucacac 1020
uggaagacuc cagugguaau cuacugggac ggaacagcuu ugaggugcgu guuugugccu 1080
guccugggag agaccggcgc acagaggaag agaaucuccg caagaaaggg gagccucacc 1140
acgagcugcc cccagggagc acuaagcgag cacugcccaa caacaccagc uccucucccc 1200
agccaaagaa gaaaccacug gauggagaau auuucacccu ucagauccgu gggcgugagc 1260
gcuucgagau guuccgagag cugaaugagg ccuuggaacu caaggaugcc caggcuggga 1320
aggagccagg ggggagcagg gcucacucca gccaccugaa guccaaaaag ggucagucua 1380
ccucccgcca uaaaaaacuc auguucaaga cagaagggcc ugacucagac ugacauucuc 1440
cacuucuugu uccccacuga cagccuccca cccccaucuc ucccuccccu gccauuuugg 1500

guuuugggguc uuugaacccu ugcuugcaau aggugugcgu cagaagcacc caggacuucc 1560
auuugcuuug ucccggggcu ccacugaaca aguuggccug cacugguguu uuguuguggg 1620
gaggaggaug gggaguagga cauaccagcu uagauuuuaa gguuuuuacu gugagggaug 1680
uuugggagau guaagaaaug uucuugcagu uaaggguuag uuuacaauca gccacauucu 1740
agguaggggc ccacuucacc guacuaacca gggaagcugu cccucacugu ugaauuuucu 1800
cuaacuucaa ggcccauauc ugugaaaugc uggcauuugc accuaccuca cagagugcau 1860
ugugaggguu aaugaaauaa uguacaucug gccuugaaac caccuuuuau uacaugggu 1920
cuagaacuug acccccuuga gggugcuugu ucccucuccc uguuggucgg uggguuggua 1980
guuucuacag uugggcagcu gguuagguag agggaguugu caagucucug cuggcccagc 2040
caaacccugu cugacaaccu cuuggugaac cuuaguaccu aaaaggaaau cucaccccau 2100
cccacacccu ggaggauuuc aucucuugua uaugaugauc uggauccacc aagacuuguu 2160
uuaugcucag ggucaauuuc uuuuuucuuu uuuuuuuuuu uuuuucuuuu ucuuugagac 2220
ugggucucgc uuuguugccc aggcuggagu ggaguggcgu gaucuuggcu uacugcagcc 2280
uuugccuccc cggcucgagc aguccugccu cagccuccgg aguagcuggg accacagguu 2340
caugccacca uggccagcca acuuuugcau guuuuguaga gauggggucu cacaguguug 2400
cccaggcugg ucucaaacuc cugggcucag gcgauccacc ugucucagcc ucccagagug 2460
cugggauuac aauugugagc caccacgucc agcuggaagg gucaacaucu uuuacauucu 2520
gcaagcacau cugcauuuuc accccacccu uccccuccuu cucccuuuuu auaucccauu 2580
uuuauaucga ucucuuauuu uacaauaaaa cuuugcugcc accugugugu cugaggggug 2640

<210> 3
<211> 21
<212> RNA
<213> Artificial

<220>
<223> siRNA

<220>
<221> misc_feature
<222> (20)..(21)
<223> n stands for any base

<400> 3
gcauuggacu ugccuugcun n 21

<210> 4
<211> 23
<212> RNA
<213> Artificial

<220>
<223> siRNA

<220>
<221> misc_feature

<222> (22)..(23)
<223> n stands for any base

<400> 4
agcaaggcaa ggcuccaaug cnn 23

<210> 5
<211> 21
<212> RNA
<213> Artificial

<220>
<223> siRNA

<220>
<221> misc_feature
<222> (20)..(21)
<223> n stands for any base

<400> 5
ggagccuugu ugcugcucun n 21

<210> 6
<211> 23
<212> RNA
<213> Artificial

<220>
<223> siRNA

<220>
<221> misc_feature
<222> (22)..(23)
<223> n stands for any base

<400> 6
agagcagcaa ggcaaggcuc cnn 23

<210> 7
<211> 21
<212> RNA
<213> Artificial

<220>
<223> siRNA

<220>
<221> misc_feature
<222> (20)..(21)
<223> n stands for any base

<400> 7
augccaagaa gcggucgugn n 21

<210> 8
<211> 23
<212> RNA
<213> Artificial

<220>

<223> siRNA

<220>
<221> misc_feature
<222> (22)..(23)
<223> n stands for any base

<400> 8
cacgaccgcu uaccuuggca unn 23

<210> 9
<211> 21
<212> RNA
<213> Artificial

<220>
<223> siRNA

<220>
<221> misc_feature
<222> (20)..(21)
<223> n stands for any base

<400> 9
gcggagccga gaagugcuan n 21

<210> 10
<211> 23
<212> RNA
<213> Artificial

<220>
<223> siRNA

<220>
<221> misc_feature
<222> (22)..(23)
<223> n stands for any base

<400> 10
uagcacuucu cgcggcuccg cnn 23

<210> 11
<211> 21
<212> RNA
<213> Artificial

<220>
<223> siRNA

<220>
<221> misc_feature
<222> (20)..(21)
<223> n stands for any base

<400> 11
gccgcgaggu gcuagcucgn n 21

<210> 12
<211> 23

<212> RNA
<213> Artificial

<220>
<223> siRNA

<220>
<221> misc_feature
<222> (22)..(23)
<223> n stands for any base

<400> 12
cgagcuagca cuucucgcgg cnn 23

<210> 13
<211> 27
<212> RNA
<213> Artificial

<220>
<223> siRNA

<220>
<221> misc_feature
<222> (26)..(27)
<223> n stands for any base

<400> 13
gcgucgcacu gaaacuuuuc guccann 27

<210> 14
<211> 27
<212> RNA
<213> Artificial

<220>
<223> siRNA

<220>
<221> misc_feature
<222> (26)..(27)
<223> n stands for any base

<400> 14
uggacgaaaa guuucagugc gacgcnn 27

<210> 15
<211> 27
<212> RNA
<213> Artificial

<220>
<223> siRNA

<220>
<221> misc_feature
<222> (26)..(27)
<223> n stands for any base

<400> 15
cacugaaacu uuucguccaa cuucunn 27

<210> 16
<211> 27
<212> RNA
<213> Artificial

<220>
<223> siRNA

<220>
<221> misc_feature
<222> (26)..(27)
<223> n stands for any base

<400> 16
agaaguugga cgaaaaguuu cagugnn 27

<210> 17
<211> 27
<212> RNA
<213> Artificial

<220>
<223> siRNA

<220>
<221> misc_feature
<222> (26)..(27)
<223> n stands for any base

<400> 17
gcacugaaac uuuucgucca acuucnn 27

<210> 18
<211> 27
<212> RNA
<213> Artificial

<220>
<223> siRNA

<220>
<221> misc_feature
<222> (26)..(27)
<223> n stands for any base

<400> 18
gaaguuggac gaaaaguuuc agugcnn 27

<210> 19
<211> 27
<212> RNA
<213> Artificial

<220>
<223> siRNA

<220>
<221> misc_feature
<222> (26)..(27)
<223> n stands for any base

<400> 19
ggcgucgcac ugaaacuuuu cguccnn 27

<210> 20
<211> 27
<212> RNA
<213> Artificial

<220>
<223> siRNA

<220>
<221> misc_feature
<222> (26)..(27)
<223> n stands for any base

<400> 20
ggacgaaaag uuucagugcg acgccnn 27

<210> 21
<211> 27
<212> RNA
<213> Artificial

<220>
<223> siRNA

<220>
<221> misc_feature
<222> (26)..(27)
<223> n stands for any base

<400> 21
cgucgcacug aaacuuuucg uccaann 27

<210> 22
<211> 27
<212> RNA
<213> Artificial

<220>
<223> siRNA

<220>
<221> misc_feature
<222> (26)..(27)
<223> n stands for any base

<400> 22
uuggacgaaa aguuucagug cgacgnn 27

<210> 23
<211> 64
<212> DNA
<213> Artificial

<220>
<223> oligonucleotide

<400> 23
tgctgagcaa ggcaaggctc caatgcgttt tggccactga ctgacgcatt ggacttgcct 60

tgct 64

<210> 24
<211> 21
<212> RNA
<213> Homo sapiens

<400> 24
gcauuggagc cuugccuugc u 21

<210> 25
<211> 64
<212> DNA
<213> Artificial

<220>
<223> oligonucleotide

<400> 25
tgctgagagc agcaaggcaa ggctccgttt tggccactga ctgacggagc cttgttgctg 60

ctct 64

<210> 26
<211> 21
<212> RNA
<213> Homo sapiens

<400> 26
ggagccuugc cuugcugcuc u 21

<210> 27
<211> 64
<212> DNA
<213> Artificial

<220>
<223> oligonucleotide

<400> 27
tgctgcacga ccgcttacct tggcatgttt tggccactga ctgacatgcc aagaagcggt 60

cgtg 64

<210> 28
<211> 21
<212> RNA
<213> Homo sapiens

<400> 28
augccaaggu aagcggucgu g 21

<210> 29
<211> 64
<212> DNA
<213> Artificial

<220>
<223> oligonucleotide

<400> 29
tgctgtagca cttctcgcgg ctccgcgttt tggccactga ctgacgcgga gccgagaagt 60

gcta 64

<210> 30
<211> 21
<212> RNA
<213> Homo sapiens

<400> 30
gcggagccgc gagaagugcu a 21

<210> 31
<211> 64
<212> DNA
<213> Artificial

<220>
<223> oligonucleotide

<400> 31
tgctgcgagc tagcacttct cgcggcgttt tggccactga ctgacgccgc gaggtgctag 60

ctcg 64

<210> 32
<211> 21
<212> RNA
<213> Homo sapiens

<400> 32
gccgcgagaa gugcuagcuc g 21

<210> 33
<211> 61
<212> DNA
<213> Artificial

<220>
<223> fragment

<400> 33
gcgagccgcg ggcaaggacc agaaccagca ccagggggag gagtagaagg agtaggggct 60

c 61

<210> 34
<211> 116
<212> DNA
<213> Artificial

<220>
<223> fragment

<400> 34
gggctcgagg agtagcatta aagctatttg tacagttatt aggatgctca cgatttgggg 60

gggcagtagt aagagcagga gaggcagaac caagtggggc agcaaggagt gctagc 116

<210> 35

<211> 107
<212> DNA
<213> Artificial

<220>
<223> fragment

<400> 35
gctagctcag gaagagaaga accacaacca gaagaaggag aagaagagga ggaaaaagag 60

gaagaacgtg gaccacaatg gaggttagga gcaagaaaac cgggctc 107

<210> 36
<211> 82
<212> DNA
<213> Artificial

<220>
<223> fragment

<400> 36
gggctcatgg acaggagaag cagcagtatg tgccgatagc gcacctgcag caagagcacc 60

acaagcatta gcccgggcct cg 82

<210> 37
<211> 51
<212> DNA
<213> Artificial

<220>
<223> fragment

<400> 37
gggcaaggac cagaaccagc accaggggga ggagtagaag gagtaggggc t 51

<210> 38
<211> 49
<212> DNA
<213> Artificial

<220>
<223> fragment

<400> 38
cctactcctt ctactcctcc ccctggtgct ggttctggtc cttgcccgc 49

<210> 39
<211> 106
<212> DNA
<213> Artificial

<220>
<223> fragment

<400> 39
cgaggagtag cattaaagct atttgtacag ttattaggat gctcacgatt tgggggggca 60

gtagtaagag caggagaggc agaaccaagt ggggcagcaa ggagtg 106

<210> 40
<211> 114
<212> DNA

<213> Artificial

<220>
<223> fragment

<400> 40
ctagcactcc ttgctgcccc acttggttct gcctctcctg ctcttactac tgcccccca 60

aatcgtgagc atcctaataa ctgtacaaat agctttaatg ctactcctcg agcc 114

<210> 41
<211> 105
<212> DNA
<213> Artificial

<220>
<223> fragment

<400> 41
ctagctcagg aagagaagaa ccacaaccag aagaaggaga agaagaggag gaaaaagagg 60

aagaacgtgg accacaatgg aggttaggag caagaaaacc gggct 105

<210> 42
<211> 97
<212> DNA
<213> Artificial

<220>
<223> fragment

<400> 42
cggttttctt gctcctaacc tccattgtgg tccacgttct tcctcttttt cctcctcttc 60

ttctccttct tctggttgtg gttcttctct tcctgag 97

<210> 43
<211> 67
<212> DNA
<213> Artificial

<220>
<223> fragment

<400> 43
catggacagg agaagcagca gtatgtgccg atagcgcacc tgcagcaaga gcaccacaag 60

cattagc 67

<210> 44
<211> 75
<212> DNA
<213> Artificial

<220>
<223> fragment

<400> 44
ccgggctaat gcttgtggtg ctcttgctgc aggtgcgcta tcggcacata ctgctgcttc 60

tcctgtccat gagcc 75

<210> 45

<211> 4146
<212> RNA
<213> Homo sapiens

<400> 45
ucgcggaggc uuggggcagc cggguagcuc ggaggucgug gcgcuggggg cuagcaccag 60
cgcucugucg ggaggcgcag cgguuaggug gaccggucag cggacucacc ggccagggcg 120
cucggugcug gaauuugaua uucauugauc cggguuuuau cccucuucuu uuuucuuaaa 180
cauuuuuuuu uaaaacugua uuguuucucg uuuuaauuua uuuuugcuug ccauucccca 240
cuugaaucgg gccgacggcu uggggagauu gcucuacuuc cccaaaucac uguggauuuu 300
ggaaaccagc agaaagagga aagagguagc aagagcucca gagagaaguc gaggaagaga 360
gagacggggu cagagagagc gcgcgggcgu gcgagcagcg aaagcgacag ggcaaagug 420
agugaccugc uuuuggggu gaccgccgga gcgcggcgug agcccucccc cuugggaucc 480
cgcagcugac cagucgcgcu gacggacaga cagacagaca ccgcccccag ccccagcuac 540
caccuccucc ccggccggcg gcggacagug gacgcggcgg cgagccgcgg gcaggggccg 600
gagcccgcgc ccggaggcgg gguggagggg gucggggcuc gcggcgucgc acugaaacuu 660
uucguccaac uucugggcug uucucgcuuc ggaggagccg ugguccgcgc gggggaagcc 720
gagccgagcg gagccgcgag aagugcuagc ucgggccggg aggagccgca gccggaggag 780
ggggaggagg aagaagagaa ggaagaggag agggggccgc aguggcgacu cggcgcucgg 840
aagccgggcu cauggacggg ugaggcggcg gugugcgcag acagugcucc agccgcgcgc 900
gcuccccagg cccuggcccg ggccucgggc cggggaggaa gaguagcucg ccgaggcgcc 960
gaggagagcg ggccgcccca cagcccgagc cggagaggga gcgcgagccg cgccggcccc 1020
ggucgggccu ccgaaaccau gaacuuucug cugucuuggg ugcauuggag ccuugccuug 1080
cugcucuacc uccaccaugc caagguaagc ggucgugccc ugcuggcgcc gcgggccgcu 1140
gcgagcgccu cucccggcug gggacgugcg ugcgagcgcg cgcgugggg cuccgugccc 1200
cacgcggguc caugggcacc aggcgugcgg cgucccccuc ugucgucuua ggugcagggg 1260
gagggggcgc gcgcgcuagg ugggagggua cccggagaga ggcucaccgc ccacgcgggc 1320
ccugcccacc caccggaguc accgcacgua cgaucugggc cgaccagccg agggcgggag 1380
ccggaggagg aggccgaggg ggcugggcuu gcguugccgc ugccggcuga aguuugcucc 1440
cggccgcugg ucccggacga acuggaaguc ugagcagcgg gggcgggagc cagagaccag 1500
ugggcagggg gugcucggac cuuggaccgc gggagggcag agagcgugga gggggcaggg 1560
cgcaggaggg agaggggggcu ugcugucacu gccacucggu cucuucagcc cucgccgcga 1620
guuugggaaa aguuuugggg uggauugcug cggggacccc cccucccugc ugggccaccu 1680
gcgccgcgcc aaccccgccc gucccccgcuc gcgucccgcu cggugcccgc ccuccccgc 1740
ccggccgggu gcgcgcggcg cggagccgau uacaucagcc cgggccuggc cggccgcgug 1800
uucccggagc cucggcugcc cgaaugggga gcccagagug gcgagcggca ccccuccccc 1860
cgccagcccu ccgcgggaag gugaccucuc gagguagccc cagcccgggg auccagagaa 1920

ccaucccuac cccuuccuac ugucuccaga cccuaccucu gcccagugcu aggaggaauu 1980
uccugacgcc ccuucucuuc acccauuucc uuuuuagccu ggagagaagc cccugucacc 2040
ccgcuuauuu ucauuucucu cugcggagaa gauccaucua accccuuucu ggccccagag 2100
uccagggaaa ggaugaucac ugucagaagu cguggcgcgg gagcccacug ggcgcuuugu 2160
cacauuccac cgaaaguccc gacuugguga cagugugcuu cccuucccuc gccaacaguu 2220
ccgagugagc ugugcuuuag cucucguggg ggugggucaa gggaggauuu gaagagucau 2280
ugccccacuu uacccuuuug gagaaauggc uugaaauuug cugugacacg ggcagcaugg 2340
gaauaguccu uccugaaccc uggaaaggag cuccugccag ccuugcacac acuuuguccu 2400
ggugaaaggc agcccuggag cagguguuuu uuuggaacuc caaaccugcc cacccaacuu 2460
gcuucugaaa gggacucuaa agggucccuu uccgcucccuc ucugacgccu ucccucagcc 2520
agaauucccu uggagaggag gcaagaggaa agccauggac aggggucgcu gcuaacaccg 2580
caaguuccuc agacccuggc acaaaggccu uggcuacagg ccuccaagua gggaggaggg 2640
ggaggagugg cugccuggcc acagugugac cuucagaggc ccccagagaa ggacaccugg 2700
ccccugccug ccuagaaccg ccccuccugu gcucccuggc cuuggaaggg guaugaaauu 2760
uccgucccccu uuccuccuug gggcccagga ggaguggagg gucccgggag aauauuguca 2820
gggggaaggc agggggugUc augggaaugg gugaggggc ugaggugcag aauccagggg 2880
gucccugcag gagccgcagu gguaagcugu ccagcuggaa gccugguaac uguuguuuuc 2940
ucuugagagg ggcuuccugu gaccuuggcu gucucuggga gcagggcugg gguaccugag 3000
uggggugcau uugggguguug ugggaaggag agggaaagaa agauggacag ugggacucuc 3060
cccuagcagg gucugguguu ccguaggcua gagugccccu cugcucugcg agugcugggc 3120
gggaggggag uuggugagag cuggagaccc ccaggaaggg cuggcagaag ccuuuccuuu 3180
ugggugcugu cagguccgca ugucuuggcg uguugaccuu cacagcuucu ggcgagggga 3240
ggaaugaucu gaugcgggug gggaggguua gaggaggccu caggccuaag guggugcagg 3300
gggcccccua ggggcugggc agugccaagg cauaaaagcc uucccugguc ccugguggca 3360
uuugaaggug cccaggugag aggggcuugg caccuccuca cccugggagg gagaagaaac 3420
cagggaacag guaggagugg gagacaggug aggcuuugga aaucuauuga ggcucuggag 3480
agauuugugu agagaggaaa augugguucu cccccagggu cuccuccugg guuuuuaccc 3540
ucuaagcaac cugugggcau gcuggguuau ucccuaaggac uagaagagcu uggaugggggg 3600
agggugguug gugcccuucg guccucggca ccccccuccg ucuccaacac cagcucaccc 3660
ugguauuugu caugucagca ggagaagguc accauguugu uuuucucgcc ccuaguccuu 3720
ccuuccugcc ccaguccaaa uuuguccucc uauuugaccu uaauacuuac cauggcuuug 3780
gaccagggaa cuagggggau agugagagca gggagaggga agugugggga agguacaggg 3840
gaccucgaca gugaagcauu cuggggguuuu ccuccugcau uucgagcucc ccagccccca 3900
acaucugguu agucuuuaac uuccucgggu ucauaaccau agcaguccag gaguggugggg 3960

cauauucugu gcccgugggg acccccgguu guguccuguu cgacucagaa gacuuggaga 4020

agccagaggc uguuggugg agggaaguga ggagggagga ggggcugggu ggcugggccu 4080

gugcacccca gccccugccc augcccaugc cuugcucucu uucuguccuc aguggucca 4140

ggcugc 4146

<210> 46
<211> 60
<212> DNA
<213> Artificial

<220>
<223> oligonucleotide for control siRNA

<400> 46
gaaatgtact gcgcgtggag acgttttggc cactgactga cgtctccacg cagtacattt 60

<210> 47
<211> 27
<212> DNA
<213> Artificial

<220>
<223> primer

<400> 47
gtcttgctct atctttcttt ggtctgc 27

<210> 48
<211> 15
<212> DNA
<213> Artificial

<220>
<223> primer

<400> 48
catctctcct atgtg 15

<210> 49
<211> 15
<212> DNA
<213> Artificial

<220>
<223> primer

<400> 49
tccaggccct cgtca 15

<210> 50
<211> 12
<212> DNA
<213> Artificial

<220>
<223> primer

<400> 50
tcagtctgag tc 12

<210> 51
<211> 12
<212> DNA
<213> Artificial

<220>
<223> primer

<400> 51
gcccacggat ct 12

<210> 52
<211> 12
<212> DNA
<213> Artificial

<220>
<223> primer

<400> 52
tgatggtgag ga 12

<210> 53
<211> 12
<212> DNA
<213> Artificial

<220>
<223> primer

<400> 53
cctcacaacc tc 12

<210> 54
<211> 12
<212> DNA
<213> Artificial

<220>
<223> primer

<400> 54
aagatgacag gg 12

<210> 55
<211> 20
<212> DNA
<213> Artificial

<220>
<223> primer

<400> 55
ttgatcctag cagaagcaca 20

<210> 56
<211> 45
<212> DNA
<213> Artificial

<220>

<223> probe

<400> 56
gctctgctgg agcccgcgcg gggctcggtt gagcgttctt gcgcg 45

<210> 57
<211> 43
<212> DNA
<213> Artificial

<220>
<223> probe

<400> 57
ccctctacgg gctccgggga gctgccctcc tggcgtgcgc ggg 43

<210> 58
<211> 44
<212> DNA
<213> Artificial

<220>
<223> probe

<400> 58
ggtggacgca tcctgaggca ccgggtccag ggccagctcg ggtg 44

<210> 59
<211> 55
<212> DNA
<213> Artificial

<220>
<223> probe

<400> 59
ggcaggcctt gctgccgcat gggttctgac ggacatcccc agccggttct gacat 55

<210> 60
<211> 27
<212> RNA
<213> Artificial

<220>
<223> siRNA

<220>
<221> misc_feature
<222> (26)..(27)
<223> n stands for any base

<400> 60
cacugaaacu uuucguccaa cuucunn 27

<210> 61
<211> 27
<212> RNA
<213> Artificial

<220>
<223> siRNA

<220>
<221> misc_feature
<222> (26)..(27)
<223> n stands for any base

<400> 61
agaaguugga cgaaaaguuu cagugnn 27

<210> 62
<211> 27
<212> RNA
<213> Artificial

<220>
<223> siRNA

<220>
<221> misc_feature
<222> (26)..(27)
<223> n stands for any base

<400> 62
gggcucgcgg cgucgcacug aaacunn 27

<210> 63
<211> 27
<212> RNA
<213> Artificial

<220>
<223> siRNA

<220>
<221> misc_feature
<222> (26)..(27)
<223> n stands for any base

<400> 63
aguuucagug cgacgccgcg agcccnn 27

<210> 64
<211> 27
<212> RNA
<213> Artificial

<220>
<223> siRNA

<220>
<221> misc_feature
<222> (26)..(27)
<223> n stands for any base

<400> 64
ccaacuucug ggcuguucuc gcuucnn 27

<210> 65
<211> 27
<212> RNA
<213> Artificial

<220>
<223> siRNA

<220>
<221> misc_feature
<222> (26)..(27)
<223> n stands for any base

<400> 65
gaagcgagaa cagcccagaa guuggnn 27

<210> 66
<211> 27
<212> RNA
<213> Artificial

<220>
<223> siRNA

<220>
<221> misc_feature
<222> (26)..(27)
<223> n stands for any base

<400> 66
gggugcauug gagccuugcc uugcunn 27

<210> 67
<211> 27
<212> RNA
<213> Artificial

<220>
<223> siRNA

<220>
<221> misc_feature
<222> (26)..(27)
<223> n stands for any base

<400> 67
agcaaggcaa ggcuccaaug cacccnn 27

<210> 68
<211> 27
<212> RNA
<213> Artificial

<220>
<223> siRNA

<220>
<221> misc_feature
<222> (26)..(27)
<223> n stands for any base

<400> 68
cauuggagcc uugccuugcu gcucunn 27

<210> 69
<211> 27
<212> RNA
<213> Artificial

<220>
<223> siRNA

<220>
<221> misc_feature
<222> (26)..(27)
<223> n stands for any base

<400> 69
agagcagcaa ggcaaggcuc caaugnn 27

<210> 70
<211> 27
<212> RNA
<213> Artificial

<220>
<223> siRNA

<220>
<221> misc_feature
<222> (26)..(27)
<223> n stands for any base

<400> 70
cggagccgcg agaagugcua gcucgnn 27

<210> 71
<211> 27
<212> RNA
<213> Artificial

<220>
<223> siRNA

<220>
<221> misc_feature
<222> (26)..(27)
<223> n stands for any base

<400> 71
cgagcuagca cuucucgcgg cuccgnn 27

<210> 72
<211> 27
<212> RNA
<213> Artificial

<220>
<223> siRNA

<220>
<221> misc_feature
<222> (26)..(27)
<223> n stands for any base

<400> 72
cgcuucggag gagccguggu ccgcgnn 27

<210> 73
<211> 27
<212> RNA
<213> Artificial

<220>
<223> siRNA

<220>
<221> misc_feature
<222> (26)..(27)
<223> n stands for any base

<400> 73
cgcggaccac ggcuccuccg aagcgnn 27

<210> 74
<211> 22
<212> DNA
<213> Artificial

<220>
<223> antisense

<400> 74
ccccgcgcgg accacggctc ct 22

<210> 75
<211> 23
<212> DNA
<213> Artificial

<220>
<223> primer

<400> 75
ttgtcttgct ctatctttct ttg 23

<210> 76
<211> 35
<212> DNA
<213> Artificial

<220>
<223> primer

<400> 76
gcgcacaccg ccgcctcacc cgtccatgag cccgg 35

<210> 77
<211> 26
<212> DNA
<213> Artificial

<220>
<223> primer

<400> 77
ttaaaagctt tcagtctgag tcaggc 26

<210> 78
<211> 22
<212> DNA
<213> Artificial

<220>
<223> primer

<400> 78
gagccttgcc ttgctgctct ac 22

<210> 79
<211> 21
<212> DNA
<213> Artificial

<220>
<223> primer

<400> 79
caccagggtc tcgattggat g 21

<210> 80
<211> 18
<212> DNA
<213> Artificial

<220>
<223> primer

<400> 80
ctttccacga cggtgaca 18

<210> 81
<211> 18
<212> DNA
<213> Artificial

<220>
<223> primer

<400> 81
tcctccatgg cagtgacc 18

<210> 82
<211> 21
<212> DNA
<213> Artificial

<220>
<223> primer

<400> 82
atgttttctg acggcaactt c 21

<210> 83
<211> 18
<212> DNA
<213> Artificial

<220>

<223> primer

<400> 83
atcagttccg gcaccttg 18

<210> 84
<211> 19
<212> DNA
<213> Artificial

<220>
<223> primer

<400> 84
gagatgcctg ggaagaagg 19

<210> 85
<211> 22
<212> DNA
<213> Artificial

<220>
<223> primer

<400> 85
ttgagtagca cactcgactt cc 22

<210> 86
<211> 27
<212> DNA
<213> Artificial

<220>
<223> Anti-T7 probe

<400> 86
ccctatagtg agtcgtatta tttaaaa 27

<210> 87
<211> 25
<212> DNA
<213> Artificial

<220>
<223> primer

<400> 87
gagcccgcgc ccggaggcgg ggtgg 25

<210> 88
<211> 35
<212> DNA
<213> Artificial

<220>
<223> primer

<400> 88
gcgcacaccg ccgcctcacc cgtccatgag cccgg 35

<210> 89
<211> 24

<212> DNA
<213> Artificial

<220>
<223> primer

<400> 89
aatatctaga atggaggagc cgca 24

<210> 90
<211> 26
<212> DNA
<213> Artificial

<220>
<223> primer

<400> 90
ttaaaagctt tcagtctgag tcaggc 26

<210> 91
<211> 144
<212> DNA
<213> Artificial

<220>
<223> del-1 oligonucleotide

<400> 91
gctagcggag cccgcgcccg gaggcggggt ggagggggtc ggggctcgcg gcgtcgcact 60

gaaacttttc gtccaacttc tgggctgttc tcgcttctcc gcgcggggga agccgagccg 120

agcggagccg cgagaagtgc tagc 144

<210> 92
<211> 146
<212> DNA
<213> Artificial

<220>
<223> del-2 oligonucleotide

<400> 92
gctagcggag cccgcgcccg gaggcggggt ggagggggtc ggggctcgcg gcgtcgcact 60

gaaacttttc gtccaacttc tgggctgttc tcgcttcgga ggagccgtgg tccgcgcccc 120

cgagcggagc cgcgagaagt gctagc 146

<210> 93
<211> 21
<212> DNA
<213> Artificial

<220>
<223> wild-type p53 decoy

<400> 93
ccccgcgtgg cccctgcacc a 21

<210> 94
<211> 21

```
<212>  DNA
<213>  Artificial

<220>
<223>  mutated p53 decoy

<400>  94
cccgcccacc cggctggtcc a                                               21

<210>  95
<211>  22
<212>  DNA
<213>  Artificial

<220>
<223>  mutated vegf antisense

<400>  95
cccgcccggg tcgtccgcac ca                                              22
```